# FASCICULE DE BREVET EUROPEEN

(11) **EP 1 940 841 B1**
(45) Date de publication et mention de la délivrance du brevet: **07.12.2016**
(21) Numéro de dépôt: 06807097.8
(22) Date de dépôt: 09.10.2006
(51) Int. Cl.: C07D 471/08, A61K 49/10

(54) **COMPOSES COMPRENANT UNE PARTIE DE RECONNAISSANCE D'UNE CIBLE BIOLOGIQUE, COUPLEE A UNE PARTIE DE SIGNAL CAPABLE DE COMPLEXER LE GALLIUM**
VERBINDUNGEN MIT EINEM EINE BIOLOGISCHE ZIELSTRUKTUR ERKENNENDEN TEIL, DAS MIT EINEM ZUM KOMPLEXIEREN VON GALLIUM FÄHIGEN SIGNALTEIL GEKOPPELT IST
COMPOUNDS COMPRISING A BIOLOGICAL TARGET RECOGNIZING PART, COUPLED TO A SIGNAL PART CAPABLE OF COMPLEXING GALLIUM

(30) Priorité: 07.10.2005 FR 0510289; 05.04.2006 FR 0602975
(43) Date de publication de la demande: 09.07.2008
(73) Titulaire: GUERBET, 93420 Villepinte (FR)
(72) Inventeur: PORT, Marc, F-95170 Deuil La Barre (FR); COROT, Claire, F-69006 Lyon (FR); GAUTHERET, Thierry, F-77590 Bois-le-Roi (FR)
(74) Mandataire: Regimbeau
(86) Numéro de dépôt international: PCT/EP2006/067211
(87) Numéro de publication internationale: WO 2007/042504

(56) Documents cités:
- EP-A2- 0 352 218
- WO-A-02/40060
- WO-A-93/19787
- WO-A-94/04485
- WO-A-98/39288
- WO-A-03/013346
- WO-A-03/059397
- WO-A-2004/089517
- WO-A-2004/112840
- WO-A-2006/090232
- WO-A-2006/100305
- US-A- 6 071 490
- US-A1- 2003 082 106
- HENZE M ET AL: "PET imaging of somatostatin receptors using" JOURNAL OF NUCLEAR MEDICINE, NEW YORK, NY, US, vol. 42, no. 7, juillet 2001 (2001-07), pages 1053-1056, XP002245466 ISSN: 0161-5505
- VELIKYAN I ET AL: "Preparation and evaluation of <68>Ga-DOTA-hEGF for visualization of EGFR expression in malignant tumors" JOURNAL OF NUCLEAR MEDICINE, SOCIETY OF NUCLEAR MEDICINE, RESTON, VA, US, vol. 46, no. 11, novembre 2005 (2005-11), pages 1881-1888, XP002404435 ISSN: 0161-5505
- HOFFEND J ET AL: "Gallium-68-DOTA-albumin as a PET blood-pool marker: experimental evaluation in vivo" NUCLEAR MEDICINE AND BIOLOGY, ELSEVIER, NY, US, vol. 32, 2005, pages 287-292, XP002413061 ISSN: 0969-8051

## Description

L'invention concerne des composés comprenant une partie de reconnaissance d'une cible biologique, couplée à une partie de signal capable de complexer le Gallium. L'invention concerne aussi des procédés d'obtention de ces composés, et décrit des procédés de screening capables de sélectionner de tels composés pour leur synthèse chimique et leurs utilisations diagnostiques.

On connaît déjà des composés désignés composés spécifiques vectorisés destinés à l'imagerie moléculaire en médecine nucléaire.
On connaît ainsi de nombreux composés comprenant un biovecteur couplé à un radionucléide. Selon la nature du biovecteur et du radionucléide, le biovecteur est couplé au radionucléide soit directement, soit par l'intermédiaire d'un chélate qui complexe le radionucléide. Selon le spectre de dégradation des radionucléides, on peut les utiliser pour une imagerie PET ou pour une imagerie SPECT.
L'imagerie PET (émission de positrons donnant lieu à une émission de photons détectés par un scanner PET) utilisant le radionucléide F18 est particulièrement utilisée pour le suivi métabolique de zones tumorales en utilisant le FDG (fluorodéoxyglucose). Un inconvénient majeur de l'utilisation des isotopes courants tels que le F18 en PET est la nécessité d'un cyclotron producteur de l'isotope, et cela en général à proximité du site d'administration du produit au patient compte tenu de la durée de vie des isotopes, ce qui pose des problèmes de coût et de logistique importants.
De nombreux composés utilisant le technicium et l'indium ont été décrits pour leur utilisation en imagerie SPECT (émission de photons, avec utilisation de radionucléides émettant une énergie de l'ordre de 100 à 200 keV notamment). Le SPECT présente toutefois une plus faible résolution spatiale que le PET et peut impliquer une visualisation du patient 2 à 3 jours après l'administration du produit du fait de la durée de vie de certains isotopes tels que l'In111.
Le choix entre le PET et le SPECT est notamment fonction de l'indication diagnostique,

Le demandeur s'est particulièrement intéressé à l'utilisation en imagerie PET du Gallium Ga 68 car cet isotope est produit, non pas par un cyclotron, mais par un générateur (Germanium Ge68 / Gallium Ga68), appareil beaucoup moins complexe et coûteux que le cyclotron.
Des composés spécifiques utilisant le Ga68 ont été décrits, notamment le Ga68/S3N (agent chélatant tetradentate amine trithiolate), le DOTATOC (DOTA-DPhel-Tyr3-octréotide, le biovecteur étant un analogue de la somatostatine). Il est rappelé que du fait de l'état d'oxydation 3+ du Ga 68, celui-ci est typiquement couplé à différents chélates dont notamment des dérivés du DOTA, DTPA, NOTA. Deux procédés de préparation sont possibles :
- le biovecteur est couplé au chélate puis le composé formé est complexé avec le Ga68 produit par le générateur
- le chélate est complexé avec le Ga68 produit par le générateur, puis le complexe formé est couplé au biovecteur.
La durée de vie du Ga68 est de 68 minutes, ce qui rend son utilisation possible en PET clinique, mais requiert comme pour le F18 (dont la demi vie est de 121 minutes) une durée de préparation faible du produit incorporant le Ga68, et de préférence inférieure à environ 40 minutes. Un procédé de préparation avec micro-ondes destiné à réduire le temps de préparation de composés avec Ga68 a été décrit dans WO2004/089425.
Le besoin demeure d'obtenir de nouveaux composés utilisant le Ga68 particulièrement efficaces notamment pour certaines indications diagnostiques non couvertes à ce jour avec cet isotope, et dont la préparation est suffisamment rapide et simple pour une utilisation clinique courante et économique.

Le demandeur a réussi à obtenir de nouveaux composés intéressants et l'invention décrit selon un aspect une méthode de sélection de composés efficaces en imagerie PET du gallium Ga68 comprenant :
- la sélection de biovecteurs B ayant une affinité au moins micromolaire, de préférence nanomolaire, à partir d'une base de molécules pharmacologiques actives
- la sélection de chélates Ch ayant avec le gallium ⁶⁸Ga³⁺ une constante d'affinité log K d'au moins 10, et de préférence d'au moins 20, d'au moins 25, d'au moins 30 ou d'au moins 35, dans une banque de chélates linéaires ou macrocycliques
- le sélection de liens chimiques (linker) L destinés au couplage d'au moins un biovecteur avec au moins un chélate (désigné Ch dans la demande)
- la synthèse des composés (B-L-Ch)
- la synthèse des composés (B-L-Ch-Ga68) par complexation du composé (B-L-Ch) avec le Ga68
- l'imagerie PET du composé (B-L-Ch-Ga68).

Par l'expression biovecteur issu d'une base de molécules pharmacologiques actives, on entend toute molécule (désignée biovecteur) ayant une activité connue pour le ciblage d'une zone pathologique et/ou diagnostique d'intérêt, notamment des molécules issues de chimiothèques, et notamment tout biovecteur ou catégorie de biovecteurs cités dans la présente demande.
Selon une réalisation la base ou chimiothèque est une banque de biovecteurs utilisés pour l'imagerie SPECT.
Selon une réalisation la base ou chimiothèque est une banque de biovecteurs utilisés pour l'imagerie PET au F18.
L'invention décrit aussi tout produit vectorisé utilisable en imagerie PET au gallium Ga68, obtenu par le procédé de sélection ci-dessus.
L'invention concerne un procédé de préparation de composés efficaces en imagerie PET du gallium Ga68 avec ou sans micro-ondes comprenant :
- la synthèse d'un composé (B-L-Ch) par couplage d'un biovecteur à un chélate linéaire ou macrocyclique
- la complexation du composé (B-L-Ch) avec le gallium Ga68 en moins de 20minutes.
Dans le cas du procédé sans micro-ondes, le rendement sera avantageusement supérieur à 30% et la pureté supérieure à 50%.

L'invention concerne aussi des composés destinés à l'imagerie PET au gallium 68 comprenant un biovecteur B et un chélate Ch capable de complexer le Ga68, lié par un linker L, le chélate comportant au moins une partie de masquage du chélate in vivo, notamment un groupe hydrophile. Avantageusement cette partie de masquage est choisie de manière à ne pas altérer l'affinité du biovecteur avec sa cible biologique.

L'invention concerne aussi des composés destinés à l'imagerie PET au gallium 68 comprenant un biovecteur B et un chélate Ch capable de complexer le Ga68, lié par un linker L, le chélate comportant en outre une partie de reconnaissance d'une cible biologique améliorant la biodistribution du composé, ladite partie de reconnaissance étant différente du biovecteur.

Le demandeur a ciblé plusieurs axes d'amélioration majeurs.
Selon un aspect, le demandeur a étudié des produits dont le chélate complexant le Gallium n'altère pas la spécificité de la reconnaissance de la cible biologique par le biovecteur du produit. Le demandeur a ainsi étudié plusieurs voies de synthèse chimique et plusieurs types de chélates n'altérant pas cette reconnaissance.
Selon un autre aspect, le demandeur a étudié des produits dont la spécificité de ciblage est particulièrement élevée. Le demandeur a ainsi étudié plusieurs voies de synthèse chimique et plusieurs types de biovecteurs améliorant la spécificité du produit. Le demandeur a ainsi recherché des biovecteurs à forte spécificité, notamment des biovecteurs connus du domaine thérapeutique, ayant de préférence une affinité au moins micromolaire, notamment nanomolaire, mais non encore utilisés dans le domaine diagnostique.
Le demandeur a aussi étudié les possibilités d'association de plusieurs biovecteurs, entre eux ou par l'intermédiaire du ou des chélates du produit :
- les biovecteurs sont couplés entre eux par un groupe de liaison chimique (linker), au moins un biovecteur étant couplé à au moins un chélate
- le composé comprend un chélate qualifié de central et porteur de plusieurs biovecteurs par l'intermédiaire de linkers appropriés
- le cas échéant le biovecteur est couplé à un chélate couplé lui-même à plusieurs chélates reliés entre eux par des linker appropriés.
Le demandeur a par ailleurs étudié un assemblage entre le ou les biovecteurs et le ou les chélates de manière à ce que l'accès à la cible ne soit pas gêné malgré la présence du ou des chélates :
- le chélate est espacé du ou des biovecteurs par un linker d'une taille suffisante et d'une structure chimique telles que la reconnaissance du ou des biovecteurs par la cible n'est pas altérée
- pour des biovecteurs d'une taille suffisante, le biovecteur comprend d'une part une partie de reconnaissance de la cible biologique non reliée directement au chélate, et d'autre part au moins une partie de structure dont la liaison (directe ou par un linker) avec un ou plusieurs chélates n'interfère pas sur la reconnaissance spécifique.
- la structure du chélate est définie de manière à ce qu'il apparaisse comme invisible ou transparent en milieu biologique vis-à-vis du biovecteur. Ces chélates sont notamment utiles lorsque le biovecteur doit atteindre un site de reconnaissance difficile d'accès tel qu'un site catalytique d'une enzyme, ou pour des biovecteurs dont le couplage avec des chélates connus pose un problème de spécificité ou de reconnaissance par leur cible biologique (notamment biovecteurs de petite taille et/ou ayant des difficultés d'accès à une zone biologique). Ce caractère transparent peut être obtenu par l'utilisation de groupes chimiques appropriés notamment des groupes hydrophiles ou au contraire lipophiles masquant le chélate. Dans ce cas le chélate comporte au moins une partie de masquage du chélate in vivo.

Parmi les associations biovecteurs/chélates, la présente invention concerne :
- un chélate central relié à plusieurs biovecteurs identiques ou différents.
- un premier ensemble [biovecteur porteur de chélates(s)] couplé par l'intermédiaire d'un linker hydrophobe ou hydrophile à un second ensemble [biovecteur porteur de chélates(s)], s'écrivant par exemple (Ch)2- B1 - Linker - B2 (Ch)2 avec Ch représentant des chélates identiques ou différents et B1 et B2 représentant des biovecteurs identiques ou différents
- plusieurs biovecteurs formant une sorte de couronne en interaction avec le gallium
- un ensemble B1- (Linker porteur de Ch) - B2 - Ch.
Le demandeur a aussi étudié des composés utilisables en imagerie PET du Ga68 et comprenant en plus du biovecteur et du chélate une structure chimique lui conférant des propriétés diagnostiques utiles, selon une logique par exemple décrite dans WO2005/082425 page 60-67, notamment des polymères (polysaccharides, poly acides aminés, polymères hydrophiles de type PEG par exemple), des systèmes d'encapsulation de type liposomes, des systèmes lipidiques de transport et/ou de libération du composé de diagnostique tels que des liposomes, des micelles (qui permettent un passage dans le système lymphatique au lieu du système circulatoire), des nanoémulsions.
Le demandeur a aussi étudié plusieurs voies de synthèse chimique et plusieurs types de chélates permettant d'activer le produit sur le site de ciblage, selon un fonctionnement qualifié d'intelligent ou smart. Lorsque le produit est à proximité de sa cible in vivo, il subit une modification de structure telle que l'affinité du biovecteur vis-à-vis de sa cible est améliorée. Par affinité améliorée, on entend que le biovecteur a une meilleure reconnaissance et/ou que sa durée d'interaction avec sa cible est augmentée. Par exemple, le produit subit une modification de conformation et/ou un clivage par exemple enzymatique entraînant une meilleure exposition du site de liaison du biovecteur vis-à-vis de sa cible. L'origine de ladite modification de structure peut être localement notamment liée au pH, au potentiel d'oxydo-réduction .... Le biovecteur peut par exemple être un substrat ou un inhibiteur d'une enzyme.

Selon un autre aspect, il est décrit des composés dont le biovecteur est couplé directement à au moins un gallium, sans utilisation de chélate, la reconnaissance du biovecteur n'étant pas altérée par l'interaction entre le biovecteur et le ou les atomes de gallium. Différents cas sont possibles, par exemple :
- le biovecteur comprend deux sites, dont un site de reconnaissance de la cible et un site d'interaction avec le gallium, ce second site formant une sorte de loge autour du gallium.
- le biovecteur comprend plusieurs sites, chaque site étant à la fois un site de reconnaissance et un site d'interaction avec le gallium, chaque site formant une sorte de loge autour du gallium. Le cas échéant ces deux sites de reconnaissance sont des biovecteurs différents ciblant des cibles identiques ou différentes.
On peut aussi utiliser des composés comprenant, en plus du chélate et de la partie biovecteur de ciblage, une partie de transport capable de véhiculer le biovecteur de ciblage jusqu'à une zone d'intérêt. Par exemple la zone d'intérêt sera un territoire biologique du cerveau rendu accessible au composé grâce à un transporteur capable de passer la barrière hémato encéphalique (BHE). On aura par exemples les associations suivantes : (Ch)-X-L1-Y ; X-(Ch)-Y ; X-L1-(Ch)-L2-Y , avec : X un biovecteur de ciblage, Y une partie de transport, L1 et L2 des linkers le cas échéant biodégradables.

Selon un autre aspect, le demandeur a étudié l'utilisation de biovecteurs connus pour leur forte spécificité pour une cible biologique associée à une zone pathologique, mais dont le couplage avec les isotopes couramment utilisés en médecine nucléaire, notamment avec le F18, est chimiquement difficile et/ou pose des problèmes de stabilité du complexe biovecteur-F18 synthétisé. Le demandeur a ainsi étudié l'utilisation de biovecteurs reconnus pour leur spécificité (typiquement d'au moins 10⁻⁹ à 10⁻¹² M), et dont le couplage avec le gallium (par l'intermédiaire du chélate) est nettement plus facile et/ou plus rapide et/ou fournit un composé plus stable qu'avec le F18. Ceci permet aussi de laisser davantage de temps au praticien dans l'utilisation clinique du produit.
Les difficultés de couplage du F18 (Kryptofix R, réaction d'échange avec le mésylate ou le trifate, utilisation de F18 anhydre, F18-F2, F18XeF2, F18 DAST ...) sont rappelées dans l'art antérieur, par exemple dans WO2005/082425.
Le demandeur a étudié en particulier l'utilisation du gallium au lieu du F18 pour des indications diagnostiques (à l'aide de biovecteurs appropriés) requérant la forte résolution spatiale du PET (2 à 3 fois supérieures à celle SPECT), notamment en oncologie (diagnostic précoce des cancers, bilan d'extension, suivi de thérapies), neurologie, cardiologie, infectiologie. On ciblera notamment la détection de zones tumorales de l'ordre de 5 mm, la détection de tumeurs primaires et de nodules métastatiques.

Le demandeur a aussi étudié l'utilisation du gallium au lieu du F18, pour des biovecteurs dont la spécificité de reconnaissance de leur cible (notamment entre une zone normale et une zone pathologique ou à risque pathologique) est assez élevée pour obtenir un bon contraste dans l'image et donc pour répondre au besoin diagnostique, même si la résolution en imagerie PET avec le gallium est moins élevée qu'avec le F18.
Le demandeur a particulièrement étudié l'utilisation du gallium pour l'indication diagnostique de perfusion myocardique en imagerie PET, éventuellement en combinaison avec un traitement thérapeutique, en sélectionnant les biovecteurs prometteurs parmi la liste des nombreux biovecteurs utilisés ou utilisables dans cette indication.

Le demandeur a aussi étudié des produits diagnostiques en imagerie PET incorporant du Ga68 pour le suivi de l'efficacité d'un produit thérapeutique, le biovecteur du composé de diagnostique étant identique ou différent du biovecteur du produit thérapeutique, les deux produits étant coadministrés simultanément ou en différé. On peut aussi utiliser des composés mixtes comprenant une partie thérapeutique et une partie diagnostique, se séparant éventuellement sur le site d'action du composé mixte. Le demandeur a aussi étudié des méthodes d'imagerie plus spécialement destinées à l'imagerie PET du Ga68, en particulier des appareillages, des séquences, des méthodes de traitement du signal, des méthodes d'acquisition, de transfert et de compilation de données, adaptées aux particularités (demi-vie ...) du Ga68. L'imagerie physiologique à l'aide du produit en imagerie PET du Ga68 est le cas échéant combinée à une lecture anatomique au scanner avec éventuellement produit de contraste pour scanner aux rayons X. On peut coadministrer de manière simultanée ou en différé un produit PET au Ga68 et un autre produit de contraste. Le demandeur a ainsi étudié des possibilités d'administration de plusieurs produits, presque simultanée ou au contraire différée de quelques heures, pour une imagerie en PET, chaque produit apportant une information diagnostique pertinente, par exemple un produit marqué au F18 et un produit marqué au Ga68. Le demandeur a aussi étudié les possibilités de combiner les résultats obtenus sur un même patient en combinant les modalités d'imagerie en utilisant des produits marqués au Ga68, par exemple en PET-CT ou en PET IRM.

Le demandeur s'est intéressé également à des composés comprenant un biovecteur utilisé en imagerie SPECT avec le technicium ou l'indium par exemple, mais qu'il serait intéressant d'utiliser en imagerie PET avec le Gallium, compte tenu de l'efficacité de la reconnaissance spécifique connue de ces biovecteurs. Le choix de tels composés est permis par le fait que la chimie est assez proche entre le gallium et ces éléments. Par exemple de tels composés comprennent un biovecteur couplé à un chélate complexant le gallium au lieu de l'Indium, avec comme avantage une obtention rapide des résultats alors que l'utilisation de l'Indium nécessitait une visualisation du patient 2 à 3 jours après l'administration du produit.
En outre le demandeur a étudié l'utilisation de biovecteurs connus pour leur utilisation dans certaines indications connues en SPECT compte tenu du radionucléide utilisé jusqu'à présent (technicium notamment), dans d'autres indications diagnostiques en imagerie PET grâce à l'utilisation du Ga68 au lieu du radionucléide utilisé jusqu'à présent.
En outre, contrairement à l'utilisation de l'indium qui nécessite un chélate/et ou un biovecteur stable au moins 2 à 3 jours, avec le gallium le chélate et/ou le composé chélate/biovecteur peut n'être stable que 1 à 3 heures (le temps de la préparation du produit et de l'imagerie du patient).
Le demandeur a en particulier étudié des composés comprenant des biovecteurs dont la cinétique de biodistribution est telle que le gallium ne soit pas désintégré avant d'atteindre sa cible biologique, ces composés atteignant leur cible chez le patient en moins de deux à trois heures.
Parmi les biovecteurs utilisés en SPECT, le demandeur a notamment étudié des améliorations mises au point pour le SPECT et susceptibles d'être utiles pour le PET du gallium. On peut citer notamment :
- le couplage avec des dérivés quinoline et quinolinine (WO2005/079886)
- le couplage avec des dérivés de ciblage mitochondrial avec un ciblage et une rétention suffisante dans le myocarde (dérivés de roténone dans WO2003/086476, dérivés inhibiteurs de MC1 du US2005/0191238)
- le couplage avec des dérivés pyridyle et imidazolyle (WO 2003/077727)
- le couplage avec des dérivés benzodiazépines (WO00/61195) pour le ciblage de GPIIbIIa (thrombus) avec des chélates porteurs de groupes sulfure
- le couplage avec des dérivés porteurs de fonctions phosphine porteurs de groupes hydroxy, polyhydroxy, carboxy ou polycarboxy (WO01/77122) pour améliorer la biodistribution notamment de biovecteurs ciblant des intégrines
- l'utilisation d'agents de protection contre l'oxydation (WO01/00637)
- l'utilisation d'agents stabilisants (agents radioprotecteurs et antimicrobiens de WO02/053192)
- l'utilisation de dérivés Cardiolite et Myoview.

Pour le choix de biovecteurs appropriés, le demandeur s'est intéressé tout particulièrement aux grandes familles de biovecteurs utilisés en thérapeutique et/ou en imagerie de diagnostique : protéines, glycoprotéines, lipoprotéines, polypeptides, peptides, peptidomimétiques, colorants, sucres, oligosaccharides, neuromédiateurs, et de manière générale tout ligand peptidique ou non connu de l'homme du métier comme capable de reconnaître au moins une cible biologique telle que notamment des récepteurs, des enzymes, sans que le couplage avec le chélate n'altère de manière non souhaitée l'activité biologique de la cible.
Pour le choix des biovecteurs, le demandeur s'est intéressé à tout biovecteur
- connu de l'art antérieur et utilisable en imagerie
- connu de l'art antérieur, non décrit pour l'imagerie, mais qui peut l'être compte tenu de sa reconnaissance spécifique d'au moins une cible biologique indicatrice d'un état ou d'un risque d'état pathologique
Le choix du biovecteur peut être réalisé en fonction de sa structure et/ou en fonction de son utilisation souhaitée en imagerie, telle que le marquage d'un mécanisme biologique susceptible d'être altéré et se traduisant par exemple par un niveau d'expression modifié de la cible du biovecteur par rapport à l'état non pathologique : récepteurs cellulaires, métabolisme cellulaire, transport intra ou extracellulaire, molécules informatrices et/ou activatrices d'effecteurs ou de cellules.

A titre d'exemples on citera les biovecteurs suivants, cités notamment dans WO2004/112839 capables de cibler un ligand associé à (intervenant directement ou indirectement et/ou surexprimés dans) un processus pathologique.
1) Les biovecteurs décrits dans les documents WO01/97850 (ciblant des récepteurs VEGF et angiopoiétine), US6372194 (polymère tel que polyhystidine), WO2001/9188 (polypeptide ciblant la fibrine), WO01/77145 (peptide de ciblage d'intégrines), WO0226776 (ligand de ciblage d'intégrine αvβ3), WO99/40947 (ligands ciblant par exemple le récepteur KDR/F1k-1 dont R-X-K-X-H et R-X-K-X-H, ou les récepteurs Tie-1 e 2), WO02062810 (glycosides de sialyl Lewis), WO02/40060 (antioxydants tels que l'acide ascorbique), US6524554 (ciblage de la tuftsine), WO02/094873 (ciblage de récepteurs à protéine G GPCR en particulier la cholécystokinine), US6489333 (association antagoniste d'intégrine et mime de la guanidine), US6511648 (quinolone ciblant αvβ3 ou 5), US2002/0106325, WO01/97861 (benzodiazépines et analogues ciblant des intégrines), WO01/98294 (imidazoles et analogues), WO01/60416 (inhibiteurs de MMP, notamment des hydroxamates), WO02/081497 (peptides de ciblage d'αvβ3 tels que RGDWXE), WO01/10450 (peptides RGD), US6261535 (anticorps ou fragments d'anticorps (FGF, TGFb, GV39, GV97, ELAM, VCAM, inductible par TNF ou IL), US5707605 (molécule de ciblage modifiée par interaction avec sa cible), WO 02/28441 (agents de ciblage de dépôts amyloïdes), WO02/056670 (peptides clivés cathepsines), US6410695 (mitoxantrone ou quinone), US6391280 (polypeptides ciblant des cellules épithéliales), US6491893 (GCSF), US2002/0128553, WO02/054088, WO02/32292, WO02/38546, WO20036059, US6534038, WO99/54317 (inhibiteurs de cystéines protéases), WO0177102, EP1121377, Pharmacological Reviews (52,n°2, 179 ; facteurs de croissance PDGF, EGF, FGF, ...), Topics in Current Chemistry (222, W.Krause, Springer), Bioorganic & Medicinal Chemistry (11, 2003, 1319-1341 ; dérivés tétrahydrobenzazépinones ciblant αvβ3).
2) Les inhibiteurs d'angiogenèse, notamment ceux testés en essais cliniques ou déjà commercialisés, notamment :
   - les inhibiteurs d'angiogenèse impliquant des récepteurs FGFR ou VEGFR tels que SU101, SU5416, SU6668, ZD4190, PTK787, ZK225846, des composés azacycles (WO00244156, WO02059110);
   - les inhibiteurs d'angiogenèse impliquant des MMP tels que le BB25-16 (marimastat), le AG3340 (prinomastat), le solimastat, le BAY12-9566, le BMS275291, le metastat, le neovastat ;
   - les inhibiteurs d'angiogenèse impliquant des intégrines tels que le SM256, le SG545, des molécules d'adhésion bloquant le EC-ECM (tels que le EMD 121-974, ou la vitaxine) ;
   - des médicaments à mécanisme d'action antiangiogenèse plus indirect tels que le carboxiamidotriazole, le TNP470, la squalamine, le ZD0101;
   - les inhibiteurs décrits dans le document WO99/40947, les anticorps monoclonaux très sélectifs pour la liaison au récepteur KDR, les analogues de la somatostatine (WO94/00489), les peptides de liaison à la sélectine (WO94/05269), des facteurs de croissance (VEGF, EGF, PDGF, TNF, MCSF, interleukines); des biovecteurs de ciblage de VEGF décrits dans Nuclear Medicine Communications ,1999, 20 ;
   - les peptides inhibiteurs du document WO02/066512.
   - des dérivés de folates
   - des agents de ciblage de la maladie d'Alzheimer
   - des exopolysaccharides
3) Des biovecteurs capables de cibler des récepteurs : CD36, EPAS-1, ARNT, NHE3, Tie-1, 1/KDR, Flt-1, Tek, neuropiline-1, endogline, pléientropine, endosialine, Ax1., a1Pi, a2ssl, a4P1, a5p1, eph B4 (éphrine), récepteur laminine A, récepteur neutrophiline 65, récepteur leptine OB-RP, récepteur chimiokine CXCR-4 (et autres récepteurs cités dans le document WO99/40947), LHRH, bombésine/GRP, récepteurs gastrine, VIP, CCK.
4) Des biovecteurs de type inhibiteurs de tyrosine kinase.
5) Les inhibiteurs du récepteur GPIIb/IIIa connus choisis parmi : (1) le fragment fab d'un anticorps monoclonal du récepteur GPIIb/IIIa, Abciximab (ReoPro™) , (2) les petites molécules peptidiques et peptidomimétiques injectées en intraveineuse telles que l'eptifibatide (Integrilin™) et le tirofiban (Aggrastat™). Etant rappelé que le choix éventuel d'un anticorps devra prendre en compte la durée d'atteinte de sa cible.
6) Des ligands antagonistes de récepteurs au fibrinogène (EP425212), des peptides ligands de récepteurs IIb/IIIa, des ligands du fibrinogène, des ligands de la thrombine, des peptides capables de cibler la plaque d'athérome, les plaquettes, la fibrine, des peptides à base d'hirudine, des dérivés à base de guanine ciblant le récepteur IIb/IIIa.
7) D'autres biovecteurs ou fragments biologiquement actifs de biovecteurs connus de l'homme du métier comme médicaments, à action anti-thrombotique, anti agrégation plaquettaire, antiathérosclérotique, antiresténotique, anticoagulante.
8) D'autres biovecteurs ou fragments biologiquement actifs de biovecteurs ciblant αvβ3, décrits en association avec des DOTA dans le brevet US6537520, choisis parmi les suivants : mitomycine, tretinoine, ribomustine, gemcitabine, vincristine, etoposide, cladribine, mitobronitol, methotrexate, doxorubicine, carboquone, pentostatine, nitracrine, zinostatine, cetrorelix, letrozole, raltitrexed, daunorubicine, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoine, streptozocine, nimustine, vindesine, flutamide, drogenil, butocin, carmofur, razoxane, sizofilan, carboplatine, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, leutinizing hormone releasing factor.
9) certains biovecteurs ciblant des types particuliers de cancers, par exemple des peptides ciblant le récepteur ST associé au cancer colo-rectal, ou le récepteur tachykinine.
10) des biovecteurs utilisant des composés de type phosphines.
11) des biovecteurs de ciblage de P-sélectine, E-sélectine (par exemple le peptide de 8 acides aminés décrit par Morikawa et al, 1996, 951).
12) l'annexine V ou des biovecteurs ciblant les processus apoptotiques.
13) tout peptide obtenu par des technologies de ciblage telles que le phage display, modifié éventuellement par des acides aminés non naturels (http//chemlibrary.bri.nrc.ca), par exemple des peptides issus de banques phage display: RGD, NGR, CRRETAWAC, KGD, RGD-4C, XXXY*XXX, RPLPP, APPLPPR.
14) d'autres biovecteurs peptidiques connus de ciblage de plaques d'athérome cités notamment dans le document WO2003/014145.
15) des vitamines (folates notamment).
16) des ligands de récepteurs hormonaux dont les hormones et les stéroïdes.
17) des biovecteurs ciblant des récepteurs opioïdes.
18) des biovecteurs ciblant des récepteurs TKI, des récepteurs CXCR (1 à 4 notamment).
19) des antagonistes LB4 et VnR.
20) des composés nitroimidazoles et benzylguanidines..
21) des biovecteurs rappelés dans Topics in Current Chemistry, vol.222, 260-274, fundamentals of receptor-based diagnostic metallopharmaceuticals, notamment :
   - des biovecteurs de ciblage de récepteurs peptidiques surexprimés dans les tumeurs (récepteurs LHRH, bombésine/GRP, récepteurs VIP, récepteurs CCK, récepteurs tachykinine par exemple), notamment les analogues de somatostatine ou de bombésine, des peptides dérivés octréotide éventuellement glycosylés, les peptides VIP, les alpha-MSH, les peptides CCK-B
   - des peptides choisis parmi : des peptides cycliques RGD, fibrine-chaîne alpha, CSVTCR, tuftsine, fMLF, YIGSR (récepteur : laminine)
22) des polysaccharides et des dérivés d'oses, des dérivés ciblant Glut.
23) des biovecteurs utilisés pour des produits de type smart.
24) des marqueurs de la viabilité myocardique (par exemple tétrofosmine et hexakis2métoxy-2méthylpropylisonitrile).
25) des traceurs du métabolisme des sucres et des graisses.
26) des ligands de récepteurs de neurotransmetteurs (récepteurs D,5HT,Ach,GABA,NA).
27) des oligonucléotides.
28) des biovecteurs peptidiques du WO03/011115 pages 36 à 43
28) des biovecteurs déjà utilisés pour le SPECT ou le PET, cités dans : WO03/018640, WO03/020701, WO03/078569, US2003/0152513, WO03/086475, WO2005/002293, US20050048000, WO2004/069365, WO2005/012335, WO2005/044312, WO2005/042033, WO2003/013346, WO2005/023314, WO2005/019247, WO2005/049096, WO2005/049095, WO2005/044313, WO2005/042033, US2004/0210041, US2005/201943, WO2005/082889.
29) des biovecteurs issus des squelettes chimiques désignés scaffolds et décrits dans US2005/026127.
30) des biovecteurs ciblant GLUT, des transporteurs GLUT (récepteurs glucose GLUT1 et autres) qui sont déjà connus pour être couples à des chélates complexés par des lanthanides tels que le Gd3+.

Pour la partie linker du produit, à titre d'exemples on citera les linkers suivants:
1) les acides aminés
2) des liens L capables d'interagir avec au moins un groupe fonctionnel de biovecteur et au moins un groupe fonctionnel du chélate. L inclut notamment des chaînes alkyles substituées ou non, saturées ou non, droites ou ramifiées, des peptides, des polyéthylènes glycoles et des polyoxyéthylènes. On citera notamment :
3)
   a.1) (CH₂)₂-phényl-NH, (CH₂)₃-NH, NH-(CH₂)₂-NH, NH-(CH₂)₃-NH, rien ou une simple liaison, (CH₂)ₙ, (CH₂)ₙ-CO-, -(CH₂)ₙNH-CO- avec n = 2 à 10 , (CH₂CH₂O)_{q}(CH₂)ᵣ-CO- , (CH₂CH₂O)_{q}(CH₂)ᵣ-NH-CO- avec q = 1-10 et r=2-10, (CH₂)ₙ-CONH-, (CH₂)ₙ-CONH - PEG, (CH₂)ₙ-NH-, 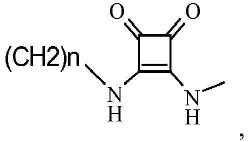 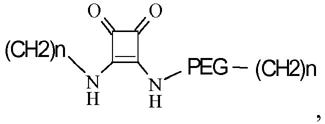 avec n=1 à 5 et avantageusement n=4 ou 5, HOOC-CH₂-O-(CH₂)₂-O-(CH₂)₂-O-CH₂-COOH; HOOC-(CH₂)₂-CO₂-(CH₂)₂-OCO-(CH₂)₂-COOH ; HOOC-CH(OH)-CH(OH)-COOH; HOOC-(CH₂)ₙ-COOH; NH₂-(CH₂)ₙ-NH₂, avec n=0-20; NH₂-(CH₂)ₙ-CO₂H ; NH₂-CH₂ - (CH₂-O-CH₂)ₙ-CO₂H avec n=1 à 10, des linkers désignés A8 à A32 du document WO 2006/095234, pages 104 et 105.
   a.2) P1-1-P2, identiques ou différents, P1 et P2 étant choisis parmi O, S, NH, rien, CO₂, NHCO, CONH, NHCONH, NHCSNH, SO₂NH-, NHSO₂-, squarate avec 1 = alkyle, alkoxyalkyle, polyalkoxyalkyle (PEG), alkyle interrompu par un ou plusieurs squarates ou par un ou plusieurs aryles, avantageusement phényles, alcényle, alcynyle, alkyle interrompus par un ou plusieurs groupes choisis parmi -NH-, -O-, -CO-, -NH(CO)-, -(CO)NH-, -O(CO)-, ou -(OC)O- .L aura par exemple un poids moléculaire compris entre 300 et 2000 g/mol, notamment entre 300 et 1000 g/mol.
   P1 et P2 sont ainsi des groupes de couplage du linker avec d'une part le chélate et d'autre part le biovecteur.
4) des liens décrits dans le brevet US 6 264 914, capables de réagir avec les groupes fonctionnels (du biovecteur et du chélate) amino, hydroxyle, sulfhydryle, carboxyle, carbonyle, carbohydrates, thioéthers, 2-aminoalcohols, 2-aminothiols, guanidinyle, imidazolyle, phénolique.
   Des groupements capables de réagir avec des groupes sulfhydryle incluent les composés alpha-haloacétyle du type X-CH₂CO- (où X=Br, Cl ou I), qui peuvent aussi être utilisés pour agir avec des groupes imidazolyle, thioéther, phénol, amino. Des groupements capables de réagir notamment avec des groupes amino incluent :
   - des composés d'alkylation : composés alpha-haloacétyle, dérivés N-maleémide, composés aryle (nitrohaloaromatiques par exemple), aldéhydes et cétones capables de formation de bases de Schiff, des dérivés époxydes tels que l'épichlorhydrine, des dérivés de triazines contenant de la chlorine très réactifs vis-à-vis de nucléophiles, des aziridines, des esters de l'acide squarique, des éthers alpha.-haloalkyle.
   - des composés d'acylation : isocyanates et isothiocyanates, des chlorures de sulfonyle, des esters tels que des nitrophénylesters ou des N-hydroxysuccinimidyl esters, des anhydrides d'acide, des acylazides, des azlactones, des imidoesters. Des groupements capables de réagir avec des groupes carboxyle incluent des composés diazo (diazoacétate esters, diazoacétamides), des composés modifiant des acides carboxyliques (carbodiimides par exemple), des dérivés isoxazolium (nitrophenylchloroformate, carbonyidiimidazoles...), des dérivés quinoline.

   Des groupements capables de réagir avec des groupes guanidinyle incluent des composés diones tels que le phenylènediglyoxal, des sels diazonium.
5) certains liens décrits dans le brevet US 6 537 520 de formule (Cr₆r₇)_{g}-(W)ₕ-(Cr₆ₐr7ₐ)_{g'}-(Z)ₖ-(W)_{h'}-(Cr₈r₉)_{g"}-(W)_{h"}-(Cr₈ₐr₉ₐ)_{g'"} avec :
   - g+h+g'+k+h'+g"+h"+g"' différent de 0;
   - W choisi parmi O, S, NH, NHCO, CONH, CO, COO, OCO, NHCSNH, NHCONH, SO₂, (OCH₂CH₂)ₛ, (CH₂CH₂O)_{s'}, (OCH₂CH₂CH₂)_{s"}, (CH₂CH₂CH₂O)ₜ ;
   - Z choisi dans le groupe : aryle substitué par 0 à 3 r₁₀, cycloalkyle en C₃-C₁₀ substitué par 0 à 3 r₁₀, système hétérocycle de 5-10 membres contenant 1-4 hétéroatomes indépendamment choisis parmi N, S, O et substitué par 0 à 3 r₁₀ ;
   - r6,r6a, r7, r7a, r8, r8a, r9, r9a indépendamment choisis parmi : H, =O, COOH, SO₃H, PO₃H, alkyle en C₁-C₅ substitué par 0 à 3 r₁₀, aryle substitué par 0 à 3 r₁₀, benzyle substitué par 0 à 3 r₁₀, alkoxy en C₁-C₅ substitué par 0 à 3 r₁₀, NHCOr₁₁, CONHr₁₁, NHCONHr₁₁, NHr₁₁, r₁₁, et un lien avec le chélate ;
   - r₁₀ indépendamment choisi parmi : un lien avec le chélate, COOr₁₁, OH, NHr₁₁, SO₃H, PO₃H, aryle substitué par 0 à 3 r₁₁, alkyle en C₁-C₅ substitué par 0 à 1 r₁₂, alkoxy en C₁-C₅ substitué par 0 à 1 r₁₂, et un hétérocycle de 5-10 membres contenant 1-4 hétéroatomes indépendamment choisis parmi N, S, O et substitué par 0 à 3 r₁₁;
   - r₁₁ est indépendamment choisi parmi : H, aryle substitué par 0 à 1 r₁₂, hétérocycle à 5-10 membres comprenant 1-4 hétéroatomes choisis parmi N, S, O et substitué par 0 à 1 r₁₂, cycloalkyle en C₃-C₁₀ substitué par 0 à 1 r₁₂, polyalkylène glycol substitué par 0 à 1 r₁₂, carbohydrate substitué par 0 à 1 r₁₂.
   - r₁₂ est un lien avec le chélate ;
   - avec k choisi parmi 0, 1, 2; h choisi parmi 0, 1,2; h' choisi parmi 0, 1,2, 3, 4, 5; h" choisi parmi 0, 1, 2, 3, 4, 5; g choisi parmi 0, 1, 2, 3, 4, 5, 6, 7, 8, 9,10; g' choisi parmi 0, 1, 2, 3, 4, 5, 6, 7, 8, 9, 10; g" choisi parmi 0, 1, 2, 3, 4, 5, 6, 7, 8, 9, 10; g"' choisi parmi 0, 1, 2, 3, 4, 5, 6, 7, 8, 9, 10; s choisi parmi 0, 1, 2, 3, 4, 5, 6, 7, 8, 9,10; s' choisi parmi 0, 1, 2, 3, 4, 5, 6, 7, 8, 9,10; s" choisi parmi 0, 1, 2, 3, 4, 5, 6, 7, 8, 9,10; t choisi parmi 0, 1, 2, 3, 4, 5, 6, 7, 8, 9,10.
6) certains liens décrits dans le document WO02/085908, par exemple une chaîne linéaire ou ramifiée de linkers choisis parmi :
   - CR6"'R7"'-, - (R6"')C=C(R7"')=, -CC-, -C(O)-, -O-, -S-, -SO₂-, -N(R3"')-, - (R6"')C=N-, -C(S)-, -P(OO(OR3"')-, -P(O)-(OR3"')O-, avec R"'3 un groupe capable de réagir avec un azote ou un oxygène
   - Une région cyclique (cycloalkyles divalents, hétérocycles divalents)
   - Des polyalkylènes, polyalkylènes glycols.
7) des linkers du document WO03/011115 pages 124-125 notamment

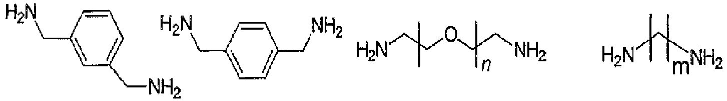

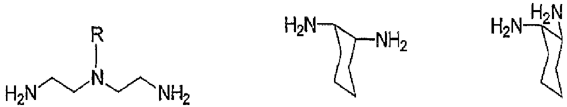

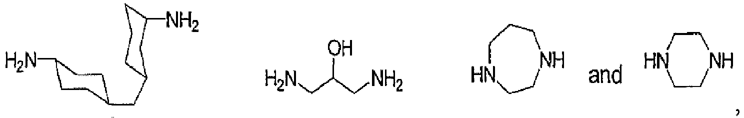

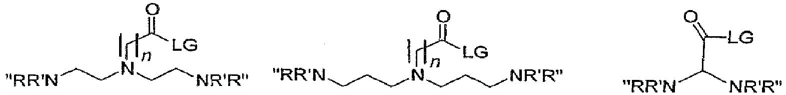

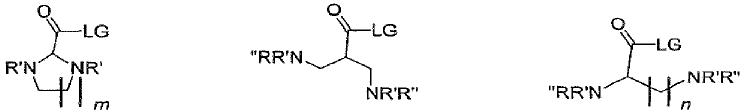

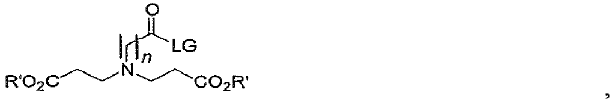

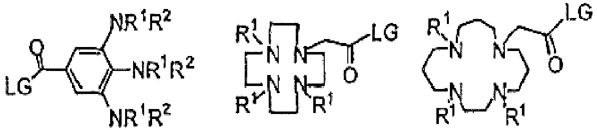

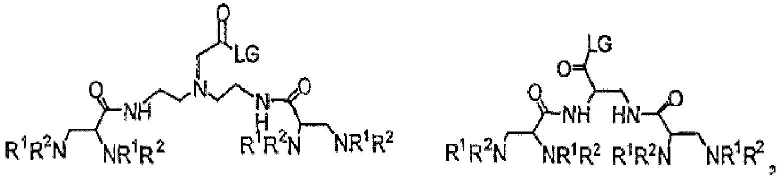

Le choix des liens (structure et taille) pourra se faire notamment de manière à contrôler notamment la charge, la lipophilie, l'hydrophilie du produit en fonction de l'indication diagnostique recherchée, pour optimiser le ciblage biologique, la biodistribution. On pourra en particulier utiliser des linkers biodégradables in vivo, des linkers PEG ou mini-PEG. L'invention concerne en particulier les composés comprenant un linker tel que le composé préparé présente une efficacité équivalente à celle des composés exemplifiés en détail dans la présent demande, l'activité étant mesurée à l'aide de techniques appropriées in vitro, in vivo.

Pour la partie chélate du produit, un grand nombre de chélates peuvent être utilisés.

On pourra utiliser notamment un chélate de formule général suivante : 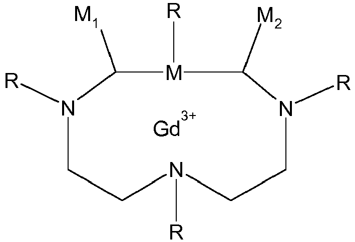 avec :
M-M1-M2 forme un noyau pyridine
ou M1 et M2 sont absents et M représente une liaison
ou M est N-R et M1 et M2 représentent un atome d'hydrogène ou un méthyle avec R est choisi indépendamment parmi CH₂CO₂- ou H ou CHX-CO₂- , avec au moins un R étant CHXCO₂- et X étant L-B ;
et en particulier un chélate linéaire choisi parmi : EDTA, DTPA diéthylènetriaminopentaacétique acide, N-[2-[bis(carboxyméthyl)amino]-3-(4-ethoxyphenyl)propyl]-N-[2-[bis(carboxyméthyl)amino]éthyle]-L-glycine (EOB-DTPA), N,N-bis[2-[bis(carboxyméthyl)amino]éthyle]-L-glutamique acide (DTPA-GLU), N,N-bis[2-[bis(carboxyméthyl)amino]éthyle]-L-lysine (DTPA-LYS), des dérivés mono- ou bis-amide du DTPA, tels que N,N-bis[2-[carboxyméthyl[(méthylcarbamoyl)méthyle]amino]éthyle] glycine (DTPA-BMA), 4-carboxy-5,8,11-tris(carboxyméthyl)-1-phenyl-2-oxa-5,8,11-triazatridecan-13-oic acide (BOPTA),

On pourra utiliser notamment un chélate macrocyclique parmi 1,4,7,10-tetraazacyclododecan-1,4,7,10-tetraacetic acide (DOTA), 1,4,7,10-tetraazacyclododecan-1,4,7-triacetic acide (DO3A), 10-(2-hydroxypropyl)-1,4,7,10-tetraazacyclododecan-1,4,7-triacetic acide (HPDO3A) 2-methyl-1,4,7,10-tetraazacyclododecan-1,4,7,10-tetraacetic acide (MCTA), (alpha, alpha ', alpha ", alpha "')-tetramethyl-1,4,7,10-tetraazacyclododecan- 1,4,7,10-tetraacetic acide (DOTMA), 3,6,9,15-tetraazabicyclo[9.3.1]pentadeca-1(15),11,13-triene-3,6,9-triacetic acide (PCTA).

On pourra aussi utiliser des dérivés dans lesquels un ou plusieurs groupes carboxyliques sont sous forme d'un sel, ester, amide correspondant ; ou un composé correspondant dans lequel un ou plusieurs groupes carboxyliques sont remplacés par un groupe phosphonique et/ou phosphinique tel que 4-carboxy-5,11-bis(carboxyméthyl)-1-phényl-12-[(phénylméthoxy)méthyl]-8-(phosphonométhyl)-2-oxa-5,8,11-triazatridecan-13-oic acide, N,N'-[(phosphonométhylimino)di-2,1-ethanediyl]bis[N-(carboxyméthyl)glycine], N,N'-[(phosphonométhylimino)di-2,1-ethanediyl]bis[N-(phosphonométhyl)glycine], N,N'-[(phosphinométhylimino)di-2,1-ethanediyl]bis[N-(carboxyméthyl)glycine], 1,4,7,10-tetraazacyclododecane-1,4,7,10-tetrakis[méthylen(méthylphosphonique)]acide, or 1,4,7,10-tetraazacyclododecane-1,4,7,10-tetrakis[méthylen(méthylphosphinique)]acide.

On peut aussi utiliser un chélate parmi : DOTA gadofluorines, DO3A, HPDO3A, TETA, TRITA, HETA, DOTA-NHS, M4DOTA, M4DO3A, PCTA et leurs dérivés 2-benzyl-DOTA,alpha- (2-phenethyl) 1,4,7,10 tetraazacyclododecane-1-acetic-4,7,10-tris (méthylacétique) acide, 2benzyl-cyclohexyldiethylenetriaminepentaacetic acide, 2-benzyl-6methyl-DTPA, 6,6"-bis [N, N, N", N"tétra (carboxyméthyl)aminométhyl)-4'- (3-amino-4-methoxyphenyl)- 2,2' : 6', 2"-terpyridine, N,N'-bis-(pyridoxal-5-phosphate) éthylènediamine- N, N'-diacétique acide (DPDP) et éthylènedinitrilotétrakis (méthylphosphonic) acide (EDTP).

De manière plus large, le ou les chélates formant l'entité signal pourront répondre à la formule du document WO01/60416.

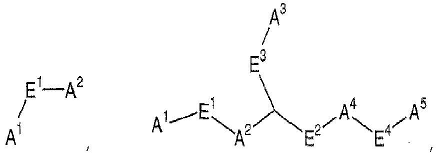

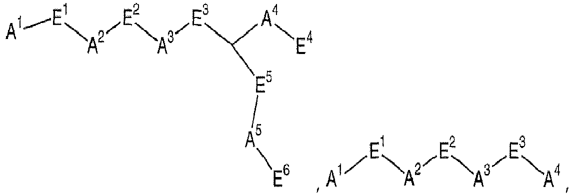

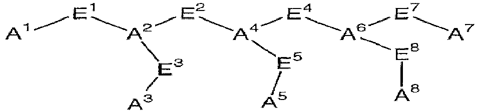

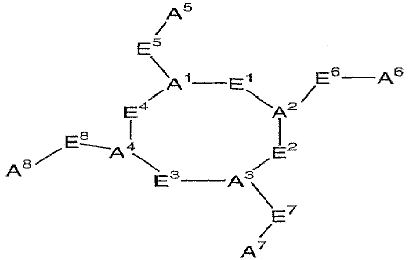

On pourra utiliser en particulier les composés DTPA, DOTA, NOTA, DO3A, et dérivés, notamment : 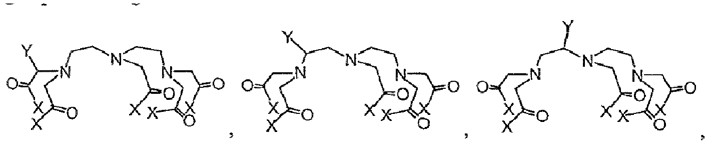 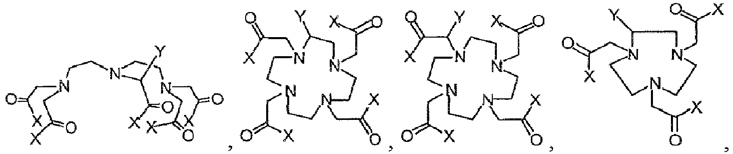 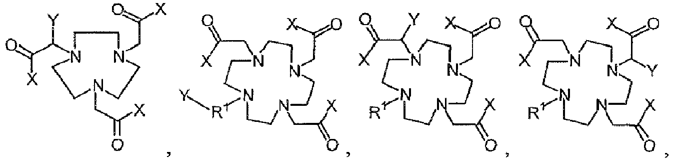 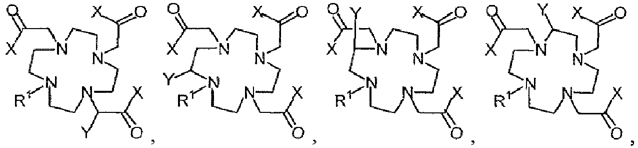 and 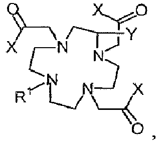 avec X un groupe capable de coordiner un cation métallique, de préférence O-, OH, NH₂, OPO₃-, NHR avec R une chaîne aliphatique.

On pourra en particulier utiliser les chélates désignés P730 du demandeur, en particulier de formule V et VI du document EP661279 (US5919432), notamment 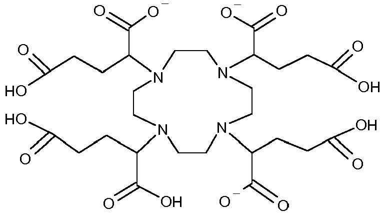 dont la préparation est décrite précisément notamment page 26 à 32, et les chélates à squelette PCTA décrits par le demandeur notamment dans US6440956, que ces chélates ou leurs intermédiaires soient porteurs ou non de chaînes hydrophiles, et en particulier de chaînes aminoalcool courtes ou longues.

On citera aussi les chélates rappelés dans WO03/011115 pages 8 à 11 notamment

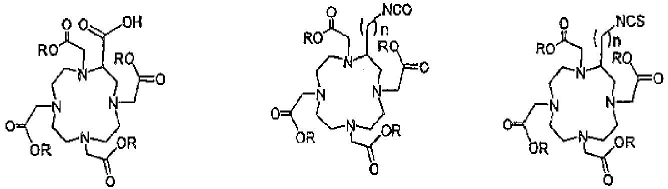

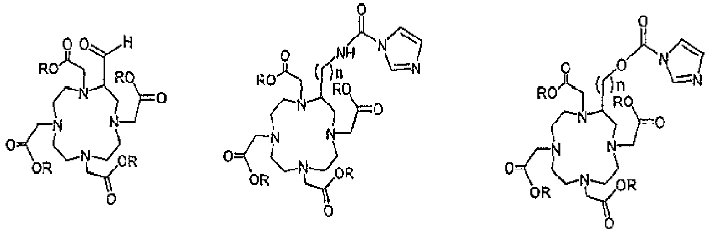

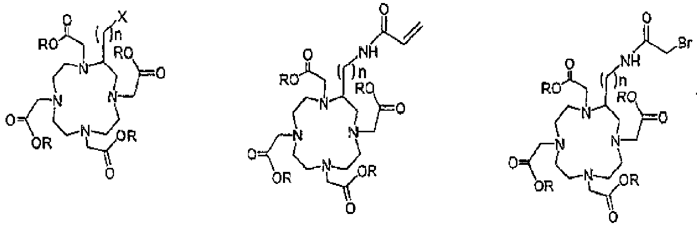

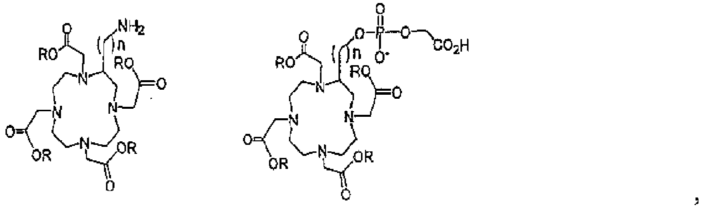

On pourra aussi utiliser des améliorations des composés suivants déjà mentionnés pour le couplage avec le gallium (HED, IDA (imino diacétique acide), desferroxamine

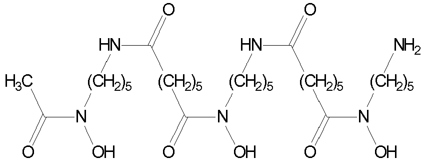

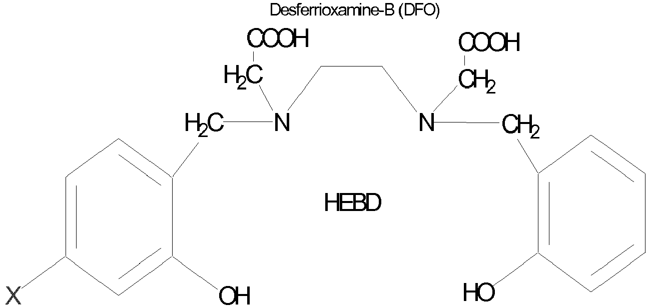

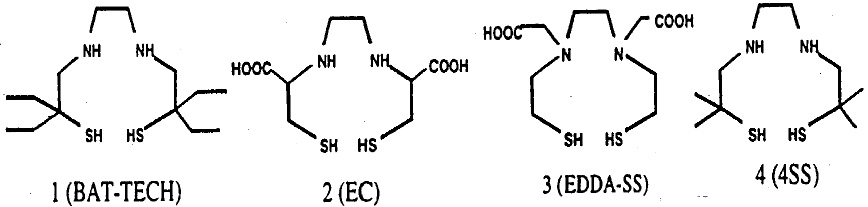

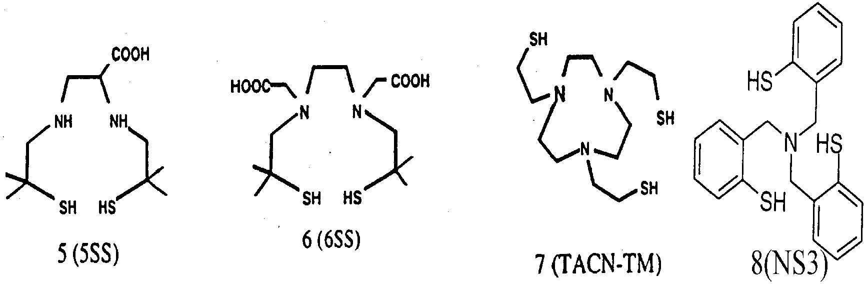

On peut aussi utiliser des chélates décrits dans Inorg. Chim. Acta (1995), 75-82 (TACN-TM (1,4,7-tris(2-mercaptoethyl)-1,4,7-triazacyclononane), Bioconj Chem (2002), 1140-1145 (chélate trithiolate tripode), et de manière générale tout chélate capable de former une cage suffisamment stable autour de Ga³⁺, notamment toute amine aliphatique, macrocyclique ou linéaire, macrocycle amine avec amines tertiaires.

Le demandeur s'est intéressé tout particulièrement à des chélates capables de complexer le gallium et possédant au moins une fonction de couplage du chélate à au moins un biovecteur.
Pour le choix des chélates, on pourra utiliser des chélates particulièrement appropriés pour le couplage du gallium, en particulier des chélates dont la valeur du log K est assez élevée (log K = ML/(M)(L) avec M le métal et L le ligand qui est le chélate ou le conjugué [chélate+biovecteur]), et notamment dont la structure permet de former une loge bien protectrice pour le Ga³⁺. On utilisera en particulier des complexes tels que le log K est de l'ordre de 20 à 40, notamment de 25 à 35. Le cas échéant, on utilisera un macrocycle porteur, en plus des 3 fonctions acides chargées négativement couplées au Ga³⁺, d'au moins un groupe protecteur du Ga³⁺. Par groupe protecteur on entend un groupe capable de protéger ou d'améliorer la coordination du Ga³⁺ avec les fonctions du chélate (fonctions COOH par exemple). Dans la mesure où la quantité de chélate injectée au patient est très faible, on peut utiliser des chélates à structure assez complexe destinée à assurer la protection du Ga³⁺, par exemple par assemblage de chélates linéaires ou macrocycliques.
On choisira en particulier des complexes plus stables que le complexe gallium III - transferrine (log K= 20,3).
Mais dans la mesure où la durée de vie du produit est très courte (environ 1 à 2 heures entre le moment de sa synthèse et la lecture au PET), on pourra choisir des chélates dont le log K est nettement plus faible mais suffisant pour la lecture souhaitée, en prenant en compte le cas échéant des données d'interaction Ga68-transferrine.

Les résultats expérimentaux en imagerie PET au Ga68 montrent l'intérêt d'espacer suffisamment la partie chélate de la partie biovecteur. Pour cela, on peut choisir un linker d'assez grande taille. A l'inverse on peut utiliser des biovecteurs de petite taille mais tels que la reconnaissance du biovecteur par sa cible n'est pas altérée de manière gênante. En particulier selon une réalisation on teste la spécificité de reconnaissance avec des linkers comprenant 1 à 5 acides aminés. On a ainsi étudié des complexes (biovecteur-chélate- Ga³⁺) dont la spécificité pour la cible est meilleure en utilisant le gallium plutôt que le technicium ou l'indium, la différence d'affinité pour la cible étant vraisemblablement due à une différence de conformation du complexe en fonction du radionucléide.
On a aussi étudié des chélates dont une ou plusieurs fonctions non liées au biovecteur sont couplées avec au moins un groupe destiné à améliorer la reconnaissance du biovecteur pour sa cible ; par exemple on utilise un groupe diminuant la clairance rénale, un groupe interagissant avec une cible voisine de la cible biologique (par exemple ce groupe stabilise la structure du site de reconnaissance du biovecteur), un groupe modifiant le pH, la charge ou d'autres paramètres physico-chimiques tels que l'hydrophilie ou la lipophilie, de manière à améliorer l'affinité du biovecteur pour sa cible et/ou à augmenter la modification du biovecteur s'il s'agit d'un substrat pour une enzyme ou un effecteur intra ou extra cellulaire.

Le demandeur a également étudié des systèmes multivalents du type :
- microémulsions (nanoparticules lipidiques) de ciblage spécifique, comprenant des chélates de gallium et un biovecteur de ciblage spécifique, par exemple pour le ciblage d'intégrines
- nanoparticules recouvertes d'une couverture de chélate de Ga68 et de biovecteur ; on citera en particulier des particules superparamagnétiques d'oxydes de fer du type (USPIO et SPIO), et des composés quantum dots, dont la surface est adaptée pour cette couverture du chélate et du biovecteur.

Dans la mesure où la préparation du Ga68 dans le générateur s'accompagne de la libération d'impuretés, le demandeur a aussi étudié des composés dont le chélate est capable de complexer le Ga68 mais pas les impuretés résiduelles éventuelles dans la solution de Ga68 sortie du générateur.
Le demandeur a par ailleurs étudié des formulations des composés vectorisés marqués au gallium. Ces formulations comprennent d'une part au moins un composé vectorisé marqué au gallium, chaque composé vectorisé comportant au moins un biovecteur couplé à au moins un chélate complexant le Ga68, et d'autre part au moins un additif permettant de stabiliser le composé vectorisé. En particulier, compte tenu de la cinétique et de la stabilité de la complexation du Ga68 par le chélate, il peut arriver que du Ga68 ne soit pas complexé de manière optimale par le chélate du composé chélate-biovecteur-Ga68, d'où un risque de Ga68 libre dans le produit injecté au patient.

La formulation administrée au patient comprend à cet effet comme additif de stabilisation un excès de chélate capable de complexer le Ga68 libre. Le chélate en excès peut être le même que le chélate du composé vectorisé, ou un chélate différent à condition qu'il soit approprié pour la complexation du Ga68 libre. Par exemple le chélate du composé vectorisé sera un chélate macrocyclique particulièrement avantageux pour la chimie du gallium, tandis que le chélate en excès dans la formulation sera un chélate linéaire plus simple à fabriquer. Grâce à une telle formulation, on pourra notamment compenser des difficultés de pureté ou de stabilité du produit issues du procédé de préparation rapide retenu. On pourra ainsi utiliser un procédé de couplage du biovecteur et du chélate du produit, par exemple par chauffage, qui soit rapide (inférieur à 20 minutes par exemple) mais qui entraîne un risque de relative instabilité de la complexation du Ga68 par le chélate, ce risque étant toutefois compensé par l'addition de chélate en excès à ce produit. Ainsi, la présente invention concerne également une formulation comprenant un composé selon l'invention (c'est à dire un complexe métallique de gallium Ga68 de formule Ch-L-B ou Ch-Lp-Bq) et un chélate linéaire ou macrocyclique Ch non complexé, avantageusement présent à une concentration de 0,01 à 100mM, avantageusement de 10 à 100mM.
La formulation peut aussi être une formulation ionique notamment de calcium. En effet avantageusement le calcium peut se lier au chélate libre en excès pouvant être présent dans la formulation ou dans l'organisme du patient. Le principe général est de répondre à un problème technique particulier issu de l'utilisation du Ga68 pour la préparation des composés vectorisés, notamment du fait de la volonté de préparer un composé rapidement qui peut ne pas permettre une purification ou une stabilisation optimale du produit. Une telle formulation permettra par exemple de pallier l'existence de sous-produits ou de réactifs en excès tels que des amines, des acides. Ainsi, la présente invention concerne également une composition comprenant un composé de l'invention et du calcium.
On pourra utiliser aussi différents mélanges de formulation par exemple décrits dans WO2005009393 pour la stabilisation face à la radiolyse notamment des biovecteurs (exemples : bloqueurs de radicaux libres, dithiocarbamates, PDTC, composés avec sélénium solubles tels que la sélénométhionine, la sélénocystéine avec le cas échéant du sodium ascorbate, des dérivés capables de réduire des acides aminés oxydés tels que la méthionine, notamment des dérivés thiol de la cystéine, le mercaptoéthanol, le dithiolthréotol). On pourra aussi utiliser des formulations arginine, lysine, polylysines et autres dérivés d'acides aminés cationiques pour limiter la réabsorption rénale. Ainsi, la présente invention concerne également une composition comprenant un composé de l'invention et au moins un agent de stabilisation face à la radiolyse. Le demandeur a également recherché des biovecteurs et des chélates tels que leur couplage avec le gallium soit suffisamment efficace et rapide.
Par exemple le document WO2004/089425 décrit un procédé avec micro-ondes pour une synthèse et une pureté améliorées du produit qui incorpore le biovecteur et le chélate couplé au Ga68. Le demandeur a examiné l'utilisation de biovecteurs susceptibles d'être sensibles au traitement par micro-ondes. En particulier une méthode de screening consiste à tester la stabilité de biovecteurs d'intérêt diagnostique suite à un traitement micro-onde.

Le besoin demeure de développer des procédés et des dispositifs permettant :
- de réduire le temps de préparation et d'améliorer la stabilité du produit en particulier pour des biovecteurs instables au micro-onde
- d'améliorer la pureté du produit
- d'augmenter la concentration du produit
- d'améliorer la sécurité du patient, notamment par une préparation dans des conditions de stérilité appropriées.
Le demandeur s'est ainsi intéressé tout particulièrement à des procédés (thermiques, physico-chimiques, chimiques ...) et des dispositifs, éventuellement déjà utilisés ou utilisables en médecine nucléaire pour d'autres isotopes que le gallium, et dont l'adaptation soit appropriée au cas du Ga68 pour résoudre ces problèmes.
Le Ga68 est obtenu par dégradation du Ge68 dont la durée de vie est de 270 jours. Les générateurs de Ge68 sont basés sur l'utilisation d'une matrice qui absorbe le Ge68, le Ga68 étant élué. Toutefois dans les générateurs connus une étape de concentration du Ga68 est nécessaire car le volume élué est trop dilué ; le demandeur a étudié des moyens pour concentrer l'éluat et/ou pour réduire le volume de l'éluat, notamment en améliorant la matrice utilisée. Le demandeur a aussi étudié le moyen de purifier l'éluat de ses impuretés (Ti⁺ notamment) susceptibles de gêner le couplage avec le chélate, et d'automatiser l'étape de concentration/purification par exemple à l'aide de colonnes de chromatographie, en utilisant un dispositif intégré ou en sortie du générateur. En sortie du générateur, le Ga68 peut être couplé au chélate, le complexe formé étant destiné à être couplé à la partie biovecteur. Selon une variante le chélate a été préalablement couplé au biovecteur afin que cette réaction de couplage n'altère pas la stabilité du chélate avec le gallium. Le cas échéant la préparation du produit comprend une étape d'élimination du gallium en excès qui n'a pas été complexé par le produit. Le cas échéant la solution de Ga68 est rendue moins acide pour faciliter le couplage avec le chélate.

En particulier on a recherché des procédés de couplage, d'une durée de moins de 20 minutes, entre d'une part le Ga68 sorti du générateur et d'autre part le composé biovecteur+chélate, les conditions de couplage étant telles que le taux de complexation du Ga68 par le chélate dudit composé soit de plus de 50, de préférence plus de 70, 80,90%, par exemple :
- chauffage très rapide à haute température sans altérer la structure du biovecteur, puis refroidissement rapide
- pas de chauffage, mais addition d'un catalyseur
- chauffage pendant moins de 10 minutes à 50°C, par exemple entre 70 et 100 °C, un excès de chélate étant ajouté pour chélater le Ga68 non complexé par le composé biovecteur+chélate.
Outre des procédés micro-ondes on peut utiliser tout type de procédé d'activation tel que des procédés à ultrason et argile.
De plus le demandeur a pu obtenir des composés stables et complexant le Ga68 comme décrit en détail plus loin avec un chauffage rapide de l'ordre de 80°C pendant 5 minutes, sans que cela ne nécessite de micro-ondes.
Le demandeur a aussi étudié l'utilisation de chélates appropriés pour un procédé de couplage du chélate et du biovecteur dans des conditions qui ne soient pas optimales mais qui suffisent pour la lecture souhaitée. Par exemple, le procédé de couplage comprend un chauffage à une température plus faible que la température optimale, mais suffisant pour une lecture PET efficace.
On a aussi recherché tout particulièrement des chélates tels que leur couplage avec le Ga68 soit très rapide et ne nécessite pas un traitement physique et/ou chimique altérant le biovecteur. Ceci est destiné à sélectionner des chélates très intéressants sur ce critère de procédé (rapidité du couplage), à condition que leur complexation du gallium soit suffisante pour l'utilisation en imagerie PET du Ga68.
On cherche aussi à optimiser la dose de gallium pour limiter la dose de radiation du patient, tout en ayant une bonne efficacité du produit. La durée d'exposition du patient étant nettement plus courte qu'avec des radionucléides de durée de vie assez longue tel que le technicium ou l'indium, la dose de produit avec gallium injecté peut ainsi être nettement plus élevée qu'avec ces radionucléides. Le demandeur a ainsi étudié des composés efficaces à une dose de radioactivité de l'ordre de 1 à 1000 Curie/mol, notamment 1 à 10, 1 à 100, voire au delà à environ 2000 à 3000 Curie/mol. On pourra aussi faire plusieurs séries de mesure, par exemple à une dose de radioactivité de l'ordre de 0,1 à 1000 Curie/mol par mesure, dans une même session d'imagerie du patient. La radioactivité du produit est par exemple comprise entre 100 et 1000 MBq/nmol.
Selon une réalisation, on utilise un kit comprenant le ligand du gallium, c'est à dire le chélate, à coupler au biovecteur ou déjà couplé au biovecteur. Le radiopharmacien ajoute à ce ligand du Gallium la solution stérile de gallium sortie du générateur.
Pour améliorer la stérilité du dispositif et le cas échéant répondre à des normes CGMP, on peut lyophiliser le ligand (biovecteur couplé au chélate) qui est mis en solution (par exemple à l'aide d'eau stérile et d'un tampon tel que de l'acétate de sodium), solution qui est additionnée par le radiopharmacien à la solution de gallium. D'autres modes de préparation du ligand peuvent être pratiqués, le principe étant d'obtenir une solution de ligand dont les paramètres recherchés (stabilité, réactivité avec le gallium, stérilité ...) sont appropriés pour la complexation du gallium. Par exemple pour des ligands posant des contraintes de faible solubilité dans l'eau avec risque de précipitation, on peut utiliser du DMSO et des excipients appropriés.

Pour ce qui est du générateur lui-même on recherche des dispositifs améliorant la miniaturisation et la stérilité tels que décrit dans WO2005/084168, par exemple avec collecte de la solution de gallium sortie du générateur dans un conditionnement vide préstérilisé, le conditionnement rempli de la solution de Ga68 étant ensuite utilisé avec le kit d'administration comprenant le ligand. En particulier, on cherche à assurer une stérilité du générateur pendant toute sa durée de vie, qui est voisine de celle du Germanium, donc d'environ un an. Des générateurs appropriés pourront aussi être du type Ti 44/SC44.
On étudie aussi des systèmes automatisés et de préférence stériles comprenant par exemple :
- une partie de production du Ga68 (générateur de Ga68 produisant une solution de Ga68, et le cas échéant des moyens de concentration, des moyens de stérilisation de la solution de Ga68, des moyens de collecte de la solution stérile de Ga68)
- une partie de couplage de la solution de Ga68 avec le ligand du Ga68 (biovecteur + chélate) à la sortie de laquelle est récupérée la solution de produit à injecter au patient ; le cas échéant cette partie comprend une solution d'additifs de stabilisation (pH, chélate en excès, groupes protecteurs ...) et des moyens de chauffage/refroidissement ;
- le cas échéant une partie d'administration de la solution de produit prêt à l'injection. Le procédé de préparation inclut de préférence un aspect de radioprotection des personnes, ce qui est obtenu par exemple par l'utilisation de dispositifs de conditionnement et/ou d'injection (seringues par exemple) appropriés.

Concernant plus particulièrement les méthodes d'imagerie des composés étudiés, On pourra par ailleurs coupler l'imagerie PET au Ga68 à certaines modalités spécifiques d'imagerie IRM.
En particulier, la combinaison du PET (ou PET CT avec le PET pour l'imagerie fonctionnelle et le scanner CT pour l'imagerie anatomique) et de IRM permet de combiner la très forte sensibilité du PET (mais avec une résolution moins bonne que l'IRM) à la très forte résolution de l'IRM (mais avec une sensibilité moins bonne que le PET). Pour améliorer le contraste, on pourra administrer de manière simultanée ou différée dans le temps au moins un produit de contraste pour PET, de préférence au Ga68, qui permet de détecter sur le corps entier toutes les zones tumorales et métastases, et au moins un produit de contraste pour IRM destiné à visualiser avec une forte résolution une ou plusieurs zones détectées avec le PET. Dans certaines indications on pourra préférer d'autres isotopes au gallium.
Des procédés automatisés seront particulièrement avantageux pour une telle combinaison d'imagerie, pour optimiser l'administration, la lecture et l'analyse d'images, en utilisant le cas échéant des injecteurs automatiques de produits de contraste. On pourra par exemple en fonction des résultats de la détection au PET zoomer automatiquement vers une ou plusieurs zones d'intérêt repérées avec le PET, avec ou sans injection de produit de contraste. On pourra utiliser par exemple un produit de contraste spécifique vectorisé pour PET au Ga68, et un produit de contraste vectorisé pour IRM au Gadolinium, les biovecteurs étant identiques ou différents, avec par exemple le biovecteur du produit PET étant capable de localiser une angiogénèse dans l'ensemble des tumeurs, et le biovecteur du produit IRM étant capable d'étudier avec précision la localisation et/ou la caractérisation de la progression de zones tumorales spécifiques dans différents territoires biologiques. L'invention concerne ainsi également un produit de contraste au Ga68 permettant de détecter par imagerie PET au moins une zone d'intérêt diagnostique comprenant le complexe ou la composition de l'invention, pour son utilisation dans une méthode d'imagerie comprenant :
a) l'administration d'au moins ledit produit de contraste au Ga68 permettant de détecter par imagerie PET au moins une zone d'intérêt diagnostique
b) l'administration d'au moins un produit de contraste pour IRM ou scanner RX, destiné à analyser spécifiquement la ou les zones d'intérêt diagnostique détectées en a).
Inversement, si un produit de contraste RX ou IRM est efficace dans une indication diagnostique, on pourra affiner le diagnostic à l'aide d'un produit au Ga68. L'invention concerne ainsi également un produit de contraste au Ga68 radiomarqué comprenant le complexe ou la composition de l'invention, pour utilisation dans une méthode d'imagerie comprenant :
a) l'administration d'au moins un produit de contraste pour IRM ou scanner RX capable d'effectuer une première analyse d'une zone d'intérêt diagnostique
b) l'administration d'au moins ledit produit de contraste au Ga68 radiomarqué pour effectuer une seconde analyse de la zone d'intérêt diagnostique ou d'une zone plus large que la zone d'intérêt.
Comme produit de contraste IRM, on pourra aussi utiliser un produit de type BPA (blood pool agent) non vectorisé pour analyser des zones tumorales avec précision grâce à la haute résolution.
Comme produit de contraste IRM, on pourra aussi utiliser des composés de type particules superparamagnétiques, USPIO notamment, et notamment tous ceux indiqués dans la demande WO2004058275 et la demande WO2005046563 (pages 5 à 9), et en particulier comportant un noyau d'oxyde de fer recouvert de dextran ou de tout dérivé du dextran.
Comme produit de contraste au scanner CT on pourra utiliser des radio isotopes pour CT, des produits iodés connus tels qu'un monomère, un dimère ionique ou non ionique, un mélange d'un monomère et d'un dimère non ionique. On pourra aussi utiliser des produits de type liposomes de produits RX ou IRM, tels que de iohexol, de gadotéridol, décrits notamment dans Investigative radiology, vol 41, n°3, 339-348, 2006. Selon une réalisation on préparera une composition de diagnostique comprenant au moins un agent de contraste au gallium détectable en PET tel que décrit dans la demande et au moins un agent de contraste détectable en IRM ou au moins un agent de contraste détectable au scanner CT. Selon une réalisation on utilisera en association un produit superparamagnétique du type USPIO et un produit utilisable pour les rayons X tel qu'un monomère ou un dimère iodé ionique ou non ionique.
Avantageusement, on peut aussi utiliser en co-administration (simultanée ou différée dans le temps) un agent capable d'augmenter l'accessibilité à la tumeur d'un produit de contraste en particulier au Ga68 et/ou la durée de passage du produit de contraste dans la tumeur. De tels agents peuvent agir par exemple en augmentant la perméabilité vasculaire dans la zone tumorale (périphérique et/ou intra-tumorale), de manière à davantage faire pénétrer le produit de contraste dans la tumeur, ou en diminuant la pression des fluides interstitiels dans la tumeur (le produit de contraste a davantage de temps pour pénétrer à l'intérieur de la tumeur). On utilise en particulier des agents dits furtifs capables d'agir très rapidement sur ces mécanismes, et éliminés rapidement de leurs zones d'action ce qui permet à la fois une amélioration du diagnostique et une non toxicité.
On peut aussi coupler l'imagerie PET tumorale à une imagerie de diffusion ou de perfusion étudiant les différences de diffusion ou la perfusion de composés ou de diffusion de l'eau dans les zones malades/saines, en faisant intervenir le cas échéant des produits de contraste PET ou IRM.
On aura par exemple les étapes suivantes :
- éventuelle première mesure au scanner ou à l'IRM, avant l'injection de produit de contraste
- mesure au PET suite à l'injection d'un produit de contraste au Gallium
- analyse des données du PET pour identifier des zones d'intérêt, notamment des zones pathologiques pour lequel le diagnostic pourra être plus approfondi
- de manière programmée ou en fonction de cette analyse, injection de produit de contraste au gadolinium pour IRM, dans une ou plusieurs zones d'intérêt
- mesure à l'IRM suite à l'injection de produit de contraste pour IRM
- éventuelle nouvelle administration de produit de contraste.
Pour optimiser le débit et la dose de produit de contraste à injecter, on pourra utiliser et paramétrer un injecteur automatique qui est capable d'intégrer les résultats de l'analyse des données d'une mesure préalable effectuée avec ou sans produit de contraste et d'optimiser le débit et la dose de produit de contraste.
Plus précisément on utilisera par exemple une installation PET/IRM ou PET/CT ou PET/CT/IRM comprenant :
- un module PET capable de faire des mesures PET
- le cas échéant un module IRM capable de faire des mesures IRM
- le cas échéant un module CT capable de faire des mesures CT
- un module d'analyse comprenant d'une part un module de réception des données du signal émis par le module de mesure (PET, IRM, scanner RX ...) suite à une mesure avec ou sans administration de produit de contraste, et d'autre part un module de traitement de ces données capable de convertir ces données en un signal de commande d'un ou plusieurs injecteurs en leur donnant des instructions concernant les paramètres d'administration de produit de contraste
- un ou plusieurs injecteurs comprenant un module de réception des instructions du module d'analyse, et un module d'injection commandé par le module de réception et relié au patient pour lui administrer la dose de produit de contraste adapté.

Parmi les données analysées par le module d'analyse, on citera les courbes de rehaussement et les mesures de relaxivité liées à l'indication diagnostique étudiée, des paramètres physico-chimiques, des données biologiques du patient susceptibles d'avoir un impact sur l'injection.

L'analyse peut se faire par exemple en regard de modèles comportementaux des produits de contraste en fonction de la catégorie de produit utilisé, et le cas échéant à l'aide d'indices de confiance associés à des cartes paramétriques calculées.
Ces données sont par exemple comparées à une base de données interne de l'installation exploitant les résultats obtenus par un logiciel de compilation de données cliniques.
On peut aussi étudier combiner des informations anatomiques IRM ou CT à des informations d'imagerie fonctionnelle PET lors de l'analyse d'image, l'analyse étant rapportée au volume de l'anomalie.

Selon un aspect l'invention décrit ainsi un système automatisé comprenant un dispositif de préparation d'une solution injectable de produit de contraste au gallium, une partie d'administration de l'administration de ladite solution, et le cas échéant des moyens de commande de cette administration en fonctions de données biologiques ou de données d'imagerie du patient.
L'invention concerne une installation d'imagerie médicale (et le procédé d'imagerie associé) comprenant :
- un dispositif d'imagerie capable d'effectuer des mesures de paramètres d'imagerie avec ou sans injection de produit de contraste
- un dispositif d'injection de produit de contraste relié au patient, le dispositif pouvant être un injecteur automatique à seringues ou à poche
- le cas échéant un dispositif d'analyse et de commande du dispositif d'injection en fonction d'une première mesure effectuée suite à l'administration d'un produit de contraste.
Le produit de contraste étant un produit radiomarqué au gallium 68 comprenant le complexe de l'invention ou la composition de l'invention.
Le dispositif est de type PET/CT, PET/IRM ou PET/CT/IRM.
Le cas échéant on effectue une mesure IRM et ou au scanner CT à l'aide d'un produit de contraste approprié, puis une mesure PET avec le produit au gallium.

La description détaillée qui suit décrit des exemples de composés appropriés notamment des complexes métalliques de gallium de formule : 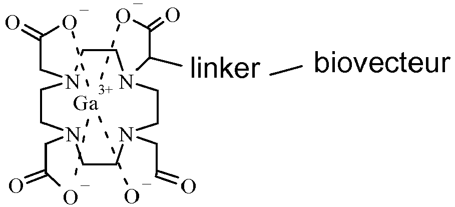 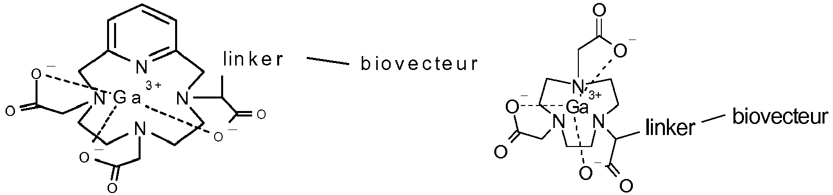 avec :
- biovecteur une molécule pharmacologiquement active, notamment un peptide.
- Linker choisi parmi : (CH₂)ₙ, (CH₂)ₙ-CO-, PEG 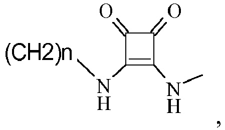 squarate couplé avec des PEG, avec n=1 à 5, avantageusement n=2 à 5, n=4 ou 5, de manière à obtenir avantageusement plusieurs longueurs et types de linkers intéressants pour la physioco-chimie et/ou la biodistribution.
L'invention concerne un complexe métallique de gallium Ga 68 de formule Ch-L-B (chélate - linker - biovecteur), ou Ch-Lp-Bq avec p=q= 2 à 5, Ch étant un chélate ayant la formule générale suivante : 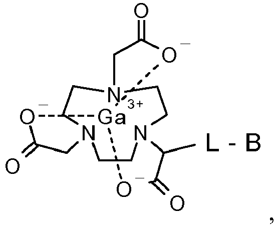 dans laquelle :
1) B est un biovecteur de ciblage d'une cible biologique associée à une pathologie, choisi parmi des enzymes (métalloprotéases, COX, tyrosine kinase), des récepteurs cellulaires VEGF, KDR, CXC, LHRH, GPIIb/IIIa, bombésine/GRP, gastrine, VIP, CCK, GLUT, folate.
2) L est un linker choisi parmi :
   a) P1-1-P2, avec P1 et P2 identiques ou différents choisis parmi O, S, NH, CO₂, -NHCO, CONH, NHCONH, NHCSNH, SO₂NH- NHSO₂-, et 1 représente un alkyle (avantageusement en C₁-C₁₀), alkoxyalkyle (avantageusement en C₁-C₁₀), polyalkoxyalkylène, alkyle interrompu par un ou plusieurs squarate ou par un ou plusieurs aryles, avantageusement phényle, alcényle (avantageusement en C₁-C₆), alcynyle (avantageusement en C₁-C₆), alkyle interrompus par un ou plusieurs groupes choisis parmi -NH-, -O-, -CO-, -NH(CO)-, -(CO)NH-, -O(CO)-, ou -(OC)O- ;
   b) (CH₂)ₙ, (CH₂)ₙ-CO-, -(CH₂)ₙNH-CO- avec n = 2 à 10 , (CH2CH20)q(CH2)r-CO- , (CH₂CH₂O)_{q}(CH₂)ᵣ-NH-CO- avec q = 1-10 et r=2-10, (CH₂)ₙ-CONH - , (CH₂)ₙ-CONH - PEG, (CH₂)ₙ-NH-, 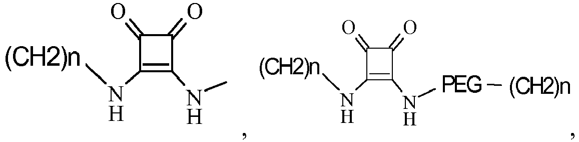 avec n=1 à 5 et avantageusement n=4 ou 5, HOOC-CH₂-O-(CH₂)₂-O-(CH₂)₂-O-CH₂-COOH ; HOOC-(CH₂)₂-CO₂-(CH₂)₂-OCO-(CH₂)₂-COOH, HOOC-CH(OH)-CH(OH)-COOH; HOOC-(CH₂)ₙ-COOH; NH₂-(CH₂)ₙ-NH₂, avec n=0-20; NH₂-(CH₂)ₙ-CO₂H ; NH₂-CH₂ - (CH₂-O-CH₂)ₙ-CO₂H avec n=1 à 10.

Par le terme « groupe alkyle, avantageusement en C₁-C₁₀ », on entend au sens de la présente invention tout groupe alkyle avantageusement de 1 à 10 atomes de carbones, linéaires ou ramifiés, en particulier, les groupes méthyle, éthyle, n-propyle, iso-propyle, n-butyle, iso-butyle, sec-butyle, t-butyle, n-pentyle, n-hexyle. Avantageusement il s'agit d'un groupe méthyle.
Par le terme « groupe alcényle, avantageusement en C₂-C₆ », on entend au sens de la présente invention tout groupe alcényle avantageusement de 2 à 6 atomes de carbones, linéaires ou ramifiés, en particulier, le groupe vinyle.

Par le terme « groupe alcynyle, avantageusement en C₂-C₆ », on entend au sens de la présente invention tout groupe alcynyle avantageusement de 2 à 6 atomes de carbones, linéaires ou ramifiés, en particulier, le groupe éthynyle.
Par le terme « groupe alkoxy avantageusement en C₁-C₁₀ », on entend au sens de la présente invention tout groupe alkoxy avantageusement de 1 à 10 atomes de carbones, linéaires ou ramifiés, en particulier, le groupe OCH₃.
Par le terme « groupe aryle », on entend au sens de la présente invention un ou plusieurs cycles aromatiques ayant 5 à 8 atomes de carbones, pouvant être accolés ou fusionnés, éventuellement substitués par des atomes d'halogène, des groupes alkyles tels que définis ci-dessus ou le groupe nitro. En particulier, les groupes aryles peuvent être des groupes monocycliques ou bicycliques, de préférence phényle, naphthyle, tetrahydronaphthyle ou indanyle. Avantageusement il s'agit d'un groupe phényle.
Par le terme de « groupe hétéroaryle », on entend au sens de la présente invention tout groupe aromatique hydrocarboné de 3 à 9 atomes contenant un ou plusieurs hétéroatomes, tels que par exemple des atomes de soufre nitrogène ou oxygène, et pouvant porter un ou plusieurs substituants, comme par exemple, un groupe alkyle en C₁-C₇ tel que défini ci-dessus, un groupe alcényle en C₂-C₇ tel que défini ci-dessus, ou un halogène. Des exemples de groupes hétéroaryle sont les groupes furyle, isoxazyle, pyridyle, pyrimidyle. On entend par polyalkoxyalkylène un polyalkoxy (C₂-C₃) alkylène (i.e polyoxyéthylènes et polyoxypropylènes), notamment le polyéthylène glycol PEG et ses monoéthers et monoesters en C₁ à C₃, de masse moléculaire de préférence de 1000 à 2000.
On entend par l'expression « molécule pharmacologiquement active associée à une pathologie » notamment tout biovecteur capable de reconnaître spécifiquement une cible biologique dont l'expression est modifiée dans une zone pathologique par rapport à l'état non pathologique, ce qui inclut tous les biovecteurs cités dans la demande.

Selon des réalisations, (L - B) représente CH₂-CH₂ - CONH - Biovecteur ou CH₂-CH₂ - NHCO - Biovecteur, les biovecteurs B étant identiques ou différents entre eux.

Selon des réalisations, les composés selon l'invention comprennent au moins un groupe capable de modifier l'hydrophilie ou la biodistribution des composés, ou de masquer le chélate de manière à ne pas altérer la reconnaissance de la cible biologique.

### Exemples

### PARTIE I : Exemples de chélates macrocycliques et de biovecteurs couplés à des chélates.

### Exemple de référence 1 : chélate squelette DOTA

### Etape 1 : Ester benzylique de l'acide 5-(1,3-Dioxo-1,3-dihydro-isoindol-2-yl)-2-(1,4,7,10tétraaza-cyclododéc-1-yl)-pentanoique

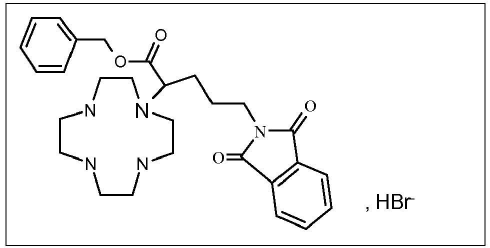

55 g de cyclen base (320 mmol) sont dissous dans 550 mL de CH₃CN, auxquels sont ajoutés goutte à goutte 119.8 g de dérivé bromé (2-Bromo-5-(1,3-dioxo-1,3-dihydro-isoindol-2-yl)-pentanoique acide benzyl ester, 288 mmol) dissous dans 550 mL de CH₃CN. Le milieu est agité à température ambiante 1 nuit. Le précipité est filtré et lavé abondamment à l'acétonitrile. Obtention de 138 g de produit sous forme d'une poudre blanche (rendement corrigé 81,3 %).
CCM : CH₂Cl₂/ MeOH / NH₄OH à 25% (80/40/3)

### Révélation UV et CuSO₄

### Rf : 0,3

### Etape 2 : Ester benzylique de l'acide 5-(1,3-Dioxo-1,3-dihydro-isoindol-2-yl)-2-(4,7,10-tris-éthoxycarbonylméthyl-1,4,7,10tétraaza-cyclododéc-1-yl)-pentanoique

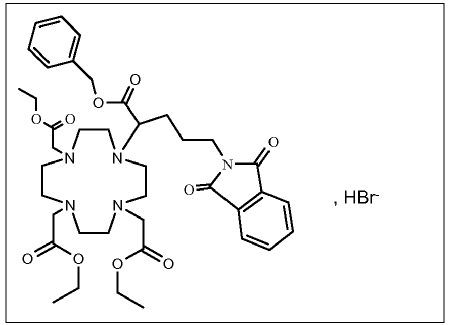

A une solution de 59.1 g d'éthylbromoacétate (Aldrich®, 358 mmol) dans le CH₃CN (1.1 1), sont additionnés 60 g du composé obtenu à l'étape 1 (102 mmol) et 50.1 g de Na₂CO₃ (464 mmol). Le milieu réactionnel est chauffé à 80°C sous couverture d'argon pendant une nuit. Après élimination du précipité, le filtrat est concentré et lavé abondamment au CH₃CN. Le produit est cristallisé dans CH₃CN par ajout goutte à goutte d'Et₂O. Obtention de 89,8 g de produit sous forme d'un solide blanc (rendement corrigé 100 %).

CCM : CH₂Cl₂ / MeOH (9/1)

### Révélation UV et KMnO₄

### Rf : 0,4

### Etape 3 : Acide 5-Amino-2-(4,7,10-tris-carboxyméthyl-1,4,7,10tétraaza-cyclododéc-1-yl) -pentanoique

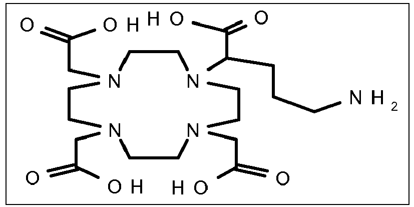

Dans le réacteur de 5 litres, on chauffe au reflux une solution de 54 g de composé obtenu à l'étape 2 (64 mmol) dans l'acide chlorhydrique 37 % (1,8 1), pendant une nuit. Après refroidissement et filtration, le filtrat est concentré et purifié sur silice silanisée (élution à l'eau). Après évaporation sous pression réduite, le produit est lavé à l'éther. Obtention de 45 g de produit sous forme d'un solide blanc. Le produit est désalifié par passage sur résine OH On isole 30 g de produit sous forme de cristaux blancs (rendement 100 %).

### HPLC : Hypercarb® 5µ, 200X4.6, 250Å

Solvant A : acide sulfurique 0,037 N
Solvant B : CH₃CN
Détection UV à 201 nm

### Tr : 18 min.

### Etape 4 : Complexe de gallium de l'Acide 5-Amino-2-(4,7,10-tris-carboxyméthyl-1,4,7,10tétraaza-cyclododéc-1-yl) -pentanoique

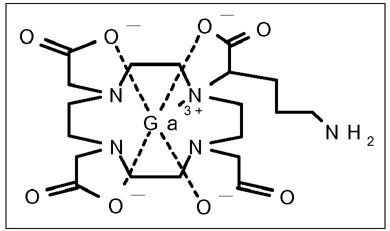

1 mg du composé obtenu à l'étape 3 (2,17 nmol) sont dissous dans 7 µL d'eau et le pH est ajusté à 5,5 par ajout d'acide chlorhydrique 6 N. On ajoute 0,920 mg de Gallium Nitrate nonahydrate (2,2 µmol), et chauffe à 80°C le milieu réactionnel. Le pH de la solution doit être maintenu vers 5 par microajout d'acide chlorhydrique 6 N. Après deux heures, le pH se stabilise. Le léger trouble est filtré sur filtre Whatman® et le filtrat est concentré. Obtention de 1,2 mg de produit sous forme de paillettes blanches (rendement corrigé 100 %).
HPLC : Hypercarb® 5µ, 200X4.6, 250Å
Solvant A : acide sulfurique 0.037 N
Solvant B : CH₃CN

### Détection UV à 201 nm

### Tr : 10 min.

### Etape 5 : Complexe de gallium de l'Acide 5-(2-Ethoxy-3,4-dioxo-cyclobut-1-énylamino)-2-(4,7,10-tris-carboxyméthyl-1,4,7,10tétraaza-cyclododéc-1-yl)-pentanoique

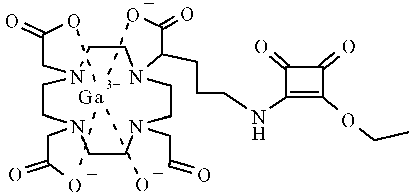

1 mg de composé obtenu à l'étape 4 sont séchés par distillation azéotropique au toluène, puis mis en suspension dans 5 µl de DMSO anhydre sous couverture d'argon. 0,5 µl de Et₃N séchée sur tamis et 0,8 mg de diéthylsquarate (Aldrich®, 2.5 éq.) sont ensuite ajoutés. Le milieu est agité à température ambiante sous couverture d'argon pendant 1 heure. Le mélange est précipité dans l'éther. Le solide obtenu est filtré, puis lavé au dichlorométhane. Après filtration et séchage, 0.98 mg d'un solide blanc (rendement de 81 %) sont récupérés.
HPLC : Symmetry C18, 5µ, 250X4.6, 100Å
A : eau TFA, pH = 2.7
B : CH₃CN

### Détection à 201 et 254 nm

### Tr : 19,8 min.

### Exemple de référence 2 : Couplages des peptides :

| Séquence | P M | m en mg |
|---|---|---|
| Asp(tBu)-Ala-His(trt)-Ser(tBu)-Phe-Ser(tBu)OH | 1073,31 | 172 |
| Leu-Ile-Lys(Boc)-Lys(Boc)-Pro-Phe-OH | 945,22 | 151 |
| Pro-Gly-Asp-(tBu)-Leu-Ser(tBu)-Arg(Pbf)-OH | 1008,25 | 161 |
| Gly-Asp(tBu)-Ala-His(trt)-Ser(tBu)-Phe-Ser(tBu)OH | 1130,36 | 180 |
| γ-Abu- Asp(tBu)-Ala-His(trt)-Ser(tBu)-Phe-Ser(tBu)OH | 1158,42 | 185 |
| 8-Amino-3,6-dioxaoctanoyl- Asp(tBu)-Ala-His(trt)-Ser(tBu)-Phe-Ser(tBu)OH | 1218,47 | 195 |

### Etape 1 : Couplage des peptide N° 1, 2, 3, 4, 5 ou 6 sur le dérivé squarate

Le composé obtenu à l'étape 5 de l'exemple 14 (100µg, 1,53 10⁻⁷ mole) est dissous dans 15 µl de solution aqueuse de Na₂CO₃ pH 9,4. Le peptide protégé (1,6 10⁻⁷ mol) est introduit en maintenant le pH à 9,4 par ajout de Na₂CO₃. Si le peptide n'est pas soluble dans l'eau quelques microgouttes de DMF sont ajoutées jusqu'à dissolution complète. Après 48h de réaction à température ambiante, le milieu est précipité dans un mélange éthanol/éther éthylique. Le précipité est filtré puis séché.

### Etape 2 : déprotection

Le composé obtenu à l'étape 1 est dissous dans un mélange de 10 µl de TFA/TIS/H₂O dans les proportions 90/5/5. Le milieu est agité 5h à température ambiante puis le solvant est évaporé sous pression réduite. Le résidu est repris dans l'éther éthylique et le précipité est filtré puis séché. Le produit est ensuite purifié par HPLC préparative sur une colonne Symmetry® avec un éluant constitué d'eau/TFA PH 3/ CH₃CN

| N° | Structure | PM |
|---|---|---|
| 1 | 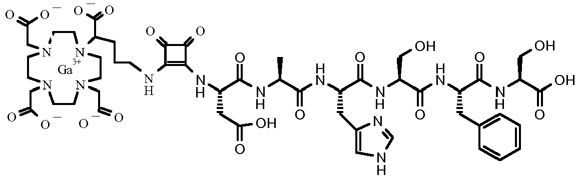 | 1267,9 |
| 2 | 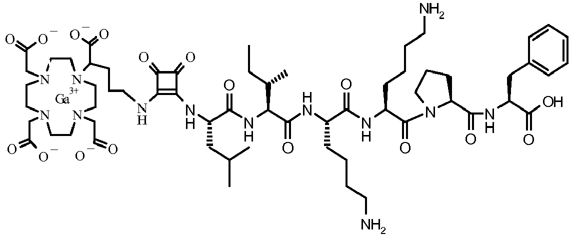 | 1350,22 |
| 3 | 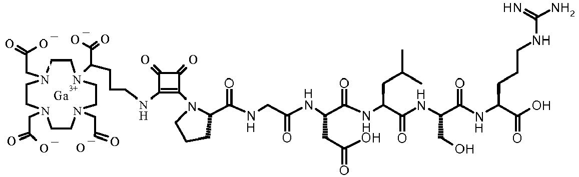 | 1248,94 |
| 4 | 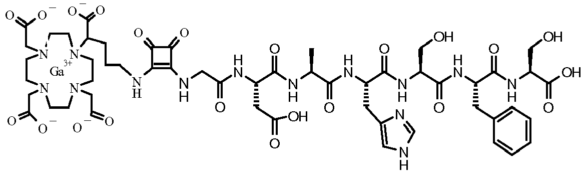 | 1324,95 |
| 5 | 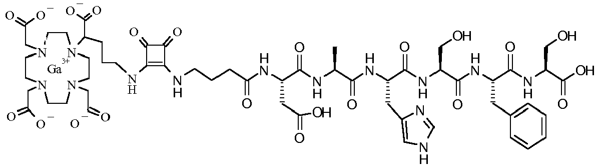 | 1353,00 |
| 6 | 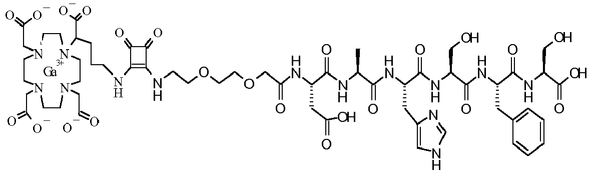 | 1413,06 |

### Exemple de référence 3a : squelette PCTA

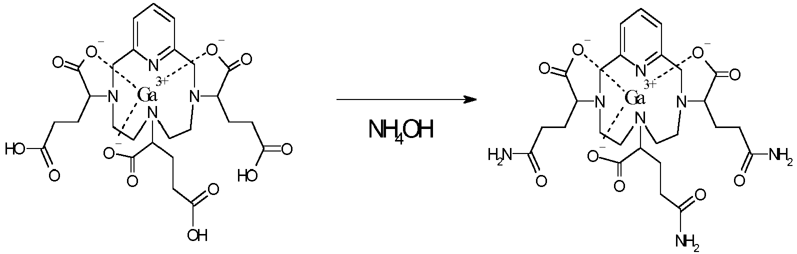

On prépare une solution contenant 0,10 mmol d'ammoniac dans 20 µL d'eau. On ajuste le pH à 6 avec HCl. On ajoute à la solution précédente 1,8 mg de complexe de gallium de l'acide 3,6,9,15-tetraazabicyclo[9,3,1]pentadeca-1(15),11,13-triene-3,6,9-tri(□-glutarique), 0,2 mg d'HOBT, 2.5 mg d'EDCI et 15 µL de dioxane. On ajuste le pH à 6. Après 24h, on concentre le milieu réactionnel à environ 7mL. On précipite le milieu réactionnel dans 70 µL d'éthanol + 20 µL d'éther. Le solide est filtré puis purifié par chromatographie sur silice silanisée RP2 en éluant à l'eau. On obtient 1,2 mg de produit.

### Exemple de référence 3b : squelette DO3A

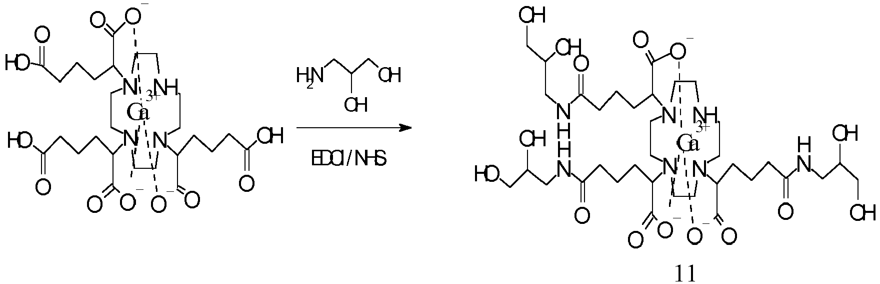

On prépare une solution contenant 0,26 mg de 3-amino-propane-1,2-diol dans 6µL d'eau. On ajuste le pH à 6 avec HCl. On ajoute à la solution précédente 0,5 mg de complexe de gallium de l'acide 2-[4,7-Bis(1,4-dicarboxy-butyl]-1,4,7,10tetraaza-cyclododec-1-yl]-hexanoïque. On ajuste à nouveau le pH avant d'ajouter 0,071 mg de sulfo-NHS et 0,062 mg d'EDCI. Le pH est contrôlé et ajusté à 6 avec NaOH 2N. Après une nuit à TA, le milieu réactionnel est concentré à environ 2 mL puis précipité dans 10ml d'éthanol. Le solide est filtré, lavé à l'éthanol et l'éther diéthylique puis purifié sur silice silanisée RP2 avec une élution à l'eau uniquement. On obtient 0,2 mg de produit.
Les chaînes aminoalcool de cet exemple 3b sont un exemple de groupes hydrophiles pouvant améliorer l'hydrophilie et le cas échéant le masquage du chélate décrit dans la demande.

### Exemple 4 : squelette NOTA

### Complexe de Gallium de l'Acide2-(4,7-Bis-carboxymethyl-[1,4,7]triazonan-1-yl)-5-(2-éthoxy-3,4-dioxo-cyclobut-1-énylamino)-pentanoique

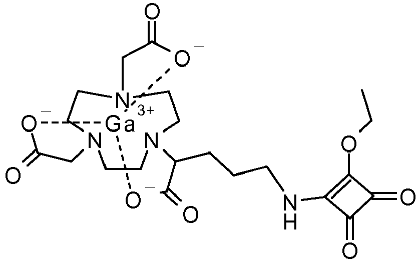

Ce composé est préparé selon le schéma de synthèse suivant au départ de triaza-1,4,7-cyclononane commercial selon les modes opératoires décrits à l'exemple 1.

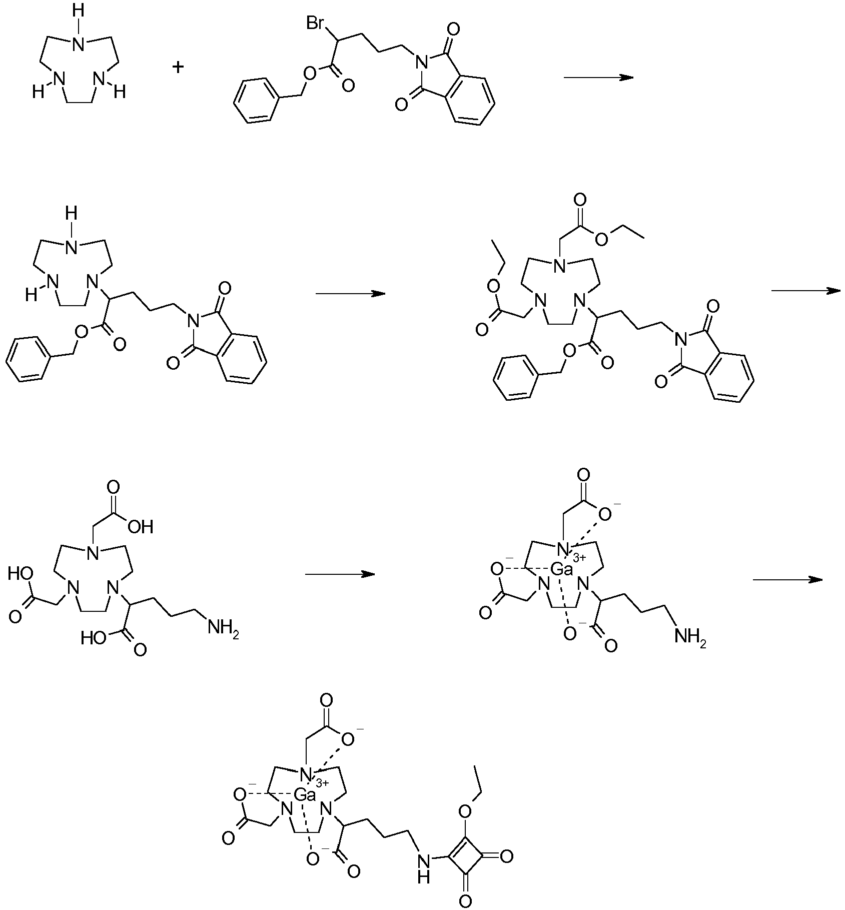

### Exemple 5

Le composé obtenu à l'exemple précédent est couplé selon le mode opératoire décrit à l'étape 1 de l'exemple 2 à une série de peptides dont la séquence est donnée à l'exemple 2. La déprotection est conduite selon la méthode décrite à l'étape 2 de l'exemple 2.

| N° | Structure | PM |
|---|---|---|
| 1 | 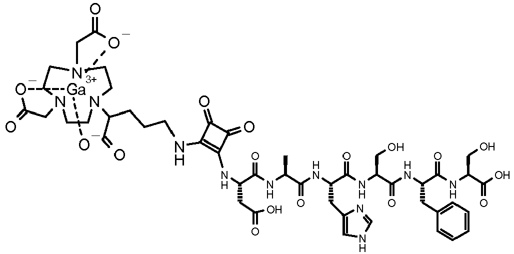 | 1167,80 |
| 2 | 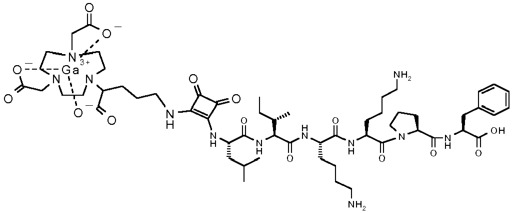 | 1250,12 |
| 3 | 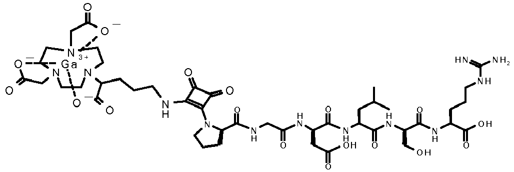 | 1148,84 |
| 4 | 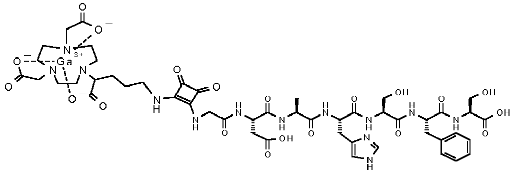 | 1224,85 |
| 5 | 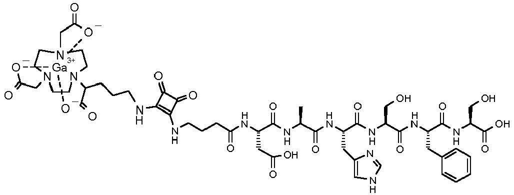 | 1252,91 |
| 6 | 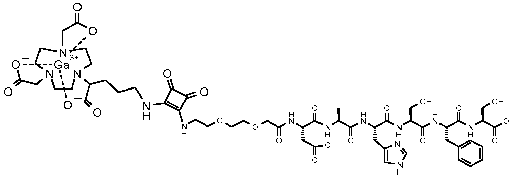 | 1312,96 |

### Exemple de référence 6

### Etape 1 : Ester diéthylique de l'acide 2-(3,6,9,15-Tétraaza-bicyclo[9.3.1] pentadéca-1(15),11,13-trién-3-yl)-pentanedioique

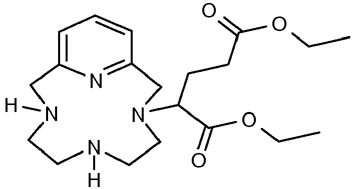

10 mg de 3,6,9,15-Tetraaza-bicyclo[9.3.1]pentadéca-1(14),11(15),12-triéne (48,5 µmol) sont dissous dans un mélange eau-acétonitrile (170 µL d'acétonitrile et 7 µL d'eau). Après ajout de 10 µL de TEA (1,4eq) le mélange est chauffé à 50°C puis 13 mg de bromoglutarate d'éthyle (1eq) sont ajoutés. Le milieu est agité 18h à 50°C.
L'acétonitrile est évaporé et le résidu repris au dichlorométhane est lavé à l'eau (100 µmL). La phase organique est séchée sur sulfate de magnésium anhydre et évaporée à sec. Huile brune. Rendement 58%

SM: 393.20 en ES+

### Etape 2: Ester diéthylique de l'acide 2-(6,9-Bis-éthoxycarbonylmethyl-3,6,9,15-tétraaza-bicyclo[9.3.1 ]pentadéca-1(15),11,13-trién-3-yl)-pentanedioique

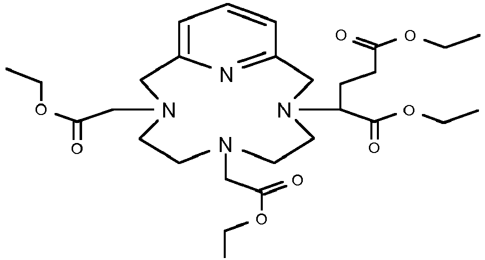

9,5 mg du composé obtenu à l'étape 1 (24 µmol) sont mis en solution dans 200 µL d'acétonitrile avec 8.2 mg de Na₂CO₃ (4,5 eq). Le mélange est porté au reflux et le bromoacétate d'éthyle (9,8 mg , 3.4 eq) est ajouté rapidement. Le milieu réactionnel est porté à reflux pendant 18h. Les sels sont filtrés et le solvant évaporé. Huile brune. Rendement quantitatif. SM : 565 en ES+

### Etape 3 : acide 2-(6,9-Bis-carboxyméthyl-3,6,9,15-tétraaza-bicyclo[9.3.1]pentadéca-1(15),11,13-trién-3-yl)-pentanedioique

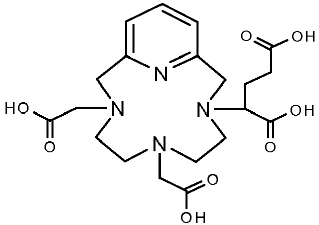

15 mg du composé obtenu à l'étape 2, (26,5 µmol) sont dissous dans 40 µL d'éthanol et 53 µL de soude 5N sont ajoutés goutte à goutte rapidement. Le mélange est porté à reflux pendant 24h. Le milieu réactionnel est dilué 10 fois à l'eau puis le pH est ajusté à 6.5 par addition de résine faiblement acide. La résine est filtrée et lavée à l'eau. Une résine fortement basique est ajoutée au filtrat puis filtrée et lavée deux fois à l'eau. Le produit est élué avec de l'acide acétique 50%. Après évaporation à sec, le produit est obtenu sous la forme de cristaux bruns. m= 8 mg rendement = 60%.
SM : 453,2 en ES+

### Etape 4: Complexe de Gallium de l'acide 2-(6,9-Bis-carboxyméthyl-3,6,9,15-tétraaza-bicyclo[9.3.1]pentadéca-1(15),11,13-trién-3-yl)-pentanedioique

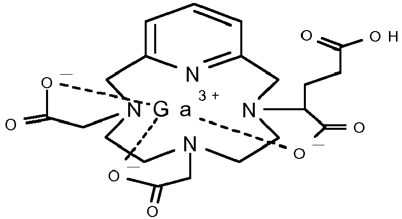

Selon le mode opératoire de l'étape 4 de l'exemple 1. Poudre blanche. Rendement quantitatif. SM : 518 en ES⁻

### Exemple de référence 7

Le composé obtenu à l'exemple précédent est couplé selon le mode opératoire décrit à l'exemple 2 à une série de peptides dont la séquence est donnée à l'exemple 2. La déprotection est conduite selon la méthode décrite à l'étape 2 de l'exemple 2.

| N° | Structure | PM |
|---|---|---|
| 1 | 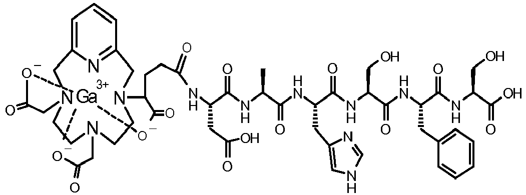 | 1163,81 |
| 2 | 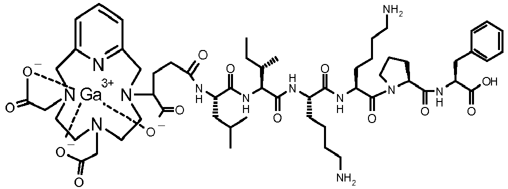 | 1246,13 |
| 3 | 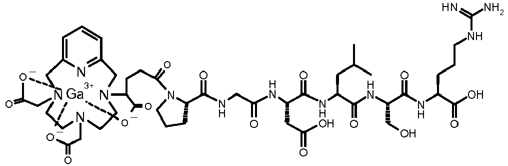 | 1144,85 |
| 4 | 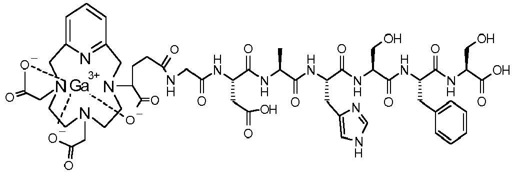 | 1220,86 |
| 5 | 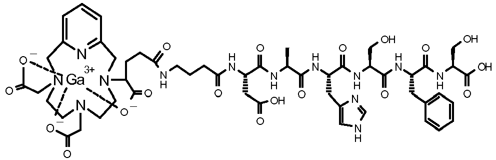 | 1248,92 |
| 6 | 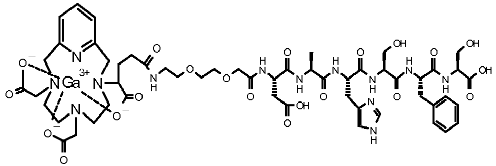 | 1308,97 |

### Exemple de référence 8: (4,7,10-Tris-tert-butoxycarbonylmethyl-1,4,7,10tetraaza-cyclododec-1-yl)-acetic acid

### Etape 1 :

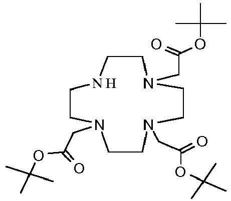

Mw = 514,71 g/mol

73 g de cyclen base (0,42 mol) et 108 g d'acétate de sodium (soit 1,3 mol) sont agités dans 1,2 L de Diméthylacétamide (DMAC), sous argon, à température ambiante pendant une demi heure. 256g de bromoacétate de terbutyle (1,3 mol) dissous dans 300 mL de DMAC sont ensuite ajoutés goutte à goutte. Le milieu réactionnel est agité à température ambiante pendant 3 semaines. Le milieu réactionnel est refroidi à 5 °C puis filtré. Les cristaux obtenus sont lavés avec 150 ml de DMAC glacé puis avec 210 mL d'acétate d'éthyle. On obtient un solide blanc avec un rendement de 60%.
C.C.M : CH₂Cl₂ / MeOH / NH₄OH à 25% (80/15/5)
Révélateur : KMnO₄ ;Rf = 0,75
HPLC : SYMMETRY C18,5 µm, 250 × 4,6 mm, 100Å

Solvant A : Eau/TFA pH 2,7 ;Solvant B : CH₃CN
Detection UV à 201nm ; Tr = 35,5 min

### Etape 2 :

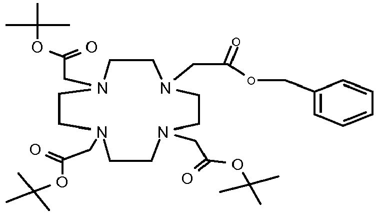

Mw = 662,87 g/mol
1g du composé obtenu à l'étape 1 et 0,38g de K₂CO₃ sont dissous dans 15 ml de CH₃CN. 0,37ml de bromoacétate de benzyle sont additionnés goutte à goutte. Le milieu réactionnel est chauffé au reflux une nuit sous argon. Après retour à température ambiante, le milieu réactionnel est filtré puis concentré. Après des lavages acido-basiques, obtention de 1g de cristaux blancs.

HPLC : SYMMETRY C18, 3,5µm, 50 × 2,1 mm,
Solvant A : acide formique 0,05% ;Solvant B : CH₃CN (+ acide formique 0,04%) Détection UV à 220 nm ;Tr = 3,8 min

### Etape 3 :

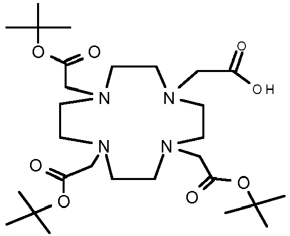

Mw = 572,75 g/mol
1g du composé obtenu à l'étape 2 sont dissous dans 20 mL d'EtOH et hydrogénés sous très faible pression (ballon de baudruche, en présence de charbon palladié, à 25° C pendant 15 h.) Après filtration et évaporation du solvant, on obtient 0,6 g de cristaux blancs (rendement de 73%).
HPLC : SYMMETRY C18, 3,5µm, 50 × 2,1 mm
Solvant A : acide formique 0,05% ;Solvant B : CH₃CN (+ acide formique 0,04%) Détection UV à 220 nm ;Tr = 3,1 min

### Exemple de référence 9

Le composé obtenu à l'exemple 8 est couplé à une série de peptides dont les séquences sont données à l'exemple 2 selon le mode opératoire suivant.

### Etape 1 : Couplage des peptides N° 1, 2, 3, 4, 5 ou 6 sur le composé de l'exemple 8

0,4g du composé obtenu dans l'exemple 8 (0,7 mmol), 144mg de DCC (0,7 mmol) et 80 mg de NHS (0,7 mmol) sont agités dans 10 mL de CH₂Cl₂ pendant 20min à TA. 20 mL d'une solution de peptide protégé (0,7 mmol) et de triéthylamine (1,4 mmol) dans le DMSO sont ajoutés à l'ester préactivé. Après 30 minutes, le milieu réactionnel est précipité dans 450 mL d'éther éthylique. Le précipité est filtré puis séché.

### Etape 2 : La déprotection est conduite selon la méthode décrite à l'étape 2 de l'exemple 2.

### Etape 3 : Complexation

Le composé obtenu à l'étape 2 (0,041 mmol) est dissous dans 1 mL de tampon acétate 0,1M à pH 4,8. On ajoute 0,041 mmol d'acétate de Gallium et on ajuste le pH à 5,5. La solution est ensuite chauffée à 80°C pendant 10 min. Le milieu réactionnel est concentré. Un chauffage pendant 5 minutes est toutefois déjà satisfaisant pour obtenir une complexation du gallium.

| N° | Structure | PM |
|---|---|---|
| 1 | 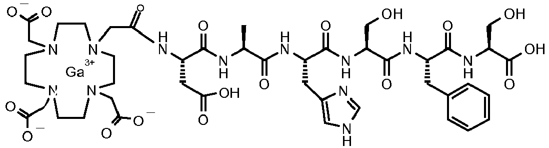 | 1115,77 |
| 2 | 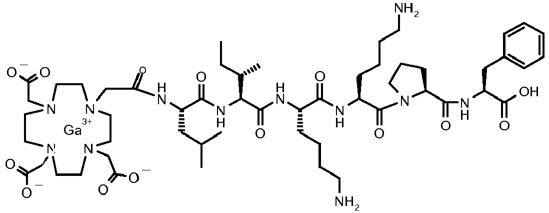 | 1198,09 |
| 3 | 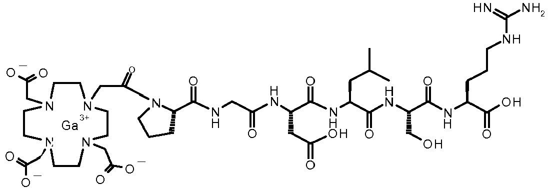 | 1096,81 |
| 4 | 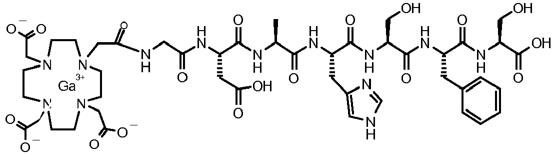 | 1172,82 |
| 5 | 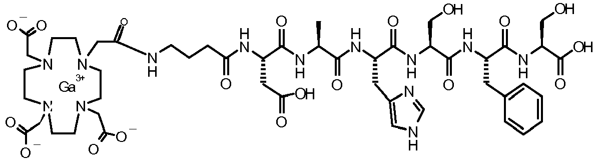 | 1200,87 |
| 6 | 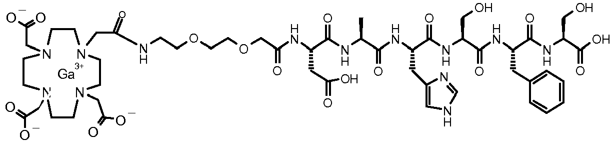 | 1260,93 |

### Exemple de référence 10

Le composé obtenu à l'exemple 8 est couplé à une série de dérivés de l'acide folique (N°7, 8 ou 9) dont les structures sont données dans le tableau ci-dessous.

| N° | Structure | PM |
|---|---|---|
| 7 | 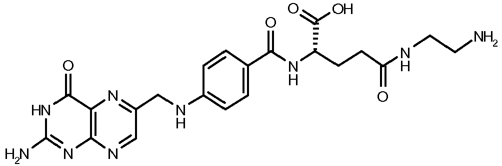 | 483,49 |
| 8 | 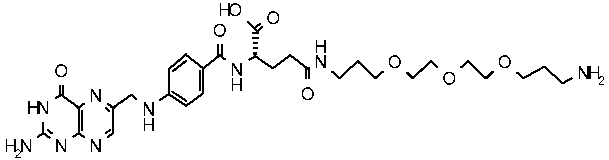 | 643,71 |
| 9 | 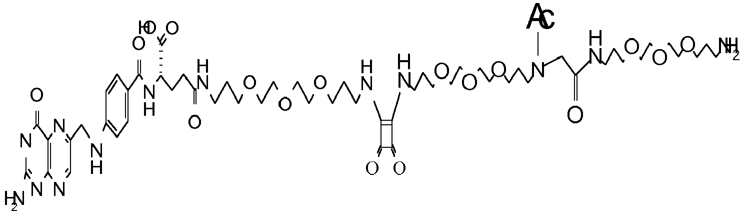 | 1244,42 |

### Synthèse des dérivés de l'acide folique :

La synthèse du dérivé n°8 est décrite dans l'exemple 11 du document WO2004/112839 page 105 à 108.

La synthèse du dérivé n°7 s'effectue selon le même mode opératoire que le dérivé 8 à l'exception de la dernière étape où le 4,7,10-Trioxa-1,13-tridecanediamine est remplacé par l'éthylène diamine.

La synthèse du dérivé n°9 s'effectue à partir du dérivé 8 qui est condensé au linker dont la structure est la suivante.

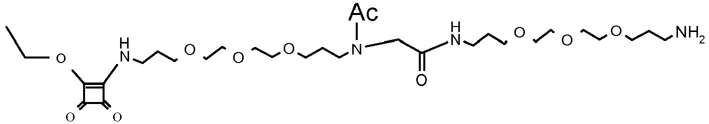

Brièvement la synthèse de ce linker s'effectue en 4 étapes à partir du 4,7,10-Trioxa-1,13-tridecanediamine monoBoc (composé a) de l'exemple 15 dans le brevet spécifique).

### Etape 1 :

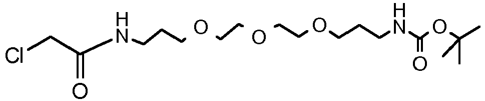

10 g de 4,7,10-Trioxa-1,13-tridecanediamine monoBoc (31,2 mmol) sont dissous à-5°C (par un bain d'acétone et de glace) dans 50 mL de CH₂Cl₂. 5 g de K₂CO₃ (36,2 mmol) dissous dans 50 mL d'eau et 5 g de chlorure de chloroacétyle (44,2 mmol) dissous dans 50 ml de CH₂Cl₂ sont ajoutés simultanément goutte à goutte à froid. Le milieu réactionnel est agité à TA pendant 1 heure. La phase organique est lavée à l'eau jusqu'à pH neutre puis filtrée et évaporée à sec. m= 11,2 g
ES⁺ : m/z (z = 1) = 397,3

### Etape 2 :

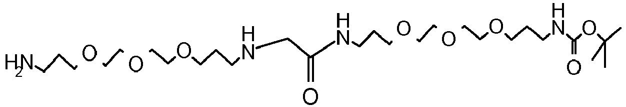

6,8g de 4,7,10-Trioxa-1,13-tridecanediamine sont dissous dans 50 ml de CH₃CN en présence de 0,85g de K₂CO₃. A cette suspension est ajoutée goutte à goutte 6,2 mmol du composé issu de l'étape 1 dissous dans 25 ml de CH₃CN. Le milieu réactionnel est chauffé au reflux pendant 2 H. Après retour à température ambiante, le milieu réactionnel est filtré puis évaporé. Le résidu obtenu est dissous dans 50 ml de CH₂Cl₂ et lavé avec 4x20 ml d'eau. La phase organique est séchée sur Na₂SO₄ puis est purifiée sur silice avec un mélange 50/50 CH₂Cl₂ / MeOH puis 30/70. m = 1.8 g (rendement 50%).
ES ⁺ M/z = 581(z=1) et M/z=291.3 (z=2)

### Etape 3 :

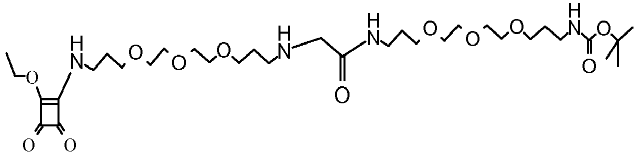

0,25g du composé issu de l'étape précédente sont mis en solution dans 1,5 ml de CH₂Cl₂. 57,5µl de diéthyle squarique acide sont ajoutés. Le milieu réactionnel est mis sous agitation pendant 18H à température ambiante. Le produit n'est pas isolé. ES ⁺ M/z = 706(z=1) et M/z=354 (z=2)

### étape 4 :

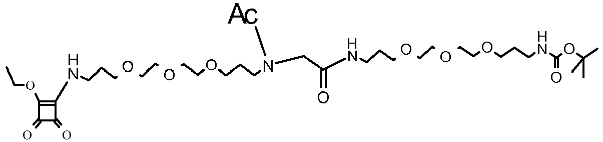

Au milieu réactionnel issu de l'étape 3, 40,6 µl d'Ac₂O sont ajoutés et l'ensemble est agité 5 minutes à température ambiante avant d'être purifié sur silice, avec CH₂Cl₂/EtOH (9/1) comme éluant. m = 0,27g (huile translucide).
ES⁺ M/z = 748 avec z=1
Les étapes 1 et 2 correspondant au couplage des dérivés de l'acide folique N° 7, 8 ou 9 sur le dérivé acide de l'exemple 8 et à la déprotection sont conduites selon le même mode opératoire que celui décrit dans l'exemple 9.

### Etape 5 : Complexation

0,041mmol du composé obtenu à l'étape 2 sont dissoutes dans l'1 ml d'eau. On ajoute 0,041 mmol de chlorure de Gallium anhydre, et on chauffe à 80°C le milieu réactionnel pendant 10min. Le milieu réactionnel est concentré.

| N ° | Structure | PM |
|---|---|---|
| 7 | 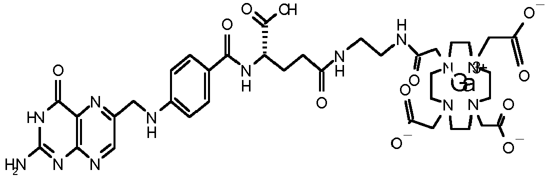 | 937,60 |
| 8 | 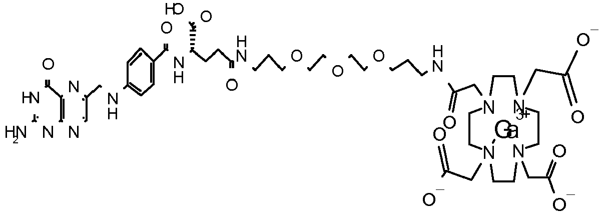 | 1096,81 |
| 9 | 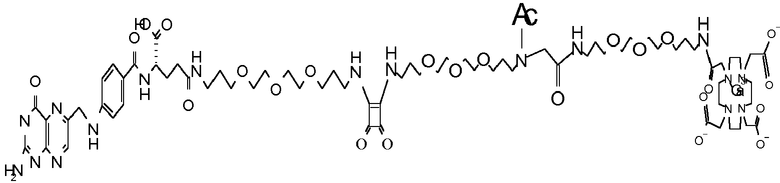 | 1698,53 |

On a décrit en détail le couplage d'un ensemble linker-biovecteur sur une seule fonction carboxylique. De manière analogue, en utilisant des protections/déprotections appropriées, on couple plusieurs ensembles linker-biovecteurs au chélate, avantageusement 3 ou 4 ensembles (un ensemble par fonction carboxylique).

### Exemple de référence 11 : 4-(4-Isothiocyanato-phenyl)-2-(4,7,10-Tris-tert-butoxycarbonylmethyl-1,4,7,10tetraaza-cyclododec-1-yl)-butyric acid ter-butyl este

### Etape 1 : Identique à celle de l'exemple 8

### Etape 2 :

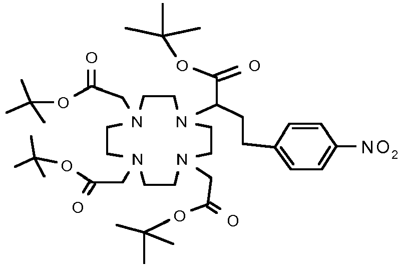

Mw = 778,01 g/mol
A une suspension de 20 g du composé obtenu à l'étape 1 et 10,8 g de K₂CO₃ dans 400 ml de CH₃CN, on ajoute goutte à goutte 26,8 g de 2-bromo-4-(4-nitrophényl)butyric acid terbutyl ester en solution dans 50 ml de CH₃CN. Après agitation 24 h à 25° C, le milieu réactionnel est filtré, lavé par CH₃CN puis concentré. Après des lavages acido-basiques, obtention de 18g de produit.
Spectre de masse : Mode **ES⁺** m/z = 779 avec z = 1

### Etape 3 : Réduction du Nitro

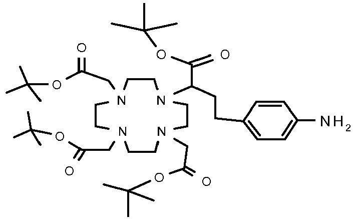

Mw = 748,02 g/mol
5 g du composé obtenu à l'étape 2 sont dissous dans 70 ml de MeOH et hydrogénés sous pression (charbon palladié à 10%, 25° C sous une pression d'hydrogène de 3.10⁵ Pa pendant 6 h). On obtient 4,2 g de produit.
Spectre de masse : Mode **ES⁺** m/z = 749 avec z = 1

### étape 4 :

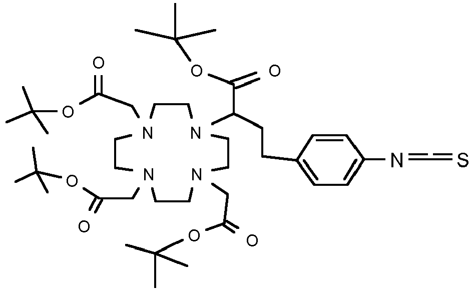

Mw = 790,08 g/mol
2 g (2,7 mmol) du produit issu de l'étape 3 sont solubilisés dans un mélange de 24 ml d'eau et 16 ml de CHCl₃. 0,45 ml (5,8 mmol) de SCCl₂ sont additionnés goutte à goutte et l'ensemble est agité 1H30. Le milieu réactionnel est décanté et la phase organique est évaporée sous vide. Le concentrat est repris dans de l'éther et agité une nuit à TA. Le précipité est filtré et séché sous vide. m= 2 g
Spectre de masse : Mode **ES⁺** m/z = 791 avec z = 1

### Exemple de référence 12

Le composé obtenu à l'exemple 11 est couplé à une série de peptides dont les séquences sont données à l'exemple 2 selon le mode opératoire suivant.

### Etape 1 : Addition des peptides N° 1, 2, 3, 4, 5 ou 6 sur le dérivé de l'exemple 11

1,2g (1,5 mmol) du composé obtenu dans l'exemple 11 sont solubilisés dans 30 ml de DMSO. Le peptide protégé (1,5 mmol) et 3mmol de triéthylamine sont agités pendant 24h. Le milieu réactionnel est ensuite précipité dans 500 ml d'éther diéthylique.

### Etape 2 et 3: La déprotection et la complexation sont réalisées comme dans l'exemple 9 (étape 2 et 3).

| N° | Structure | PM |
|---|---|---|
| 1 | 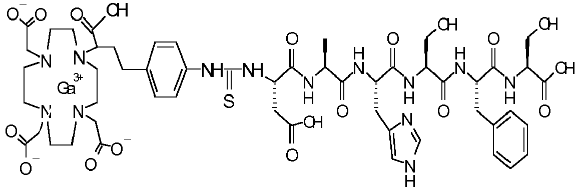 | 1295,01 |
| 2 | 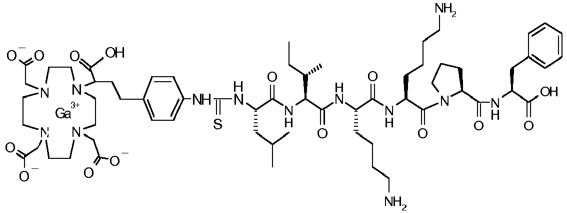 | 1377,33 |
| 3 | 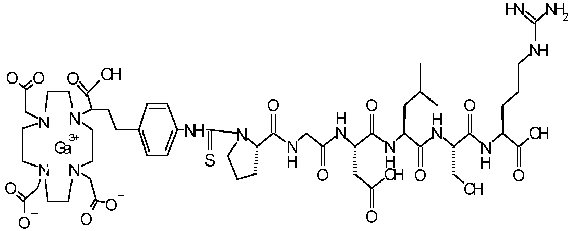 | 1276,05 |
| 4 | 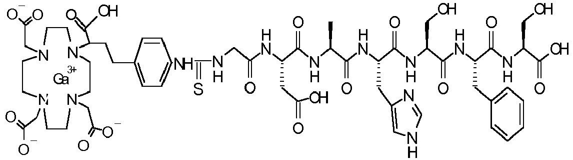 | 1352,06 |
| 5 | 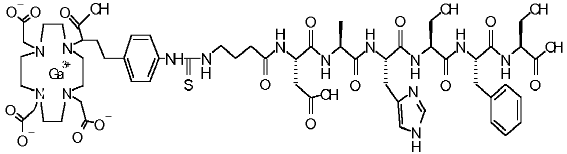 | 1380,12 |
| 6 | 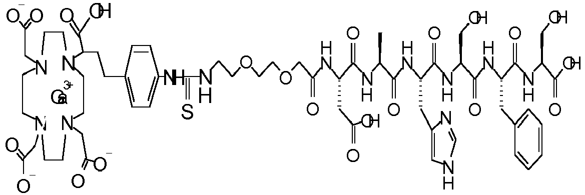 | 1440,17 |

### Exemple de référence 13

Le composé de l'exemple 11 a été couplé à une série de dérivés de l'acide folique dont les structures sont décrites dans l'exemple 10. Les étapes 1 et 2 correspondant aux étapes de couplage et de déprotection sont conduites selon le même mode opératoire que celui décrit dans l'exemple 12 (étape 1 et 2). La complexation s'effectue dans les mêmes conditions qu'à l'étape 3 de l'exemple 10.

| N° | Structure | PM |
|---|---|---|
| 7 | 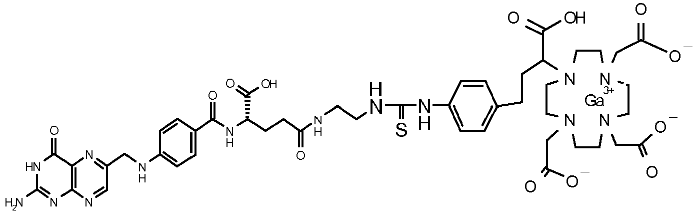 | 1115,84 |
| 8 | 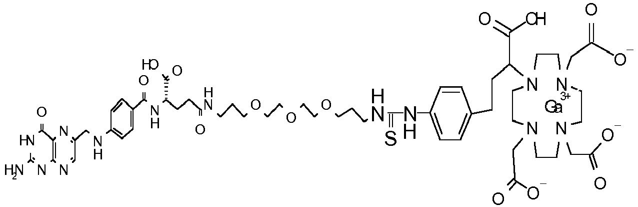 | 1276,05 |
| 9 | 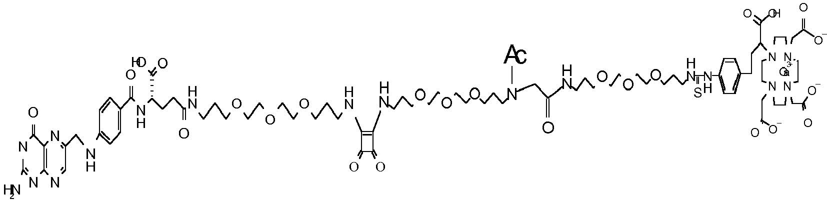 | 1876,77 |

### Exemple 14: [4,7-Bis-tert-butoxycarbonylmethyl-10-(4-Isothiocyanato-benzyl)-1,4,7,10tetraaza-cyclododec-1-yl)-acetic acid ter-butyl este

### Etape 1 : Identique à celle de l'exemple 8

### Etape 2 :

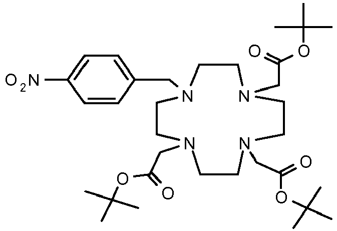

Mw = 649,83 g/mol
A une suspension de 20 g du composé obtenu à l'étape 1 et 10,8 g de K₂CO₃ dans 400 mL de CH₃CN, on ajoute goutte à goutte 16,8 g de 1-bromo-4-nitrobenzene en solution dans 50 mL de CH₃CN. Après agitation 24 h à 25° C, le milieu réactionnel est filtré, lavé par CH₃CN puis concentré. Après des lavages acido-basiques, obtention de 18g de produit. Spectre de masse : Mode **ES⁺** m/z = 649 avec z = 1

### Etape 3 : Réduction du Nitro

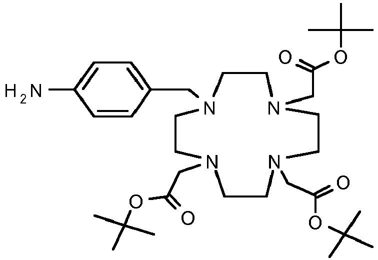

Mw = 619,85 g/mol
5 g du composé obtenu à l'étape 2 sont dissous dans 70 ml de MeOH et hydrogénés sous pression (charbon palladié à 10%, 25° C sous une pression d'hydrogène de 3.10⁵ Pa pendant 6 h). On obtient 4,2 g de produit. Spectre de masse : Mode **ES⁺** m/z = 619 avec z = 1

### étape 4 :

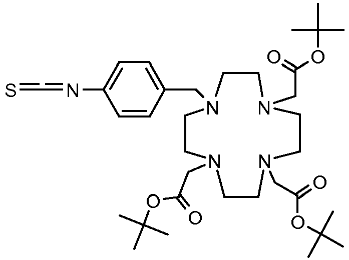

Mw = 661,91 g/mol
2 g (3,2 mmol) du produit issu de l'étape 3 sont solubilisés dans un mélange de 24 mL d'eau et 16 ml de CHCl₃. 0,53 ml (6,9 mmol) de SCCl₂ sont additionnés goutte à goutte et l'ensemble est agité 1H30. Le milieu réactionnel est décanté et la phase organique est évaporée sous vide. Le concentrat est repris dans de l'éther et agité une nuit à TA. Le précipité est filtré et séché sous vide. m= 2 g ; Spectre de masse : Mode **ES⁺** m/z = 661 avec z = 1

### Exemple de référence 15

Le composé obtenu à l'exemple 14 est couplé à une série de peptides dont les séquences sont données à l'exemple 2 selon le même mode opératoire que celui décrit dans l'exemple 12.

| N° | Structure | PM |
|---|---|---|
| 1 | 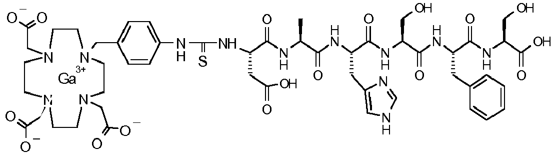 | 1222,948 |
| 2 | 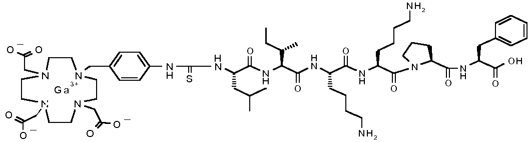 | 1305,27 |
| 3 | 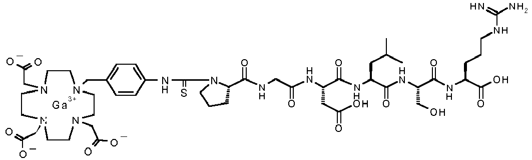 | 1203,99 |
| 4 | 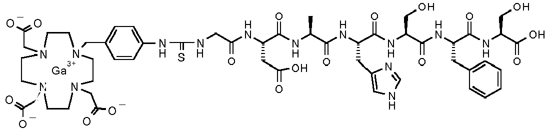 | 1280,00 |
| 5 | 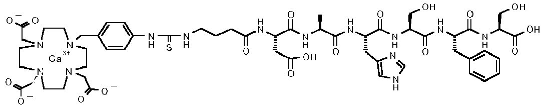 | 1308,05 |
| 6 | 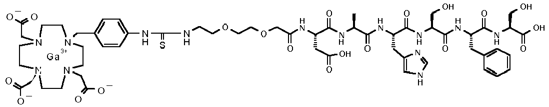 | 1368,40 |

### Exemple de référence 16

Le composé de l'exemple 14 a été couplé à une série de dérivés de l'acide folique dont les structures sont décrites dans l'exemple 10 selon le même mode opératoire que dans l'exemple 13.

| N ° | Structure | PM |
|---|---|---|
| 7 | 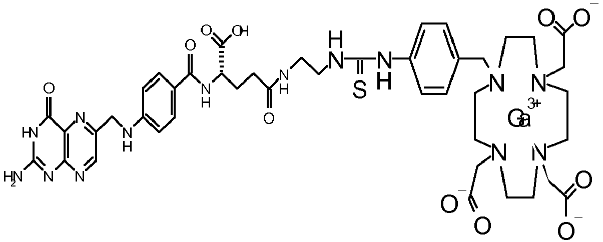 | 1043,77 |
| 8 | 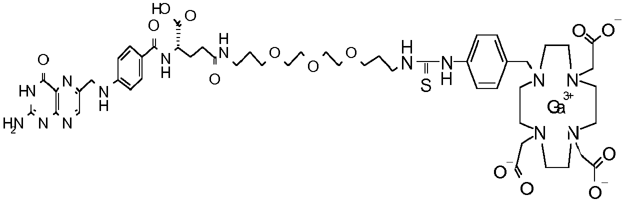 | 1203,99 |
| 9 | 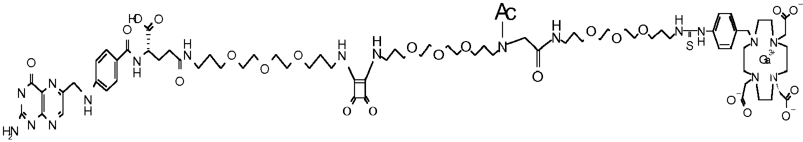 | 1804,70 |

### Exemple de référence 17 : 5-(2-Ethoxy-3,4-dioxo-cyclobut-1-enylamino)-2(4,7,10-Tris-tert-butoxycarbonylmethyl-1,4,7,10tetraaza-cyclododec-1-yl)-pentanoic acid ter-butyl ester

### Etape 1 : Identique à celle de l'exemple 8

### Etape 2 :

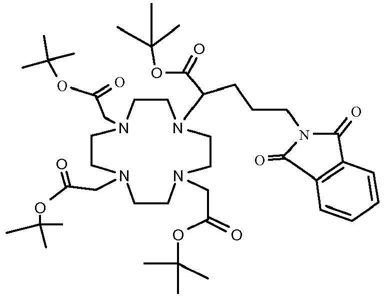

Mw = 816,06 g/mol
A une suspension de 20 g du composé obtenu à l'étape 1 et 10,8 g de K₂CO₃ dans 400 mL de CH₃CN, on ajoute goutte à goutte 29,8 g de 2-bromo-5-(1,3-dioxo-1,3-dihydro-isoindol-2-yl)-pentanoic acide terbutyl ester en solution dans 50 ml de CH₃CN. Après agitation 24 h à 25° C, le milieu réactionnel est filtré, lavé par CH₃CN puis concentré. Après des lavages acido-basiques, obtention de 20g de produit.
Spectre de masse : Mode **ES⁺** m/z = 815 avec z = 1

### Etape 3 :

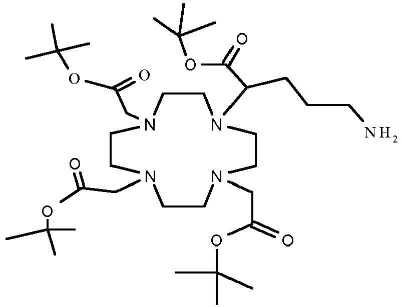

Mw = 685,95 g/mol
5,7 mL d'hydrazine hydrate puis 5 g du composé obtenu à l'étape 2 sont ajoutés à 15 mL d'eau chauffée à 80°C. Le milieu réactionnel est agité à 80°C pendant 3 heures. Après refroidissement, le pH est diminué à 1 avec HCl 12N. Après filtration la solution est évaporée sous vide. On obtient 3,6 g de produit.
Spectre de masse : Mode **ES⁺** m/z = 985 avec z = 1

### étape 4 :

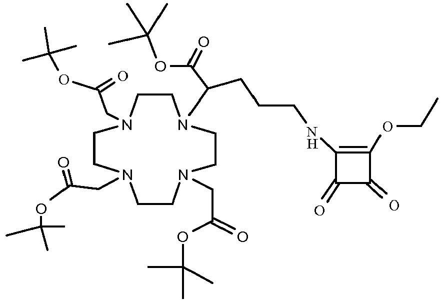

Mw = 810,05 g/mol
Même mode opératoire qu'à l'étape 5 de l'exemple 1.
Spectre de masse : Mode **ES⁺** m/z = 811 avec z = 1

### Exemple de référence 18

Le composé obtenu à l'exemple 17 est couplé à une série de peptides dont les séquences sont données à l'exemple 2.

### Etape 1 et 2 : même mode opératoire que celui décrit dans l'exemple 2.

### Etape 3 : La complexation est réalisée selon le même mode opératoire qu'à l'étape 3 de l'exemple 9.

### Exemple de référence 19

Le composé de l'exemple 17 a été couplé à une série de dérivés de l'acide folique dont les structures sont décrites dans l'exemple 10.

### Etape 1 et 2 : même mode opératoire que celui décrit dans l'exemple 2.

### Etape 3 : La complexation est réalisée selon le même mode opératoire qu'à l'étape 3 de l'exemple 10.

| N° | Structure | PM |
|---|---|---|
| 7 | 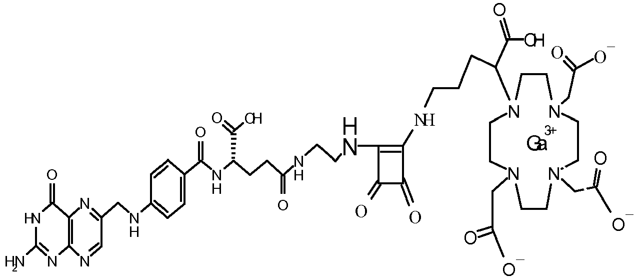 | 1089,70 |
| 8 | 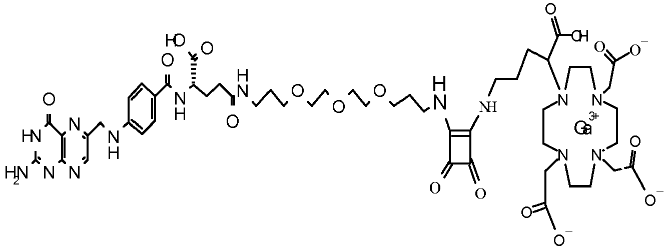 | 1249,95 |
| 9 | 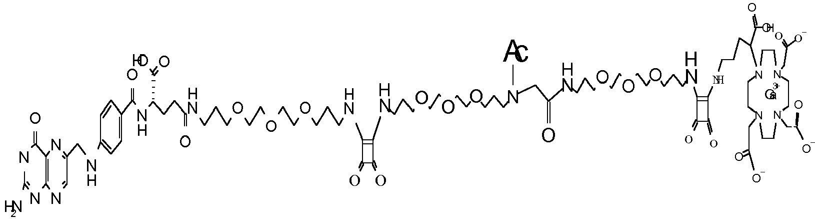 | 1850,66 |

### Exemple de référence 20: 2-(4,7,10-Tris-tert-butoxycarbonylmethyl-1,4,7,10tetraaza-cyclododec-1-yl)-pentanoic acid 1-ter-butyl ester

### Etape 1 : Identique à celle de l'exemple 8

### Etape 2 :

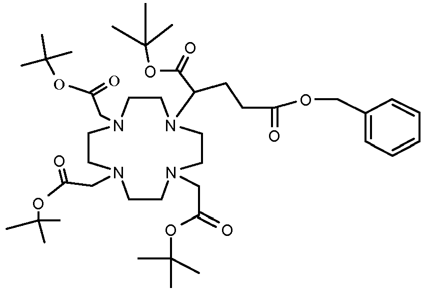

Mw = 791,05 g/mol
A une suspension de 20 g du composé obtenu à l'étape 1 et 10,8 g de K₂CO₃ dans 400 ml de CH₃CN, on ajoute goutte à goutte 27,8 g de 2-bromo-5-(benzyl)-pentanoic acide terbutyl ester en solution dans 50 ml de CH₃CN. Après agitation 24 h à 25° C, le milieu réactionnel est filtré, lavé par CH₃CN puis concentré. Après des lavages acido-basiques, obtention de 23g de produit.
Spectre de masse : Mode **ES⁺** m/z = 792 avec z = 1

### Etape 3 :

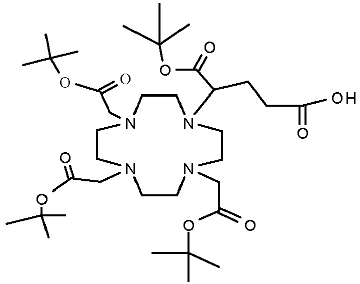

Mw = 700,92 g/mol
10g du composé obtenu à l'étape 2 sont dissous dans 200 ml d'EtOH et hydrogénés sous très faible pression (ballon de baudruche, en présence de charbon palladié, à 25° C pendant 15 h.) Après filtration et évaporation du solvant, on obtient 8 g de produit.
Spectre de masse : Mode **ES⁺** m/z = 702 avec z = 1

### Exemple de référence 21

Le composé obtenu à l'exemple 20 est couplé à une série de peptides dont les séquences sont données à l'exemple 2 selon le même mode opératoire que celui décrit dans l'exemple 9.

| N° | Structure | PM |
|---|---|---|
| 1 | 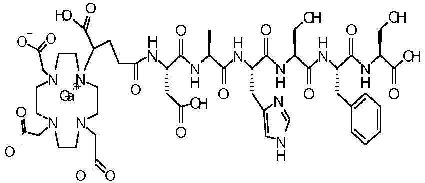 | 1187,83 |
| 2 | 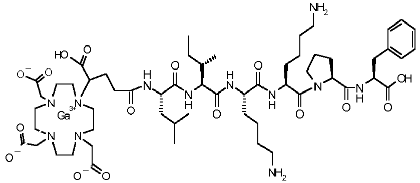 | 1270,15 |
| 3 | 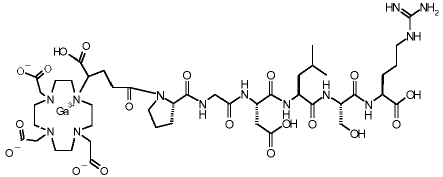 | 1168,87 |
| 4 | 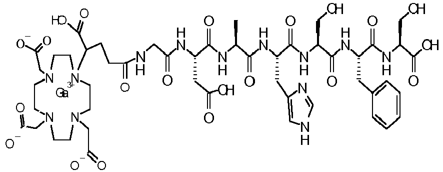 | 1244,88 |
| 5 | 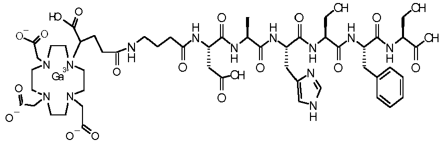 | 1272,94 |
| 6 | 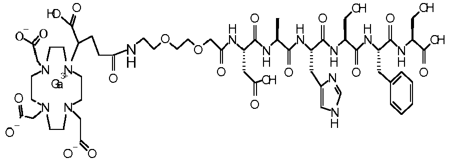 | 1332,99 |

### Exemple 22

Le composé de l'exemple 20 a été couplé à une série de dérivés de l'acide folique dont les structures sont décrites dans l'exemple 10 selon le même mode opératoire que celui décrit dans l'exemple 10.

| N° | Structure | PM |
|---|---|---|
| 7 | 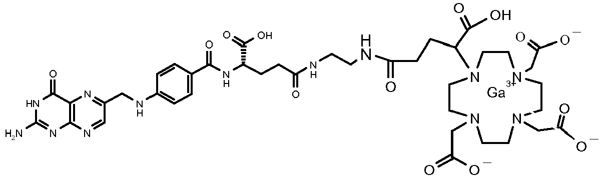 | 1008,66 |
| 8 | 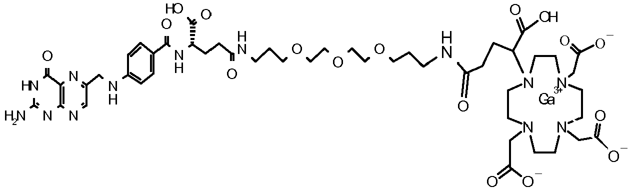 | 1168,87 |
| 9 | 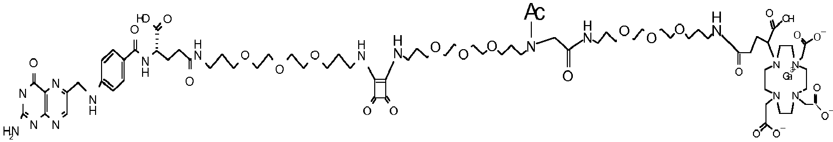 | 1769,59 |

D'autres composés avantageux sont obtenus en couplant de manière appropriée au moins deux chaînes carboxyliques du chélate (au lieu d'une), les composés du tableau ci-dessus portant alors autant de biovecteurs que de chaînes couplées.

### Exemple de référence 23 : 2-(6,9-Bis-tert-butoxycarbonylmethyl-3,6,9,15tetraaza-bicylo[9.3.1]pentadeca-1(15)11,13-trien-3-yl)-pentanoic acid 1-ter-butyl ester

### Etape 1 :

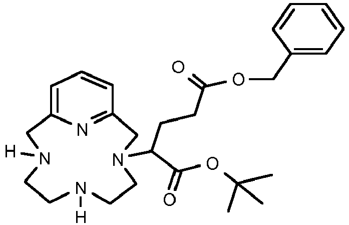

Mw = 482,63 g/mol
10 g de 3,6,9,15-Tetraaza-bicyclo[9.3.1]pentadéca-1(14),11(15),12-triéne (48,5mmol) sont dissous dans un mélange eau-acétonitrile (170 mL d'acétonitrile et 7 mL d'eau). Après ajout de 10ml de TEA (1,4eq) le mélange est chauffé à 50°C puis 17,3 g de 2-bromo-5-benzyl pentanedioic acid terbutyl ester (1eq) sont ajoutés. Le milieu est agité 18h à 50°C.
L'acétonitrile est évaporé et le résidu repris au dichlorométhane est lavé à l'eau (100mL). La phase organique est séchée sur sulfate de magnésium anhydre et évaporée à sec. Huile brune. Rendement 58%
Spectre de masse : Mode **ES⁺** m/z = 483 avec z = 1

### Etape 2 :

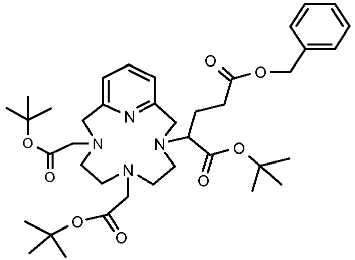

Mw = 710,92 g/mol
5 g du composé obtenu à l'étape 1 (10mmol) sont mis en solution dans 200 mL d'acétonitrile avec 6,4 g de K₂CO₃ (4,5 eq). Le mélange est porté à reflux et le bromoacétate de terbutyle (6,9 g ; 3,4 eq) est ajouté rapidement. Le milieu réactionnel est porté à reflux pendant 18h. Les sels sont filtrés et le solvant évaporé. Huile brune : 6g.
Spectre de masse : Mode **ES⁺** m/z = 712 avec z = 1

### Etape 3 :

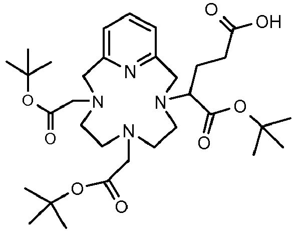

Mw = 620,79 g/mol
5g du composé obtenu à l'étape 2 sont dissous dans 20 ml d'EtOH et hydrogénés sous très faible pression (ballon de baudruche, en présence de charbon palladié, à 25° C pendant 15 h.) Après filtration et évaporation du solvant, on obtient 4 g de produit.

Spectre de masse : Mode ES⁺ m/z = 622 avec z = 1

### Exemple de référence 24

Le composé obtenu à l'exemple 23 est couplé à une série de peptides dont les séquences sont données à l'exemple 2 selon le même mode opératoire que celui décrit dans l'exemple 9.
Les produits obtenus sont identiques à ceux de l'exemple 7.

### Exemple de référence 25

Le composé de l'exemple 23 a été couplé à une série de dérivés de l'acide folique dont les structures sont décrites dans l'exemple 10 selon le même mode opératoire que celui décrit dans l'exemple 10.

| N° | Structure | PM |
|---|---|---|
| 7 | 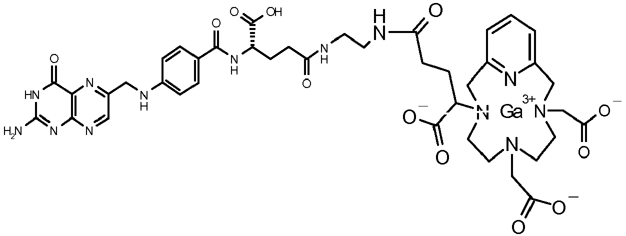 | 984,64 |
| 8 | 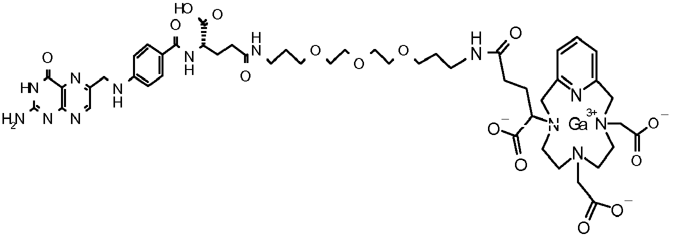 | 1144,85 |
| 9 | 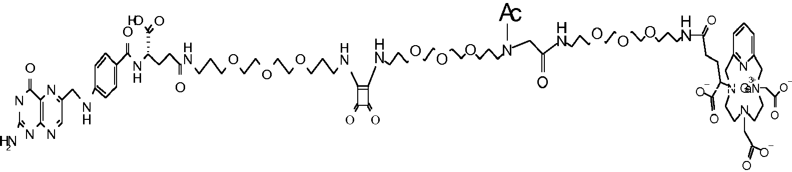 | 1745,57 |

### Exemple 26: 2-(4,7-Bis-tert-butoxycarbonylmethyl-[1,4,7]triazonan-1-yl)-5-(2-ethoxy-3,4-dioxo-cyclobut-1-enylamino)-pentanoicacid ter-butyl este

### Etape 1 :

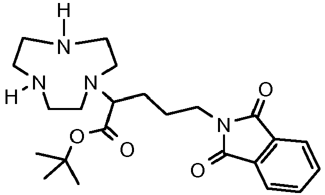

Mw = 430,55 g/mol
10 g de triaza-1,4,7-cyclononane (77,4mmol) sont dissous dans un mélange eau-acétonitrile (170 mL d'acétonitrile et 7 mL d'eau). Après ajout de 10,7 g de K₂CO₃, on ajoute goutte à goutte 29,6 g de 2-bromo-5-(1,3-dioxo-1,3-dihydroisoindol-2-yl)-pentanoic acide terbutyl ester en solution dans 50 ml de CH₃CN. Après agitation 24 h à 25° C, le milieu réactionnel est filtré, lavé par CH₃CN puis concentré. Après des lavages acido-basiques, obtention de 20g de produit
Spectre de masse : Mode **ES⁺** m/z = 431,5 avec z = 1

### Etape 2 : même mode opératoire qu'à l'étape 2 de l'exemple 23

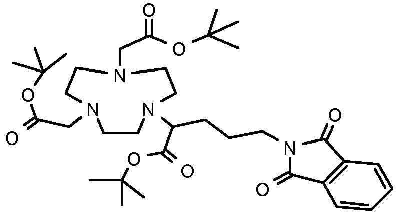

Mw = 658,84 g/mol

### Etape 3 et 4 : même mode opératoire qu'à l'étape 3 et 4 de l'exemple 17

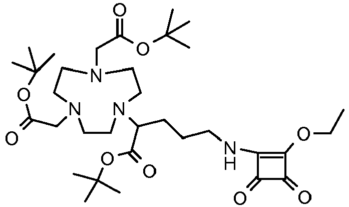

Mw = 652,84 g/mol

### Exemple 27

Le composé obtenu à l'exemple 26 est couplé à une série de peptides dont les séquences sont données à l'exemple 2 selon le même mode opératoire que celui décrit dans l'exemple 18.
Les produits obtenus sont identiques à ceux de l'exemple 5.

### Exemple 28

Le composé de l'exemple 26 a été couplé à une série de dérivés de l'acide folique dont les structures sont décrites dans l'exemple 10 selon le même mode opératoire que celui décrit dans l'exemple 19.

| N° | Structure | PM |
|---|---|---|
| 7 | 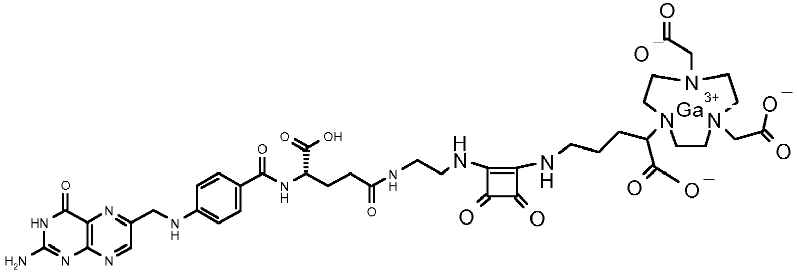 | 988,63 |
| 8 | 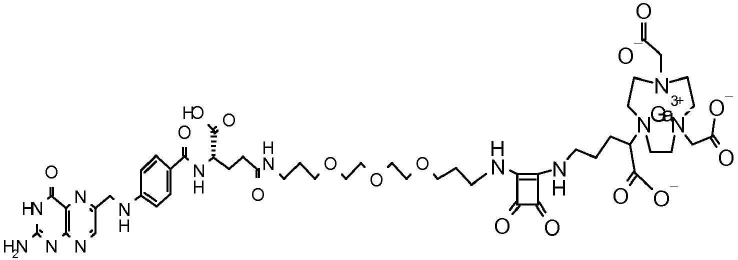 | 1148,84 |
| 9 | 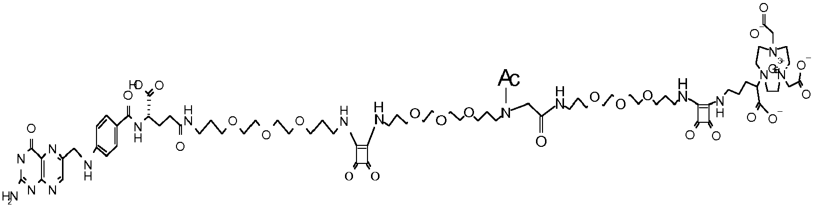 | 1749,56 |

### Exemple de référence 29

Le composé obtenu à l'étape 3 de l'exemple 17 est couplé à une série de squelettes chimiques (scaffolds) décrits dans l'art antérieur pour leur activité de ciblage thérapeutique, selon le mode opératoire suivant :

### étape 1 : Couplage des scaffolds sur le composé obtenu à l'étape 3 de l'exemple 17.

5 mmol de scaffold, 1g de DCC (5 mmol) et 0,6g de NHS (5 mmol) sont agités dans 50ml de CH₂Cl₂ pendant 20min à TA. 20ml d'une solution du composé obtenu à l'étape 3 de l'exemple 17 (5 mmol) et de triéthylamine (5 mmol) dans le DMSO sont ajoutés à l'ester préactivé. Après 30 minutes, le milieu réactionnel est précipité dans 600ml d'éther éthylique. Le précipité est filtré puis séché.

### Etape 2 et 3 : étape de déprotection et de complexation

Même mode opératoire que les étapes 2 et 3 de l'exemple 9

| **Structure** | **PM** |
|---|---|
| 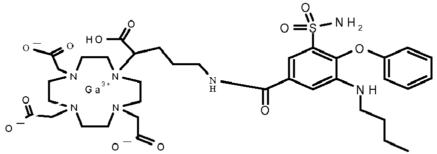 | 874,62 |
| 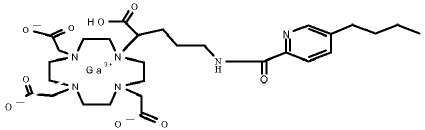 | 689,42 |
| 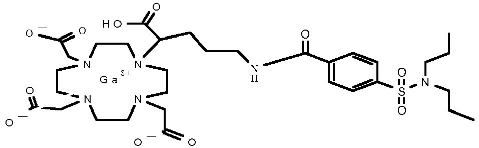 | 795,56 |
| 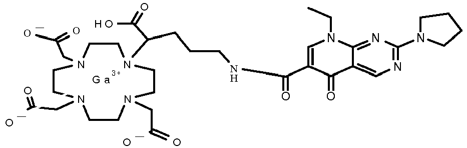 | 798,51 |
| 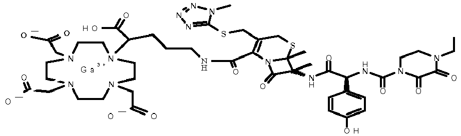 | 1183,93 |
| 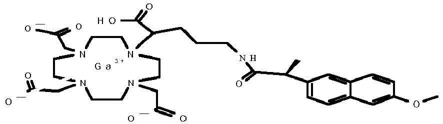 | 740,47 |
| 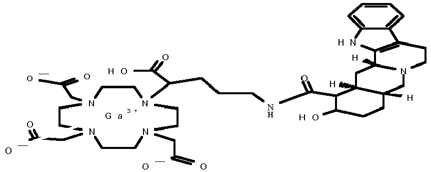 | 850,63 |
| 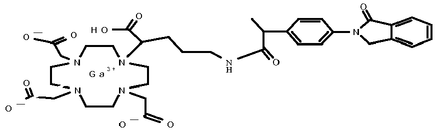 | 791,51 |
| 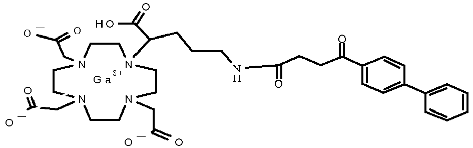 | 764,49 |
| 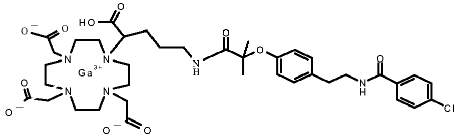 | 872,03 |
| 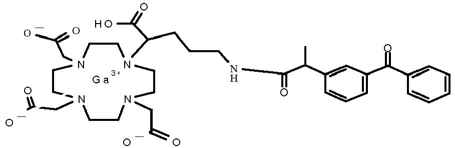 | 764,49 |
| 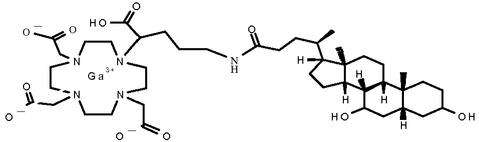 | 902,78 |
| 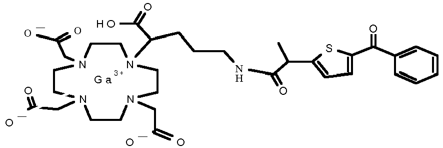 | 770,51 |
| 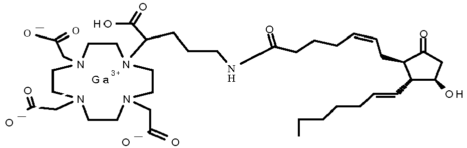 | 832,65 |
| 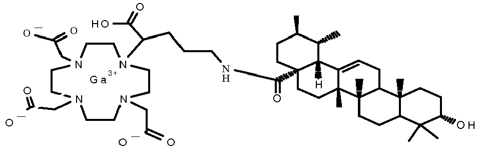 | 981,95 |
| 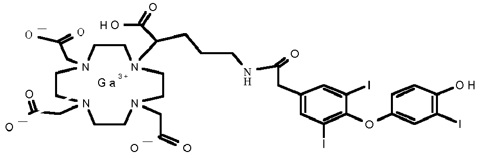 | 1132,14 |
| 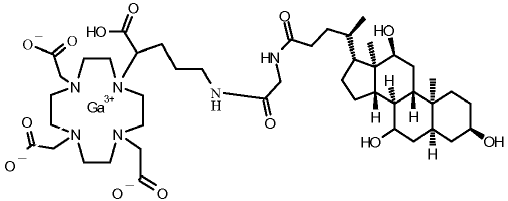 | 975,84 |
| 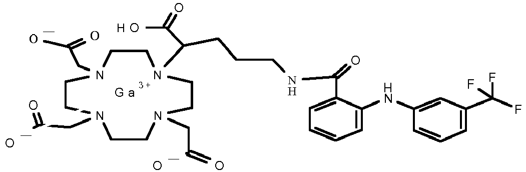 | 791,44 |
| 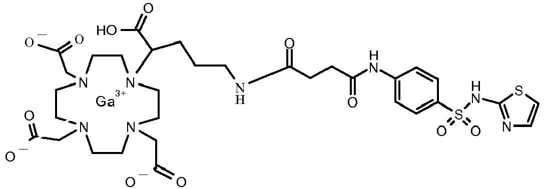 | 865,59 |
| 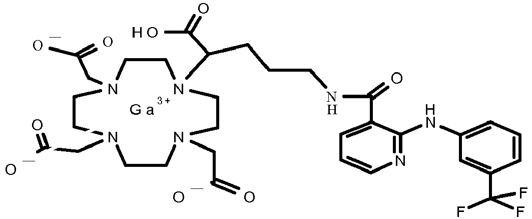 | 792,42 |
| 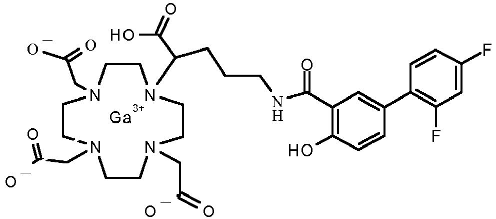 | 760,40 |
| 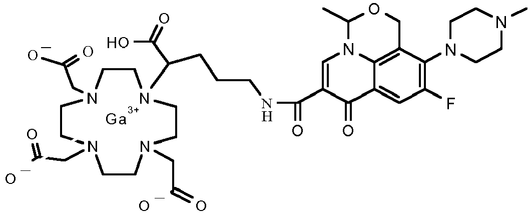 | 871,58 |
| 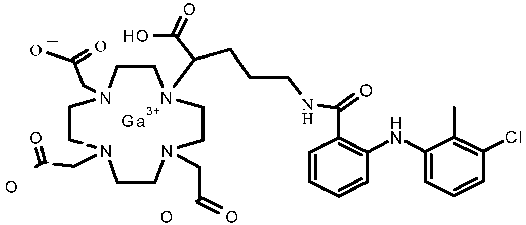 | 771,91 |
| 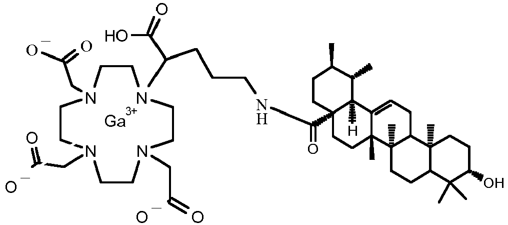 | 981,95 |
| 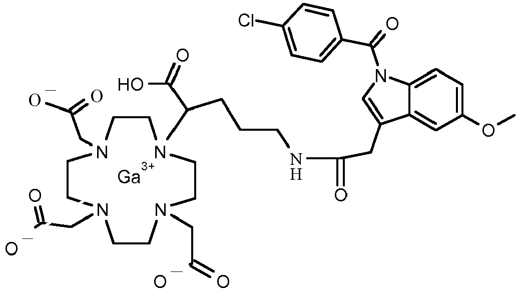 | 853,97 |
| 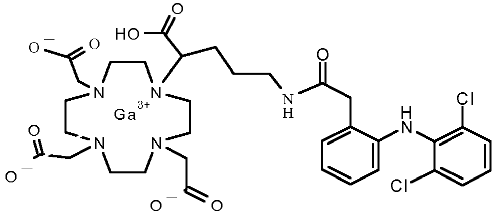 | 806,36 |

### Exemple de référence 30

Le composé obtenu à l'étape 3 de l'exemple 17 est couplé à une série de peptides dont les séquences sont données à l'exemple 2 selon le même mode opératoire que celui décrit dans l'exemple 29.

| N° | Structure | PM |
|---|---|---|
| 1 | 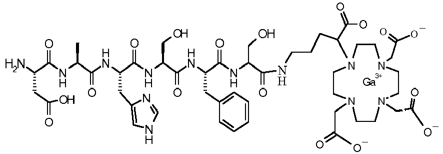 | 1172,86 |
| 2 | 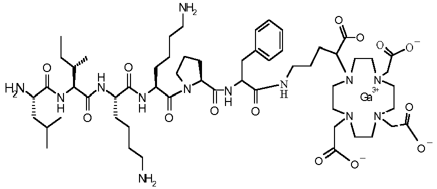 | 1255,18 |
| 3 | 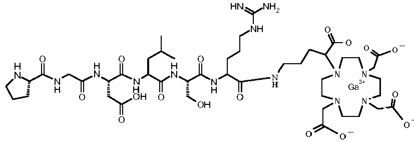 | 1153,90 |
| 4 | 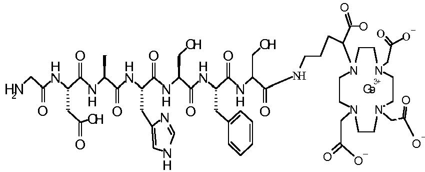 | 1229,91 |
| 5 | 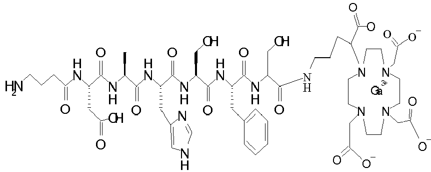 | 1257,97 |
| 6 | 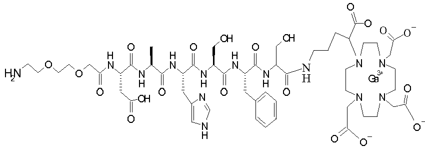 | 1318,02 |

### PARTIE II : Exemple de couplage des chélates avec le gallium.

Exemple avec radio marquage au 68Ga d'un composé chélate-biovecteur (5-100 nmol chélate-peptide), avec activation micro-onde 1 minute à 100 W tel que décrit dans WO2004089425.

### Exemple :

- Addition d'acétate de sodium Ga68 à l'éluat du générateur Ga-Ge avec ajustement du pH de l'éluat à 5.5.
- Addition du composé chélate-biovecteur
- Activation au micro-onde
- Refroidissement à température ambiante.

## Revendications

1. Complexe métallique de gallium Ga 68 de formule Ch-L-B (chélate - linker- biovecteur), ou Ch-Lp-Bq avec p=q= 2 à 5, Ch étant un chélate ayant la formule générale suivante : 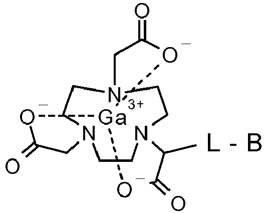 dans laquelle :
1) B est un biovecteur de ciblage d'une cible biologique associée à une pathologie, ledit biovecteur étant choisi parmi le folate, des métalloprotéases, COX, tyrosine kinase, et des récepteurs cellulaires choisis parmi VEGF, KDR, CXC, LHRH, GPIIb/IIIa, bombésine/GRP, gastrine, VIP, CCK, et GLUT,
2) L est un linker choisi parmi :
a) P1-1-P2, avec P1 et P2 identiques ou différents choisis parmi O, S, NH, CO₂, -NHCO, CONH, NHCONH, NHCSNH, SO₂NH-, et NHSO₂-,
et 1 représente un alkyle (avantageusement en C₁-C₁₀), alkoxyalkyle (avantageusement en C₁-C₁₀), polyalkoxyalkylène, alkyle interrompu par un ou plusieurs squarates ou par un ou plusieurs aryles, avantageusement phényle, alcényle (avantageusement en C₂-C₆), alcynyle (avantageusement en C₂-C₆), alkyle interrompus par un ou plusieurs groupes choisis parmi -NH-, -O-, -CO-, - NH(CO)-, -(CO)NH-, -O(CO)-, ou -(OC)O- ;
b) (CH₂)ₙ, (CH₂)ₙ-CO-, -(CH₂)ₙNH-CO- avec n = 2 à 10 , (CH₂CH₂O)_{q}(CH₂)ᵣ-CO-, (CH₂CH₂O)_{q}(CH₂)ᵣ-NH-CO- avec q = 1-10 et r=2-10, (CH₂)ₙ-CONH-, (CH₂)ₙ-CONH-PEG, (CH₂)ₙ-NH-, 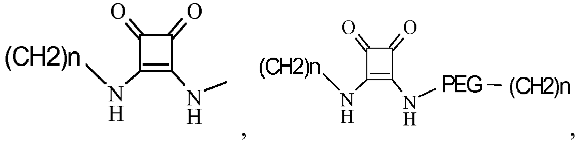 avec n=1 à 5 et avantageusement n=4 ou 5, HOOC-CH₂-O-(CH₂)₂-O-(CH₂)₂-O-CH₂-COOH ; HOOC-(CH₂)₂-CO₂-(CH₂)₂-OCO-(CH₂)₂-COOH ; HOOC-CH(OH)-CH(OH)-COOH; HOOC-(CH₂)ₙ-COOH; NH₂-(CH₂)ₙ-NH₂, avec n=0-20; NH₂-(CH₂)ₙ-CO₂H ; NH₂-CH₂ - (CH₂-O-CH₂)ₙ-CO₂H avec n=1 à 10.

2. Complexe selon la revendication 1, choisi parmi :

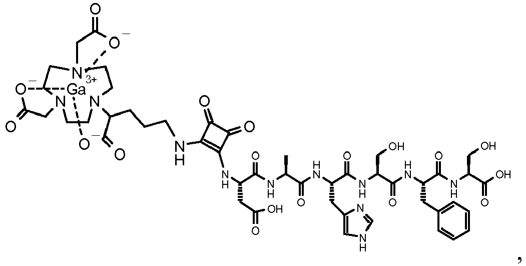

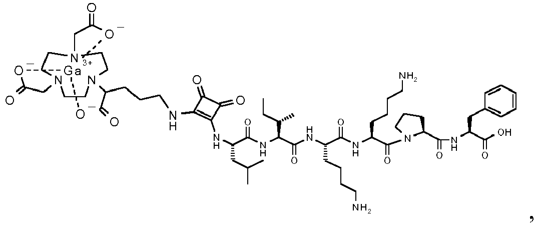

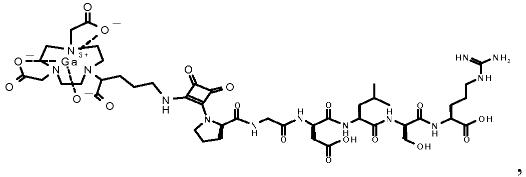

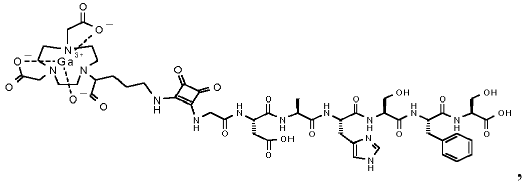

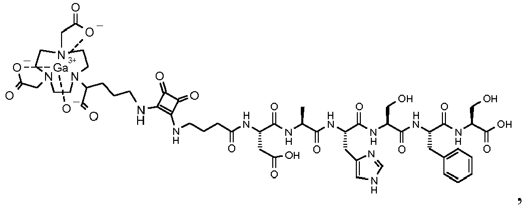

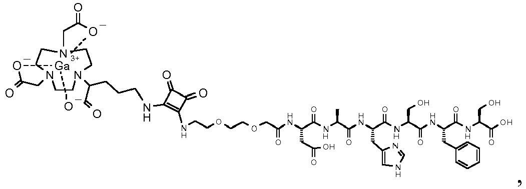

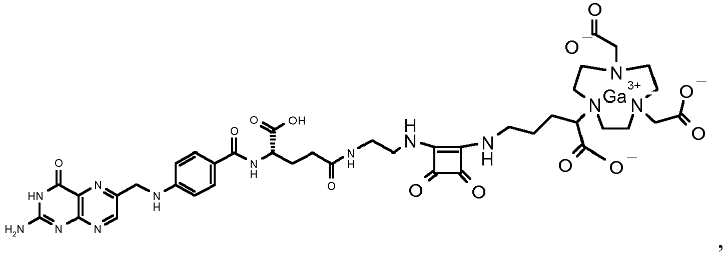

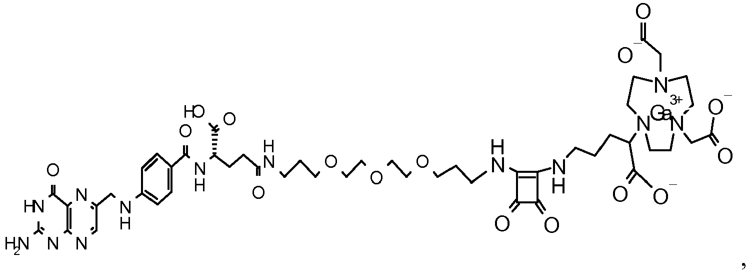

3. et 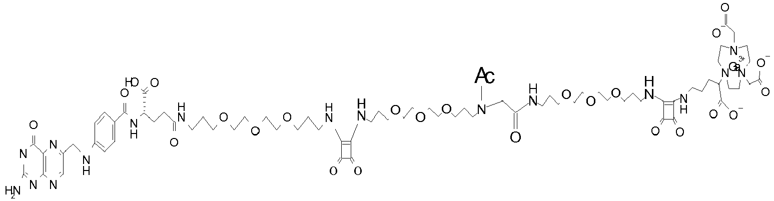 Complexe selon la revendication 1 ou 2, **caractérisé en ce que** le chélate comporte au moins une partie de masquage in vivo du chélate notamment un groupe hydrophile.

4. Complexe selon l'une quelconque des revendications 1 à 3, **caractérisé en ce que** le chélate comporte en outre une partie de reconnaissance d'une cible biologique améliorant la biodistribution du composé et/ou le transport du composé, ladite partie de reconnaissance étant différente du biovecteur.

5. Composition comprenant un complexe selon l'une quelconque des revendications 1 à 4 et un chélate linéaire ou macrocyclique Ch tel que défini dans la revendication 1 non complexé, présent à une concentration de 0,01 à 100 mM, avantageusement de 10 à 100 mM.

6. Composition comprenant un complexe selon l'une quelconque des revendications 1 à 4 et du calcium.

7. Composition comprenant un complexe selon l'une quelconque des revendications 1 à 4 et au moins un agent de stabilisation face à la radiolyse.

8. Kit d'administration d'un produit marqué au gallium Ga68 comprenant un complexe selon l'une quelconque des revendications 1 à 4 ou une composition selon l'une des revendications 5 à 7.

9. Composition de diagnostic comprenant d'une part un agent de contraste au gallium Ga68 détectable en PET comprenant un complexe selon l'une quelconque des revendications 1 à 4 ou une composition selon l'une quelconque des revendications 5 à 7 et d'autre part un agent de contraste détectable en IRM et/ou un agent de contraste détectable au scanner CT.

10. Produit de contraste au Ga68 permettant de détecter par imagerie PET au moins une zone d'intérêt diagnostique comprenant un complexe selon l'une quelconque des revendications 1 à 4 ou une composition selon l'une quelconque des revendications 5 à 7, pour utilisation dans une méthode d'imagerie comprenant :
a) l'administration d'au moins ledit produit de contraste au Ga68, et
b) l'administration d'au moins un produit de contraste pour IRM ou scanner RX, destiné à analyser spécifiquement la ou les zones d'intérêt diagnostique détectées en a).

11. Produit de contraste au Ga68 radiomarqué comprenant un complexe selon l'une quelconque des revendications 1 à 4 ou une composition selon l'une quelconque des revendications 5 à 7, pour utilisation dans une méthode d'imagerie comprenant :
a) l'administration d'au moins un produit de contraste pour IRM ou scanner RX capable d'effectuer une première analyse d'une zone d'intérêt diagnostique, et
b) l'administration d'au moins ledit produit de contraste au Ga68 radiomarqué pour effectuer une seconde analyse de la zone d'intérêt diagnostique ou d'une zone plus large que la zone d'intérêt.

12. Installation d'imagerie médicale de type PET/CT ou PET/IRM ou PET/CT/IRM mettant en oeuvre une méthode selon la revendication 10 ou 11 comprenant :
- un dispositif d'imagerie capable d'effectuer des mesures de paramètres d'imagerie PET/IRM ou PET/CT ou PET/CT/IRM avec ou sans injection de produit de contraste
- un dispositif d'injection de produit de contraste marqué au Gallium 68 comprenant un complexe tel que défini dans l'une des revendications 1 à 4 ou une composition selon l'une quelconque des revendications 5 à 7, relié au patient
- un dispositif d'injection d'un produit de contraste pour imagerie RX ou IRM
- le cas échéant un dispositif d'analyse et de commande du dispositif d'injection du produit pour imagerie RX ou IRM en fonction d'une première mesure effectuée suite à l'administration du produit de contraste radiomarqué au gallium 68.

13. Procédé de préparation de complexes selon l'une quelconque des revendications 1 à 4 sans micro-ondes comprenant :
1) la synthèse d'un composé (B-L-Ch) par couplage d'un biovecteur à un chélate linéaire ou macrocyclique
2) la complexation du composé (B-L-Ch) avec le gallium Ga68 par chauffage en moins de 10 minutes.

## Patentansprüche

1. Gallium-Ga-68-Metallkomplex der Formel Ch-L-B (Chelat-Linker-Biovektor) oder Ch-Lp-Bq, wobei p=q=2 bis 5, wobei Ch ein Chelat der folgenden allgemeinen Formel ist: 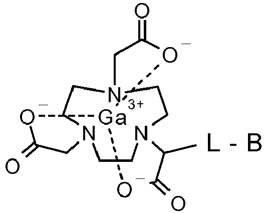 in der:
1) B ein Biovektor zum Ansteuern eines mit einer Pathologie assoziierten biologischen Angriffspunkts ist, wobei der Biovektor aus Folat, Metalloproteasen, COX, Tyrosinkinase und Zellrezeptoren, ausgewählt aus VEGF, KDR, CXC, LHRH, GPIIb/IIIa, Bombesin/GRP, Gastrin, VIP, CCK und GLUT, ausgewählt ist,
2) L ein Linker, ausgewählt aus den Folgenden, ist:
a) P1-1-P2, wobei P1 und P2, die gleich oder verschieden sind, aus O, S, NH, CO₂, -NHCO, CONH, NHCONH, NHCSNH, SO₂NH- und NHSO₂-ausgewählt sind,
und 1 Alkyl (vorteilhaft C₁-C₁₀), Alkoxyalkyl (vorteilhaft C₁-C₁₀), Polyalkoxyalkylen, Alkyl, unterbrochen durch ein oder mehrere Squaraten oder durch ein oder mehrere Aryle, vorteilhaft Phenyl, Alkenyl (vorteilhaft C₂-C₆, Alkinyl (vorteilhaft C₂-C₆), Alkyl, unterbrochen durch eine oder mehrere Gruppen ausgewählt aus -NH-, -O-, -CO-, -NH(CO)-, -(CO)NH-, -O(CO)- oder -(OC)O-, bedeutet;
b) (CH₂)ₙ, (CH₂)ₙ-CO-, -(CH₂)ₙNH-CO-, wobei n = 2 bis 10, (CH₂CH₂O)_{q}(CH₂)ᵣ-CO-, (CH₂CH₂O)_{q}(CH₂)ᵣ-NH-CO-, wobei q = 1-10 und r=2-10, (CH₂)ₙ-CONH -, (CH₂)ₙ-CONH -PEG, (CH₂)ₙ-NH-, 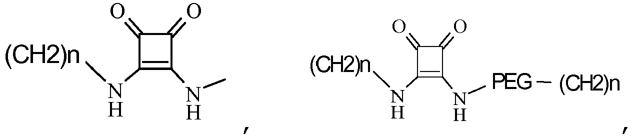 wobei n=1 bis 5 und vorteilhaft n=4 oder 5, HOOC-CH₂-O-(CH₂)₂-O-(CH₂)₂-O-CH₂-COOH; HOOC-(CH₂)₂-CO₂-(CH₂)₂-OCO-(CH₂)₂-COOH; HOOC-CH(OH)-CH(OH)-COOH; HOOC-(CH₂)ₙ-COOH; NH₂-(CH₂)ₙ-NH₂, wobei n=0-20; NH₂-(CH₂)ₙ-CO₂H; NH₂-CH₂-(CH₂-O-CH₂)ₙ-CO₂H, wobei n=1 bis 10.

2. Komplex nach Anspruch 1, ausgewählt aus: 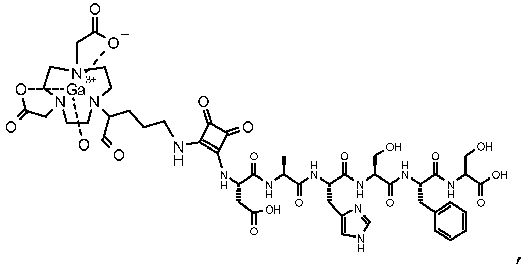 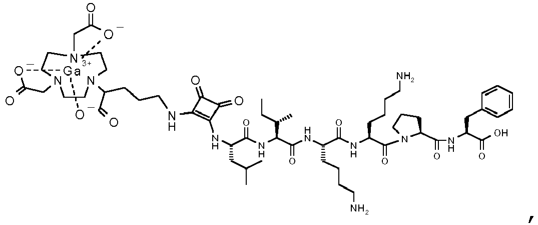 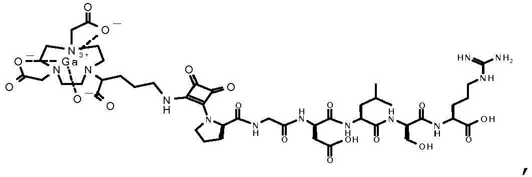 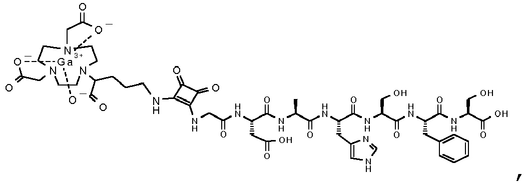 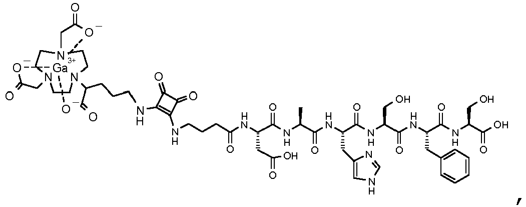 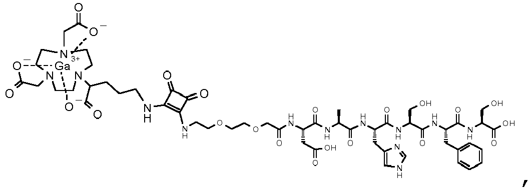 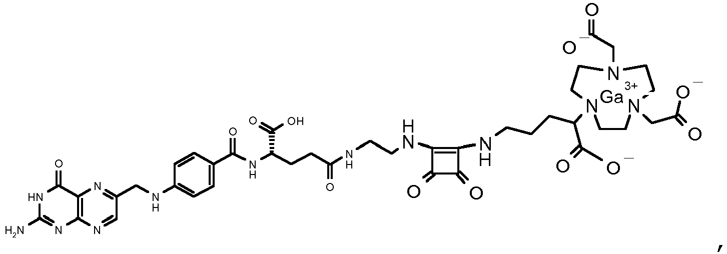 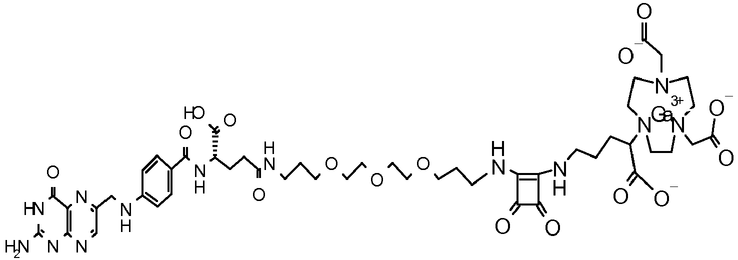 und

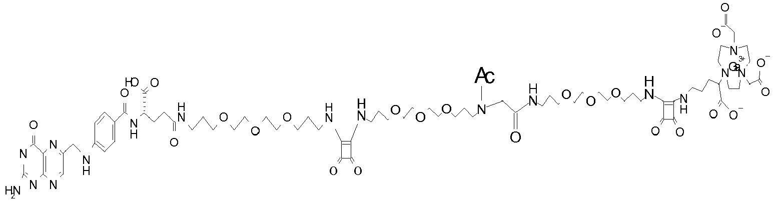

3. Komplex nach Anspruch 1 oder 2, **dadurch gekennzeichnet, dass** das Chelat mindestens eine Gruppierung zur in-vivo-Maskierung des Chelats, insbesondere eine hydrophile Gruppe, umfasst.

4. Komplex nach einem der Ansprüche 1 bis 3, **dadurch gekennzeichnet, dass** das Chelat weiterhin eine Erkennungsgruppierung eines biologischen Angriffspunkts umfasst, der die biologische Verteilung der Verbindung und/oder den Transport der Verbindung verbessert, wobei diese Erkennungsgruppierung vom Biovektor verschieden ist.

5. Zusammensetzung, umfassend einen Komplex nach einem der Ansprüche 1 bis 4 und ein nichtkomplexiertes geradkettiges oder makrocyclisches Chelat Ch wie in Anspruch 1 definiert, das in einer Konzentration von 0,01 bis 100 mM, vorteilhaft von 10 bis 100 mM vorliegt.

6. Zusammensetzung, umfassend einen Komplex nach einem der Ansprüche 1 bis 4 sowie Calcium.

7. Zusammensetzung, umfassend einen Komplex nach einem der Ansprüche 1 bis 4 sowie mindestens einen Radiolysestabilisator.

8. Kit zum Verabreichen eines mit Gallium Ga68 markierten Produkts, das ein Komplex nach einem der Ansprüche 1 bis 4 oder eine Zusammensetzung nach einem der Ansprüche 5 bis 7 umfasst.

9. Diagnostische Zusammensetzung, die erstens ein mittels PET nachweisbares Gallium-Ga68-Kontrastmittel, das einen Komplex nach einem der Ansprüche 1 bis 4 oder eine Zusammensetzung nach einem der Ansprüche 5 bis 7 umfasst, und zweitens ein mittels IRM nachweisbares Kontrastmittel und/oder ein mittels CT-Scanner nachweisbares Kontrastmittel umfasst.

10. Ga68-Kontrastprodukt, das die Entdeckung mittels PET-Imaging von mindestens einer diagnostischen Zone von Interesse gestattet und das einen Komplex nach einem der Ansprüche 1 bis 4 oder eine Zusammensetzung nach einem der Ansprüche 5 bis 7 umfasst, zur Verwendung in einem Imaging-Verfahren, umfassend:
a) die Verabreichung von mindestens dem Ga68-Kontrastprodukt, und
b) die Verabreichung von mindestens einem IRM- oder Röntgen-Scan-Kontrastprodukt, das der spezifischen Analyse der in a) nachgewiesenen diagnostischen Zone(n) von Interesse dient.

11. Radioaktiv markiertes Ga68-Kontrastprodukt, das einen Komplex nach einem der Ansprüche 1 bis 4 oder eine Zusammensetzung nach einem der Ansprüche 5 bis 7 umfasst, zur Verwendung in einem Imaging-Verfahren, das Folgendes umfasst:
a) die Verabreichung von mindestens einem IRM- oder Röntgen-Scan-Kontrastprodukt, das fähig ist, eine erste Analyse einer diagnostischen Zone von Interesse durchzuführen, und
b) die Verabreichung von mindestens dem radioaktiv markierten Ga68-Kontrastprodukt zum Durchführen einer zweiten Analyse der diagnostischen Zone von Interesse oder einer Zone, die größer als die Zone von Interesse ist.

12. Medizinische Bildgebungsvorrichtung des PET/CT-oder PET/IRM- oder PET/CT/IRM-Typs, mit der ein Verfahren nach Anspruch 10 oder 11 durchgeführt wird und die Folgendes umfasst:
- Imaging-Einheit, die fähig ist, Messungen von PET/IRM- oder PET/CT- oder PET/CT/IRM-Imaging-Parametern mit oder ohne Injektion von Kontrastprodukt durchzuführen
- Einheit zum Injizieren des mit Gallium 68 markierten Kontrastprodukts, das einen Komplex wie in einem der Ansprüche 1 bis 4 definiert oder eine Zusammensetzung nach einem der Ansprüche 5 bis 7 umfasst, die mit dem Patienten verbunden ist
- Einheit zum Injizieren eines Kontrastprodukts für das Röntgen- oder IRM-Imaging
- gegebenenfalls eine Einrichtung zum Analysieren und Steuern der Einrichtung zum Injizieren des Produkts für das Röntgen- oder IRM-Imaging in Abhängigkeit einer ersten Messung, die nach der Verabreichung des mit Gallium 68 radioaktiv markierten Kontrastprodukts erfolgt.

13. Verfahren zur Herstellung von Komplexen nach einem der Ansprüche 1 bis 4 ohne Mikrowellen, das Folgendes umfasst:
1) Synthese einer Verbindung (B-L-Ch) durch Koppeln eines Biovektors mit einem geradkettigen oder makrocyclischen Chelat
2) Komplexieren der Verbindung (B-L-Ch) mit Gallium Ga68 durch mindestens zehnminütiges Erhitzen.

## Claims

1. Metal complex of gallium Ga68 of formula Ch-L-B (chelate - linker - biovector), or Ch-Lp-Bq with p = q = 2 to 5, Ch being a chelate having the following general formula: 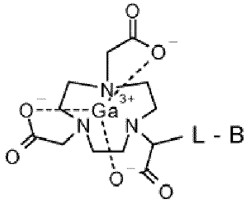 in which:
1) B is a biovector for targeting a biological target associated with a pathological condition, said biovector being chosen from folate, metalloproteases, COX, tyrosine kinase, and cell receptors chosen from VEGF, KDR, CXC, LHRH, GPIIb/IIIa, bombesin/GRP, gastrin, VIP, CCK and GLUT,
2) L is a linker chosen from:
a) P1-1-P2, with P1 and P2, which may be identical or different, chosen from O, S, NH, CO₂, -NHCO, CONH, NHCONH, NHCSNH, SO₂NH-, and NHSO₂-,
and 1 represents an alkyl (advantageously C₁-C₁₀), alkoxyalkyl (advantageously C₁-C₁₀), polyalkoxyalkylene, alkyl interrupted with one or more squarates or with one more aryls, advantageously phenyl, alkenyl (advantageously C₂-C₆), alkynyl (advantageously C₂-C₆), alkyl interrupted with one or more groups chosen from -NH-, -O-, -CO-, -NH (CO) -, - (CO) NH-, -O(CO)-, or -(OC)O-;
b) (CH₂)ₙ, (CH₂)ₙ-CO-, -(CH₂)ₙNH-CO- with n = 2 to 10, (CH₂CH₂O)_{q}(CH₂)ᵣ-CO-, (CH₂CH₂O)_{q}(CH₂)ᵣ-NH-CO- with q = 1-10 and r = 2-10, (CH₂)ₙ-CONH-, (CH₂)ₙ-CONH-PEG, (CH₂)ₙ-NH-, 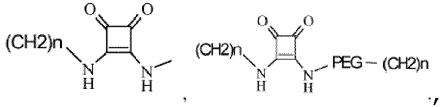 with n = 1 to 5 and advantageously n = 4 or 5, HOOC-CH₂-O(CH₂)₂-O-(CH₂)₂-O-CH₂-COOH; HOOC-(CH₂)₂-CO₂-(CH₂)₂-OCO-(CH₂)₂-COOH; HOOC-CH(OH)-CH(OH)-COOH; HOOC-(CH₂)ₙ-COOH; NH₂-(CH₂)ₙ-NH₂, with n = 0-20; NH₂-(CH₂)ₙ-CO₂H; NH₂-CH₂-(CH₂-O-CH₂)ₙ-CO₂H with n = 1 to 10.

2. Complex according to Claim 1, chosen from: 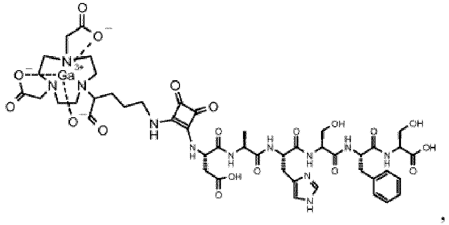 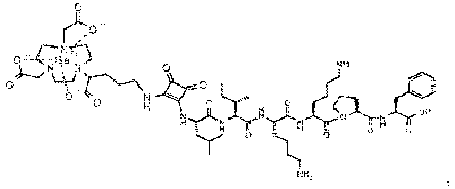 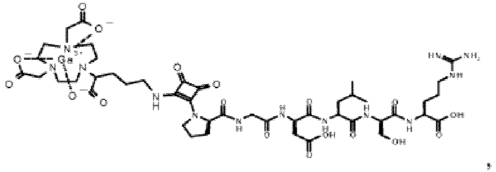 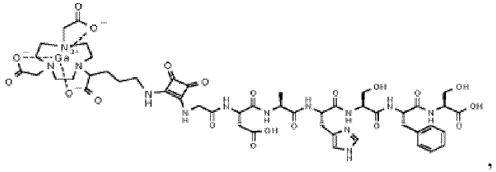 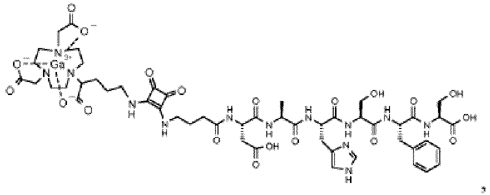 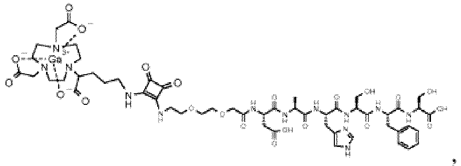 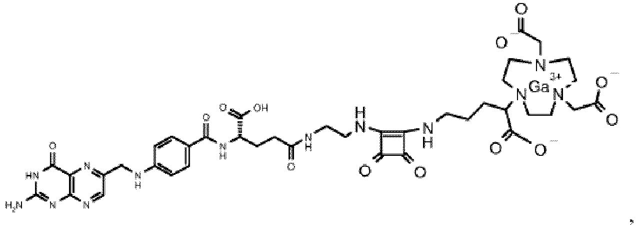 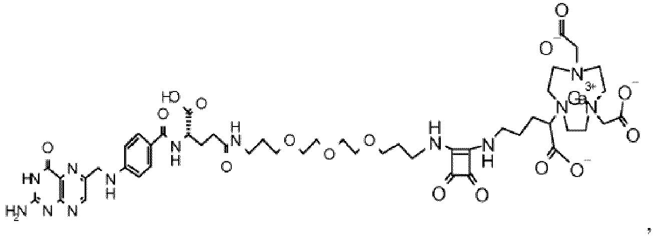 and

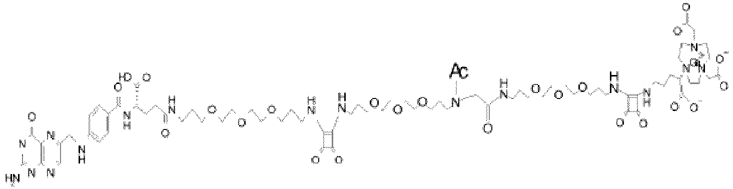

3. Complex according to Claim 1 or 2, **characterized in that** the chelate comprises at least one part for *in vivo* masking of the chelate, in particular a hydrophilic group.

4. Complex according to any one of Claims 1 to 3, **characterized in that** the chelate also comprises a part for recognition of a biological target which improves the biodistribution of the compound and/or the transport of the compound, said recognition part being different from the biovector.

5. Composition comprising a complex according to any one of Claims 1 to 4 and a linear or macrocyclic chelate Ch as defined in Claim 1 which is not complexed, present at a concentration of from 0.01 to 100 mM, advantageously from 10 to 100 mM.

6. Composition comprising a complex according to any one of Claims 1 to 4 and calcium.

7. Composition comprising a complex according to any one of Claims 1 to 4 and at least one stabilizing agent with respect to radiolysis.

8. Kit for administering a gallium Ga68-labelled product comprising a complex according to any one of Claims 1 to 4 or a composition according to one of Claims 5 to 7.

9. Diagnostic kit comprising, firstly, a PET-detectable gallium Ga68 contrast agent comprising a complex according to any one of Claims 1 to 4 or a composition according to any one of Claims 5 to 7 and, secondly, an MRI-detectable contrast agent and/or a CT scan-detectable contrast agent.

10. Ga68 contrast product which makes it possible to detect, by PET imaging, at least one zone of diagnostic interest, comprising a complex according to any one of Claims 1 to 4 or a composition according to any one of Claims 5 to 7, for use in an imaging method comprising:
a) the administration of at least said Ga68 contrast product, and
b) the administration of at least one contrast product for MRI or for X-ray scan, intended for specifically analysing the zone(s) of diagnostic interest detected in a).

11. Radio labelled Ga68 contrast product comprising a complex according to any one of Claims 1 to 4 or a composition according to any one of Claims 5 to 7, for use in an imaging method comprising:
a) administering at least one contrast product for MRI or X-ray scan, capable of carrying out a first analysis of a zone of diagnostic interest, and
b) administering at least said radio labelled Ga68 contrast product for carrying out a second analysis of the zone of diagnostic interest or of a zone that is broader than the zone of interest.

12. Apparatus for medical imaging of PET/CT or PET/MRI or PET/CT/MRI type, implementing a method according to Claim 10 or 11, comprising:
- an imaging device capable of carrying out PET/MRI or PET/CT or PET/CT/MRI imaging parameter measurements with or without injection of contrast product,
- a device for injecting a gallium 68-labelled contrast product comprising a complex as defined in one of Claims 1 to 4 or a composition according to any one of Claims 5 to 7, connected to the patient,
- a device for injecting a contrast product for X-ray or MRI imaging,
- where appropriate, a device for analysing and controlling the device for injecting the product for X-ray or MRI imaging as a function of a first measurement carried out following the administration of the gallium 68-radio labelled contrast product.

13. A process for preparing complexes according to any one of Claims 1 to 4 without microwaves, comprising:
1) the synthesis of a compound (B-L-Ch) by coupling a biovector to a linear or macrocyclic chelate,
2) the complexation of the compound (B-L-Ch) with gallium Ga68 by heating in less than 10 minutes.
